(19) Europäisches Patentamt
European Patent Office
Office européen des brevets

(11) EP 4 053 135 A1

(12) EUROPEAN PATENT APPLICATION
published in accordance with Art. 153(4) EPC

(43) Date of publication:
**07.09.2022 Bulletin 2022/36**

(21) Application number: **20883322.8**

(22) Date of filing: **30.10.2020**

(51) International Patent Classification (IPC):
***C07F 9/564*** *(2006.01)* ***A61K 31/04*** *(2006.01)*
***A61K 31/06*** *(2006.01)* ***A61K 31/664*** *(2006.01)*
***A61P 35/00*** *(2006.01)*

(52) Cooperative Patent Classification (CPC):
**A61K 31/04; A61K 31/06; A61K 31/664; A61P 35/00; C07F 9/564**

(86) International application number:
**PCT/CN2020/125123**

(87) International publication number:
**WO 2021/083310 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2019 CN 201911060133**
**20.12.2019 CN 201911323908**

(71) Applicant: **Ascentawits Pharmaceuticals, Ltd.**
**Shenzhen, Guangdong 518118 (CN)**

(72) Inventors:
- **DUAN, Jianxin**
  **Shenzhen, Guangdong 518118 (CN)**
- **LI, Anrong**
  **Shenzhen, Guangdong 518118 (CN)**
- **CAI, Xiaohong**
  **Shenzhen, Guangdong 518118 (CN)**

(74) Representative: **Michalski Hüttermann & Partner Patentanwälte mbB**
**Kaistraße 16A**
**40221 Düsseldorf (DE)**

# (54) ANTI-CANCER COMPOUNDS ACTING AS NON-PGP SUBSTRATE

(57) Provided are anti-cancer compounds which act as a non-PGP substrate, same being compounds of formula 1-1, or a pharmaceutically acceptable salt, a prodrug or a solvate thereof, and the medical use of these compounds in the treatment of cancers, tumors, conditions caused by cancers or tumors, or cell proliferative diseases. Also provided is a method for treating cancers, tumors, conditions caused by cancers or tumors, or cell proliferative diseases using the anti-cancer compounds which act as non-PGP substrates as described above.

EP 4 053 135 A1

## Description

## BACKGROUND

### Technical Field

The disclosure relates to the further research of the compounds disclosed in the patent application number PCT/US2016/021581 (publication number WO2016145092A1), corresponding to China application number 2016800150788 (publication number CN107530556A). More particularly, the disclosure relates to the technical field of the research of the cancer therapeutic compound.

### Description of Related Art

The prodrug of DNA alkylating agents, as a cancer therapeutic compound, developed by our company (application number PCT/US2016/021581, publication number WO2016/145092; application number PCT/US/2016/062114, publication number WO2017/087428) that targets highly expressed aldo-keto reductase 1C3 (AKR1C3) are specifically metabolically activated under the action of AKR1C3 in vivo. The S-form AST-3424 of TH2870 is taken as an example:

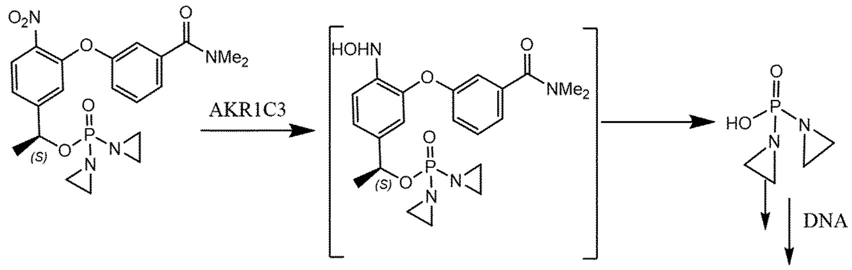


AST-3424 Unstable intermediate Cell death

Stable Compound

DNA alkylating agents in the form of a prodrug targeting the highly expressed aldo-keto reductase AKR1C3 will bind to AKR1C3 in vivo, and then undergo a metabolic reaction to finally produce cytotoxic DNA alkylating agents.

However, these compounds are not solids, but oily substances, causing the following difficulties in developing of the preparations:

The separation and the purification are complicated and costly. Since it is an oily substance, it cannot be purified by high-efficiency and low-cost recrystallization or slurry, so it can only be purified by column chromatography, which is complicated and thus results in high cost for the preparation of active pharmaceutical ingredients.

The preparation is inconvenient and with poor stability. Since it is an oily substance, it is inconvenient to handle during the transferring/measuring process. Importantly, the preparation is inconvenient to develop and diversify the formulation. Generally, only a freeze-dry powder injector can be developed for drug administration, so the drug administration is limited and with higher costs.

Therefore, we are dedicated to improve the anti-cancer compound with this kind of mechanism.

## SUMMARY

During the research process, the compounds above were substituted by various groups. When the O, S atoms on the ortho-position to the nitro group on the benzene ring were not connected to a group similar to the groups in application number PCT/US2016/021581, publication number WO2016/145092; application number PCT/US2016/062114, publication number WO2017/087428; application number PCT/US2016/025665, publication number WO2016/161342 (a C6-C10 aryl group, a 5-15 membered heteroaryl group, or $-N=CR^1R^2$) but a biphenyl group or the groups similar to the biphenyl (pyridylbenzene), except that the corresponding compounds have the activity of killing cancer cells activated by AKR1C3, the compounds were proved by experiments that they are not the substrates bonded to P-gp in cells. That is, the compounds accidentally had a better ability to pass through the blood-brain barrier (not related to the substrates bonded P-GP). It means that the compounds may have the potential to develop as a medicine for the tumors or cancers of the central nervous system (mainly the brain).

The following technical solutions are provided in this disclosure.

An anti-cancer compound for acting as a non-PGP substrate, wherein the compound is a compound having a

formula I-1, or a pharmaceutically acceptable salt or a solvate thereof:

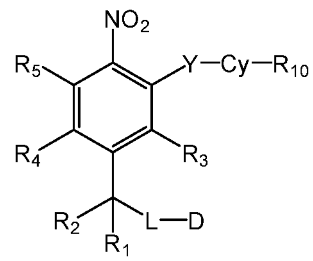

I-1

wherein,

$R_1$ and $R_2$ are respectively independently hydrogen, deuterium, an aryl group or a Z-substituted aryl group, a heteroaryl group, a heterocycle or a Z-substituted heteroaryl group, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group;

$R_3$ is hydrogen, halogen, a cyano group or an isocyano group, a thiocyanato group or an isothiocyanato group, hydroxyl group, a thiol group, an amino group, oxime, hydrazone, OTs, OMs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, or

$R_3$ is $-CONR^6R^7$, $-SO_2NR^6R^7$, $-SO_2R^6$, $-OCO-R^6$,$-OCOO-R^6$, $-COOR^6$, $-NR^6COR^7$, $-OCOR^6$, $-NR^6SO_2R^7$, and $-NR^6CONR^7$;

$R_4$ and $R_5$ are respectively independently hydrogen, halogen, a cyano group or an isocyano group, a thiocyanato group or an isothiocyanato group, a hydroxyl group, a thiol group, an amino group, oxime, hydrazone, OTs, OMs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$, $-NR^6CONR^7$, or $R_4$ and $R_5$ and the atom of the bonding benzene ring bonded thereto form a 7-15 membered fused ring or a Z-substituted fused ring;

$R^6$ and $R^7$ are each independently hydrogen, a cyano group or an isocyano group, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, or

$R^6$ and $R^7$ and atoms bonded thereto form a 3-7 membered heterocyclyl group or a Z-substituted 3-7 membered heterocyclyl group;

Cy is a 5-10 membered aromatic ring, heterocyclic ring or heteroaromatic ring, and hydrogens of the ring are each independently substituted by deuterium, halogen, a cyano group or an isocyano group, a thiocyanato group or an isothiocyanato group, a hydroxyl group, a thiol group, an amino group, oxime, hydrazone, OTs, OMs, a $C_1$-$C_6$ aliphatic group, or the

hydrogens of the ring are each independently substituted by $C_1$-$C_6$ aliphatic group substituted by halogen, a cyano group, an isocyano group, a hydroxyl group, a thiol group, an amino group, oxime, hydrazone;

$R_{10}$ is a 3-18 membered cyclohydrocarbyl group, an aryl group or a fused ring, a heterocycle, a fused heterocycle, a heteroaryl group or a Z-substituted $C_3$-$C_{18}$ cycloalkyl group, an aryl or a fused ring, heterocycle, a fused heterocyclyl group, a heteroaryl group or a $C_1$-$C_{18}$ hydrocarbyl group or a Z-substituted hydrocarbyl group; or

$R_{10}$ is -Q-Cz,

Q is -O-, -S-, -CO-, $-SO_2-$, -SO-, -OCO-, -OCOO-, $-NR^6CO-$, $-NR^6SO_2-$, $-OCONR^6$; and

Cz is a 3-18 membered cyclohydrocarbyl group, an aryl group or a fused ring, a heterocycle, a fused heterocycle, a heteroaryl group or a Z-substituted 3-18 membered cycloalkyl group, an aryl group or a fused ring, a heterocycle,

a fused heterocyclyl group, a heteroaryl group or a $C_1$-$C_{18}$ hydrocarbyl group or a Z-substituted hydrocarbyl group;
Y is -O- or -S- or -$SO_2$-, -SO-;
L and D are selected from the following three situations:

(1) L is selected from -O-, -S-, -OCOO-, -$NR^6$CO-, -OCO-, -$NR^6SO_2$-, -OCON$R^6$-, quaternary ammonium, -$OSO_2$,

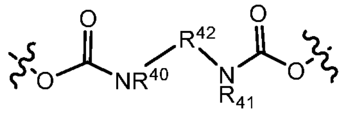 ,

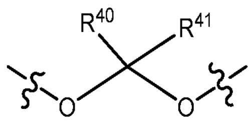 , 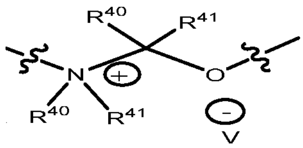 , 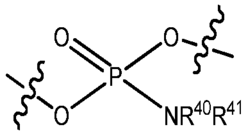 ,

$R^{40}$ and $R^{41}$ are independently hydrogen, a $C_1$-$C_6$ alkyl group, a $C_2$-$C_6$ alkenyl group, a $C_2$-$C_6$ alkynyl group, a $C_3$-$C_8$ cycloalkyl group, a $C_6$-$C_{10}$ aryl group, a 4-15 membered heterocycle or a 5-15 membered heteroaryl group;
$R^{42}$ is $C_2$-$C_3$ alkylenyl, heterylene or one to three $C_1$-$C_6$ alkyl-substituted $C_2$-$C_3$ alkylenyl, $C_1$-$C_6$ alkyl-substituted heterocycloalkylene;
V(-) is any anion;
D is a moiety for making D-OH an anti-cancer drug, wherein -OH is an aliphatic group, a phenolic hydroxyl group, or a -OH moiety attached to the phosphate ester;

(2) L is selected from

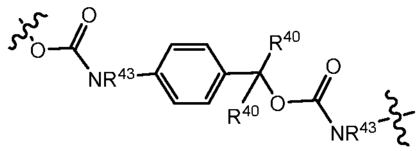 ,

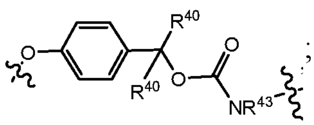 ;

$R^{40}$ is defined as above;
$R^{43}$ is hydrogen or form to heterocycle with D,
phenylene is Z-substituted or unsubstituted; and
D is a moiety for making D-$NR^{43}$H an anti-cancer drug; or

(3) L is selected from a bond, -O-C($R^{40}R^{41}$)$_2$, -O-C($R^{40}R^{41}$)-$NR^{40}R^{41}$(+)-C($R^{40}R^{41}$)-, or , wherein

$R^{40}$, $R^{41}$ and V(-) are defined as above; and
D is an anti-cancer drug with a tertiary or secondary nitrogen atom, wherein the tertiary or secondary nitrogen atom is bonded to L; and
an alkyl group, an alkenyl group, an alkynyl group, a cycloalkyl group, an aryl group, a heterocycle, a heteroaryl group, and ether in the definitions of the L and D are Z-substituted or unsubstituted;
the Z-substituted group is halogen, a cyano group or an isocyano group, a thiocyanato group or an isothiocyanato group, a hydroxyl group, a thiol group, an amino group, oxime, hydrazone, OTs, OMs, a $C_1$-$C_3$ alkyl group or a substituted alkyl group, a $C_2$-$C_3$ alkenyl group or a substituted alkenyl group, a $C_2$-$C_3$ alkynyl group or a substituted alkynyl group, a $C_3$-$C_8$ cycloalkyl group or a substituted cycloalkyl group, an aromatic ring, a heterocycle, a heteroaryl group and a fused ring or a substituted aryl group, heterocycle, heteroaryl group and a fused ring, wherein
the substitution manners are mono-substitutied or double-substitutied; and

the substituent is halogen, a cyano group or an isocyano group, a thiocyanato group or an isothiocyanato group, a hydroxyl group, a thiol group, an amino group, oxime, hydrazone, OTs, OMs; and the compound does not comprises

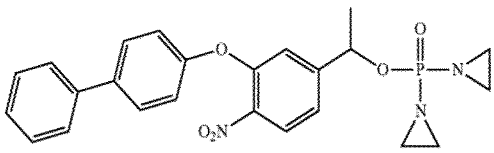
,

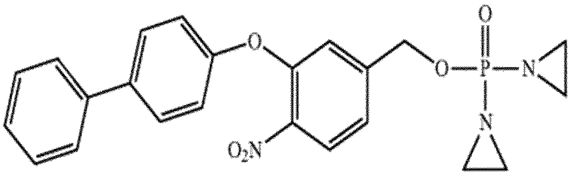
,

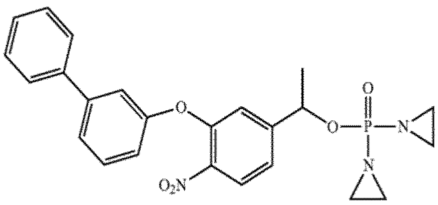
,
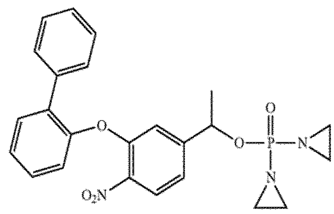
,

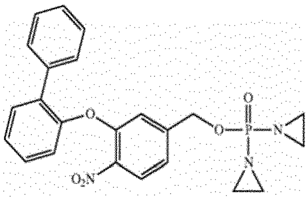
,
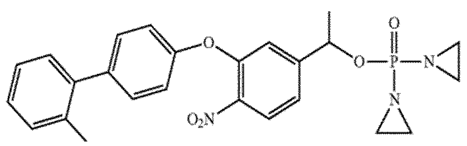
,

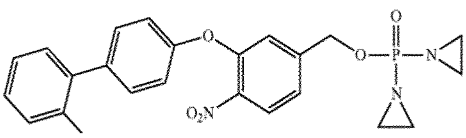
,
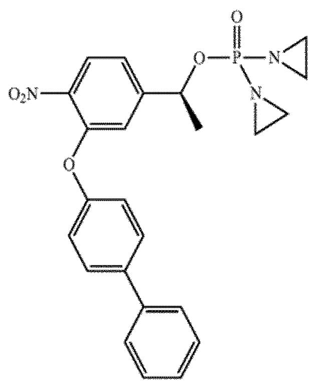
,

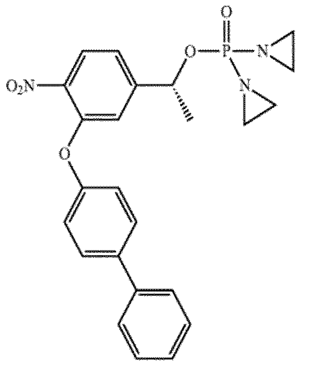

,

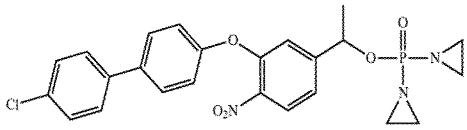

,

or

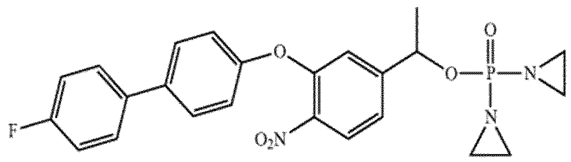

.

The terms "heterocycle" and "heteroaryl group" include a 3-membered ring, a 4-membered ring, a 5-membered ring, a 6-membered ring, and a 7-membered ring.

For example:

3-membered ring: oxirane, aziridine, and thiirane;
4-membered ring: azetidine, oxetane, thietane, and azete;
5-membered ring: pyrrolidine, pyrroline, 1-pyrroline, 2-pyrroline, 3-pyrroline, pyrrole, pyrazolidine, 2-pyrazoline, imidazole, pyrazole, furan, oxolane (THF), dihydrofuran, tetrahydrothiophene (THT), thiophene, sulfolane, phosphole, oxazole, 1,2,3-trizole, 1,2,4-trizole, and 1,3,4-thiadiazole;
6-membered ring: piperidine, tetrahydropyran (THP), tetrahydrothiopyran, pyridine, pyran, thiopyran, dihydropyridine, morpholine, piperazidine, pyridazine, pyrazine, 1,3,5-triazine, and 1,3,5-trithiane;
7-membered ring: azepane, oxepane, thiepane, azepine, oxepine, and thiepine.

The term "fused ring" is defined as the combination of a heterocycle and a heteroaryl group above or the combination with a cycloalkane. The combination may be a single bond or may be in a form of sharing one, two, or even three atoms. Some common fused ring structures are given as following: naphthalene, quinolone, indole, isoindole, isoquinoline, cinnoline, quinoxaline, biphenyl, coumarin, fluorene, dibenzopyran, carbazole, anthracene, azaanthracene, phenothiazine, adamantine, azulene, phenanthrene, anthraquinone, flavonoid, isoflavonoid, for examples.

Obviously, the compounds described above also include an isotope substituted compounds. Typically, the substitution way is that the hydrogen atom (H) is substituted by deuterium (D), or $-CH_3$ is substituted by $-CD_3$.

Particularly, the moiety substituted by deuterium is located on the Ph-$C^x$ of the formula I, i.e., $R_1$ and/or $R_2$ is deuterium.

Furthermore, the compound described above, wherein

-D is $-P(Z^1)(Z^5-X^5-Y^5)_n$, wherein
$Z^5$ is N, S, or O,
$X^5$ is any substituted ethylidene,
$Y^5$ is halogen or $-OSO_2-R^{20}$, wherein $R^{20}$ is any substituted hydrocarbyl group, aryl group, cyclic heteroaryl group, heterocycle and heteroaryl group, and
n is 1 or 2; or
$Z^5-X^5-Y^5$ is $-NCH_2CH_2$;
$Z^1$ is O or S.

The structure above is an amino phosphate ester alkylating agent.

Moreover, the compound:

-L-D is $-O-P(Z^1)(Z^5-X^5-Y^5)_n$,
$Z^5$ is N, S or O,
$X^5$ is any substituted ethylidene,
$Y^5$ is halogen or $-OSO_2-R^{20}$, wherein $R^{20}$ is any substituted hydrocarbyl group, aryl group, cycloalkyl group, heterocycle; and heteroaryl group, and
n is 1 or 2; or
$Z^5-X^5-Y^5$ is $-NCH_2CH_2$;
$Z^1$ is O or S.

Further, -L-D is $OP(Z^1)(NR^{30}CH_2CH_2Cl)_2$, $-OP(Z^1)(NR^{30}CH_2CH_2Br)_2$, $-OP(Z^1)(NR^{30}{}_2)(N(CH_2CH_2X^1)_2)$, $-OP(Z^1)(N(CH_2)_2)_2$, or $-OP(Z^1)(N(CH_2CH_2Cl)_2)_2$, wherein every $R^{30}$ is each independent hydrogen, a $C_1$-$C_6$ hydrocarbyl group, or two $R^{30}$ group and the nitrogen atom bonded thereto form 5-7 membered heterocycle; $Z^1$ is O or S; $X^1$ is Cl, Br or $-SOS_2Me$.

Further, -L-D is $-OP(Z^1)(NHCH_2CH_2Cl)_2$, $-OP(Z^1)(NHCH_2CH_2Br)_2$, $-OP(Z^1)(NH_2)(N(CH_2CH_2X^1)_2)$, $-OP(Z^1)(N(CH_2)_2)_2$, or $-OP(Z^1)(N(CH_2CH_2Cl)_2)_2$; and $X^1$ is Cl, Br or $-SOS_2Me$.

Furthermore, the anti-cancer compound described above, wherein

$R_{10}$ is a 5-18 membered cycloalkyl group, an aryl group or a fused ring, a heterocycle, a fused heterocycle, a heteroaryl group, or a Z-substituted 5-18 membered cycloalkyl group, an aryl group or a fused ring, a heterocycle, a fused heterocycle, a heteroaryl group, or a $-CF_3$ or C1 substituted hydrocarbyl group; or
$R_{10}$ is -O-Cz, Cz is a 5-18 membered cycloalkyl group, an aryl group, a fused ring, a heterocycle, a fused heterocycle, a heteroaryl group, or a Z-substituted 5-18 membered cycloalkyl, an aryl group, a fused ring, a heterocycle, a fused heterocycle, a heteroaryl group, or a $-CF_3$ or C1 substituted hydrocarbyl group.

Furthermore, the anti-cancer compound, wherein

$R_{10}$ is 7-18 membered a cycloalkyl group, an aryl group, a fused ring, a heterocycle, a fused heterocycle, a heteroaryl group, or a Z-substituted 7-18 membered cycloalkyl group, an aryl group, a fused ring, a heterocycle, a fused heterocycle, a heteroaryl group, or a $-CF_3$ or C1 substituted hydrocarbyl group; or
$R_{10}$ is -O-Cz, Cz is a 7-18 membered cycloalkyl group, an aryl group, a fused ring, a heterocycle, a fused heterocycle, a heteroaryl group, or a Z-substituted 7-18 membered cycloalkyl group, an aryl group, a fused ring, a heterocycle, a fused heterocycle, a heteroaryl group, or a $-CF_3$ or C1 substituted hydrocarbyl group.

In some embodiments of the present disclosure, the compound includes the structure of formula 1-2:

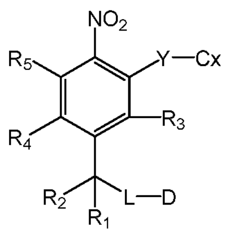

I-2

wherein,

Cx is selected from biphenyl, Z-substituted biphenyl, phenylpyridyl, Z-substituted phenylpyridyl, and biphenyl and phenylpyridyl that with a substituent of $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$, $-NR^6SO_2NR^6R^7$, $-COR^6$, and $-NR^6CONR^7$.

The compound with the structure of formula 1-2, wherein -Y- is connected to a para-position of a carbon atom connecting two benzenes of -Cx, and a substituent on biphenyl is F or methyl.

The compound of the present disclosure, preferably, $R_3$, $R_4$, and $R_5$ are each independent hydrogen.

The compound of the present disclosure, preferably, $R_1$, $R_2$ are each independent hydrogen, deuterium, $-CH_3$, $-CD_3$, $-CF_3$.

The compound of the present disclosure, preferably, Y is -O-.

The compound with the structure of formula I-1 or 1-2, wherein

Cy is a 5-10 membered heteroaryl group or a benzene ring, or
hydrogens thereon are each independent substituted by deuterium, halogen, a cyano group or an isocyano group, a thiocyanato group or an isothiocyanato group, hydroxyl, a thiol group, an amino group, oxime, hydrazone, OTs, OMs, a $C_1$-$C_6$ aliphatic group; or substituted by a $C_1$-$C_6$ aliphatic group substituted by halogen, a cyano group or an isocyano group, a hydroxyl group, a thiol group, an amino group, oxime, and hydrazone.

Moreover, a substituent on Cy is hydrogen, deuterium, halogen, $-CH_3$, or $-CF_3$.
Cy is selected from a 5-8 membered aromatic heterocycle, a heteroatom is N, S or O, and the number of the heteroatom is 1, 2, or 3.
The compound in the present disclosure is preferably selected from the structures below:

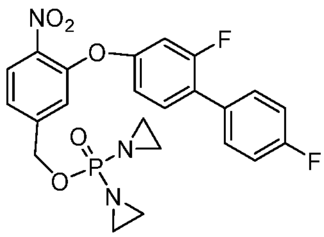
,
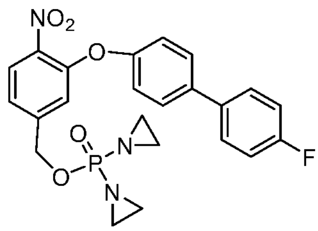
,

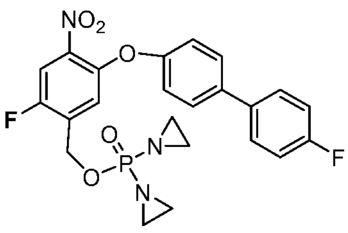
,
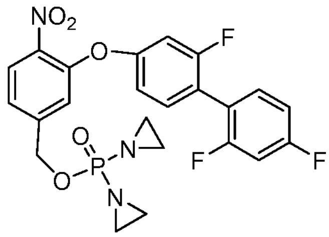
,
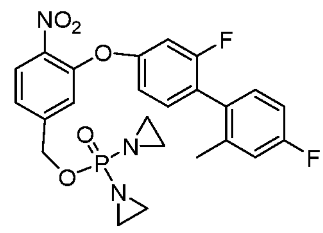
,

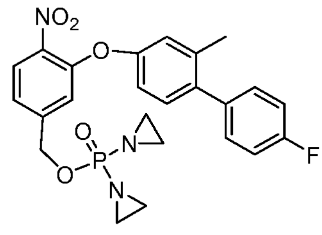
,
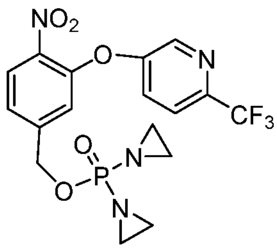
,
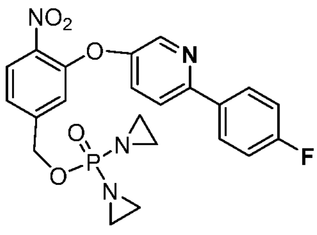
,

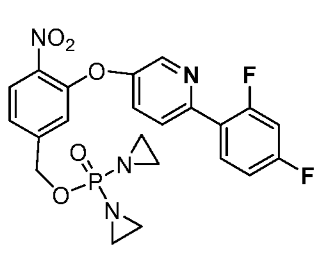
,
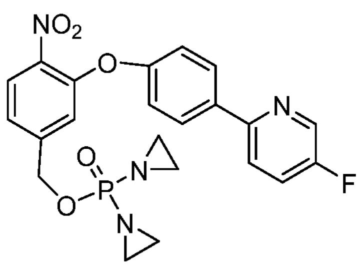
,
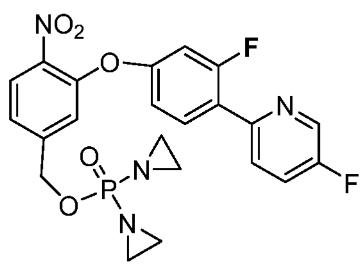
,

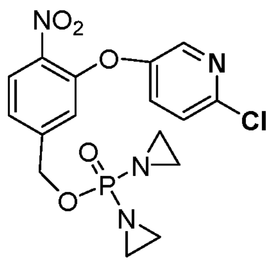, 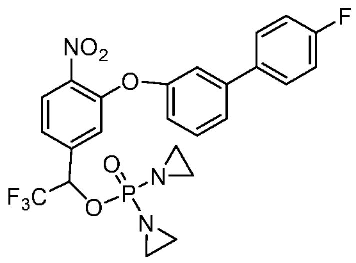, 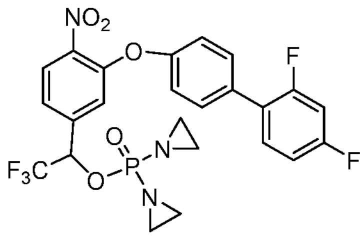,

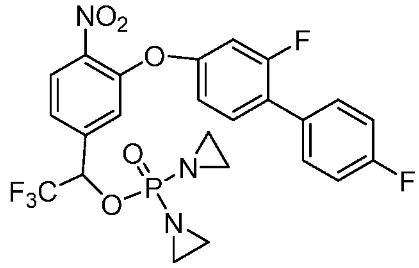, 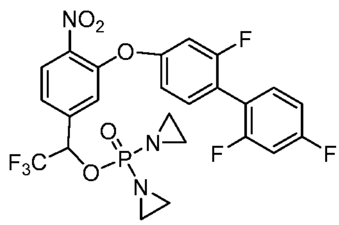,

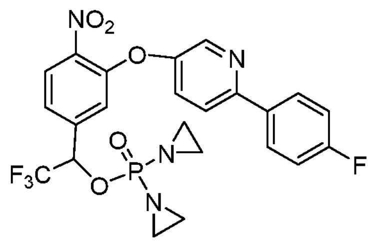, 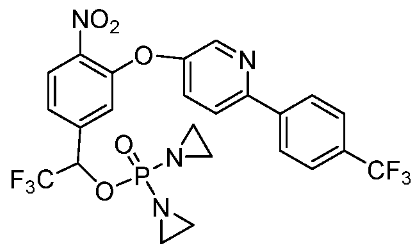, and

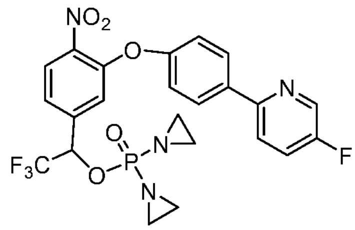

The compound in the present disclosure, wherein:

D-OH is selected from the anti-cancer drug containing -OH below: gemcitabine, estramusting, pudnimnstine, chlorozotocin, ranimustine, mannomustine, mitobronitol, dibromodulcitol, aclacinomycins, anthramycin, bleomycin, carubicin, doxorubicin, carzinophilin, chromomycin, dactinomycin, daunorubicin, mycophenolic acid, nogalamycin, olivomycin, peplomycin, plicamycin, puromycin, streptonigrin, streptozocin, tubercidin, ubenimex, zinostatin, zorubicin, denopterin, fludarabine, ancitabine, azacitidine, 6-azauridine, cytarabine, dideoxyuridine, doxifluridine, enocitabine, floxuridine, L-asparaginase, pulmozyme, aceglatone, elliptinium acetate, etoglucid, interferon-alpha, interferon-beta, interferon-gamma, interleukin-2, lentinan, mitoxantrone, mopidamol, pentostatin, pirarubicin, podophyllinic acid, sizofiran, paclitaxel, teniposide, tenuazonic acidm vinblastine, and vincristine;

D-$NR^{43}H$ is selected from the anti-cancer drug below: erlotinib, meturedepa, uredepa, imatinib, trimethylolomelamine, gefitinib, uracil mustard, carmustine, chlorozotocin, fotemustine, nimustine, ranimustine, dacarbazine, mannomustine, actinomycin, anthramycin, bleomycin, cactinomycin, carubicin, doxorubicin, carzinophilin, dactinomycin, peplomycin, puromycin, streptozocin, ubenimex, zinostatin, denopterin, pteropterin, trimetrexate, 6-mercaptopurine, thiamiprine, thioguanine, 6-azauridine, carmofur, dideoxyuridine, doxifluridine, enocitabine, floxuridine, 5-fluorouracil, tegafur, L-asparaginase, pulmozyme, amsacrine, bisantrene, demecolcine, diaziquone, elliptinium acetate, flutamide, hydroxyurea, interferon-alpha, interferon-beta, interferon-gamma, interleukin-2, mitoxantrone, nitracrine, pentostatin, phenamet, 2-ethylhydrazide, procarbazine, razoxane, erlotonib, urethane, vinblastine, and vincristine; and

the anti-cancer drug containing secondary or tertiary nitrogen is selected from: altretamine, triethylenemelamine,

chlorambuci, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, pipobroman, actinomycin, anthramycin, carzinophilin, dactinomycin, nogalamycin, porfiromycin, puromycin, streptozocin, tubercidin, fludarabine, ancitabine, azacitidine, cytarabine, dideoxyuridine, enocitabine, floxuridine, L-asparaginase, pulmozyme, aldophosphamide glycoside, bestrabucil, diaziquone, interferon-alpha, interferon-beta, interferon-gamma, interleukin-2, mitoguazone, mopidamo, nitracrine, pentostatin, phenamet, razoxane, spirogermanium, tamoxifen, triaziquone, 2,2',2"-trichlorotriethylamine, vinblastine, and vincristine.

Obviously, the drugs described above are only a partial list of the drugs on the market, and other drugs are also acceptable.

Furthermore, wherein the salt is a basic salt or an acid salt, and the solvate is a hydrate or an alcoholate.

A drug containing the compounds described above is further provided in the present disclosure.

The anti-tumor or anti-cancer drug with the compound described above is further provided in the present disclosure, wherein the tumor or the cancer includes:
lung cancer, non-small cell lung cancer (NSCLC), liver cancer, pancreatic cancer, gastric cancer, bone cancer, esophageal cancer, breast cancer, prostate cancer, testicular cancer, colorectal cancer, ovarian cancer, bladder cancer, cervical cancer, melanoma, squamous cell carcinoma, basal cell carcinoma, adenocarcinoma, carcinoma of sweat gland, carcinoma of sebaceous glands, papillary carcinoma, adenocarcinoma papillary, cystic adenocarcinoma, cystocarcinoma, medullary cancer, bronchial carcinoma, bone cell carcinoma, epicytoma, bile duct carcinoma, carcinoma chorionic, endoderm cancer, seminoma, Wilms tumor, gliocytoma, astrocytic glioma, medulloblastoma, craniopharyngeal duct tumor, ependymocytoma, pineal body tumor, hemocytoblastoma, vocal cord neuroma, meningioma, neuroblastic tumors, neuroblastoma of optic nerve, retinoblastoma, neurofibroma, fibroma sarcomatosum, desmocytoma, fibroma, fibroadenoma, fibrochondroma, fibrocystoma, fibromyxoma, osteofibroma, fibromyxosarcoma, fibropapilloma, myxomatous sarcoma, myxocystoma, myxochondroma, myxochondrosarcoma, myxochondrofibrosarcoma, myxoadenoma, myxoblastoma, liposarcoma, lipomata, lipoadenoma, lipoblastoma, lipochondroma, lipofibroma, lipoangioma, lipomyxoma, chondrosarcoma, chondrophyma, chondromatous myoma, chordoma, chorioadenoma, trichoepithelioma, chorioblastoma, osteosarcoma, osteoblastoma, osteochondrofibroma, osteochondrosarcoma, osteochondroma, osteocystoma, cementoma, osteofibroma, osteofibrous chondrosarcoma, hemangiosarcoma, angioma, angiolipoma, angiochondroma, hemangioblastoma, angioceratoma, angioglioma, hemangioendothelioma, hemangiofibroma, angiomyoma, angiolipoma, hematolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphlsarcoma, lymphangiofibroma, lymphocytoma, lymphepithelioma, lymphoblastoma, endothelioma, endoblastoma, synovioma, synovial sarcoma, mesothelioma, mesocytoma, Ewing's sarcoma, leiomyoma, leiomyosarcoma, leiomyoblastoma, leiomyofibroma, rhabdomyoma, rhabdomyosarcoma, rhabdomyomyxoma, acute lymphocytic leukemia, acute myelogenous leukemia, chronic disease cells, polycythemia, endometrial carcinoma, glioma, colorectal cancer, thyroid cancer, urothelial carcinoma or multiple myeloma.

The cancer or the tumor is a cancer or a tumor of the central nervous system.

Further, the cancer or the tumor is a primary brain cancer or a primary tumor, or a metastatic cancer or a metastatic tumor transferred to the brain.

Furthermore, the compound provided above, the salt is a basic salt or an acid salt.

About the compounds described herein, the compounds further include a salt of the structural formula I-1, i.e., a pharmaceutically acceptable salt of the compounds provided in the present disclosure. The salt may be a basic salt, including a salt formed by the compound with an inorganic base (e.g., an alkali metal hydroxide or an alkaline earth metal hydroxide) or a salt formed by the compound with an organic base (e.g., ethanolamine, diethanolamine or triethanolamine).

Besides, the salt may be an acid salt, including a salt formed by the compound with an inorganic acid (e.g., hydrochloric acid, hydrobromic acid, hydroiodic acid, nitric acid, perchloric acid, sulfuric acid, phosphoric acid) or a salt formed by the compound with an organic acid (e.g., methanesulfonic acid, trifloromethanesulfonic acid, ethylsulfonic acid, benzenesulfonic acid, p-toluenesulfonic acid, fumaric acid, oxalic acid, maleic acid, citric acid). The selection and preparation of the acceptable salts and solvates of the compounds are common techniques in the art.

Further, the compound provided in the present disclosure, wherein the solvate is a hydrate or an alcoholate.

The medical purpose of the anti-cancer compound I-1 for acting as a non-PGP substrate is to treat a cancer, a tumor or a cell histiocytosis caused by the cancer or the tumor:

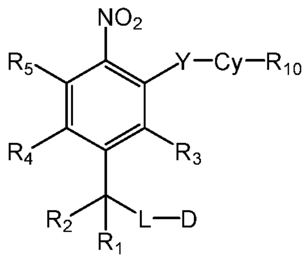

I-1

wherein, the definition of $R_1$, $R_2$, $R_3$, $R_4$, $R_5$, $R_{10}$, Cy, Y, L and D is the same as defined above.

The medical purpose is used to prepare the drug for treating a cancer, a tumor or a cell histiocytosis caused by the cancer or the tumor.

Preferably, the compound for the medical purpose is selected from:

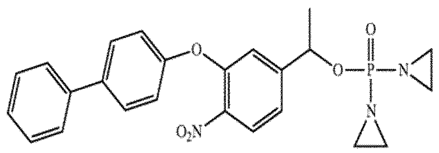 , 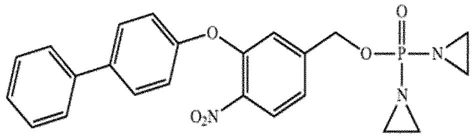 ,

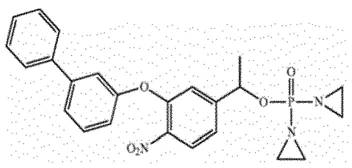 , 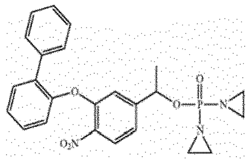 , 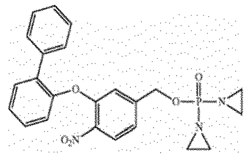 ,

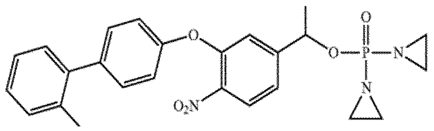 , 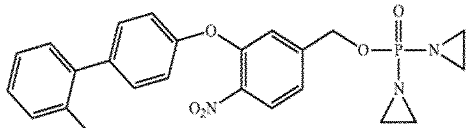 ,

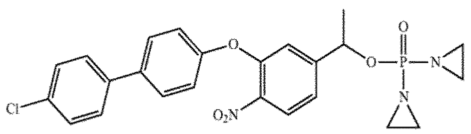 , 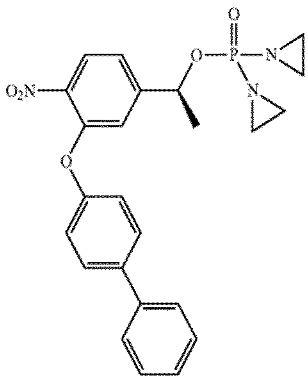 , 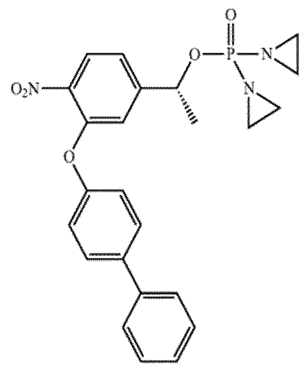 ,

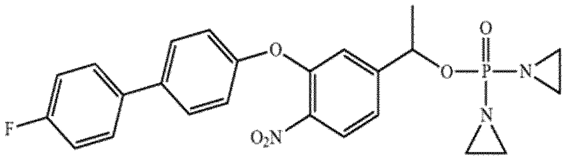 , 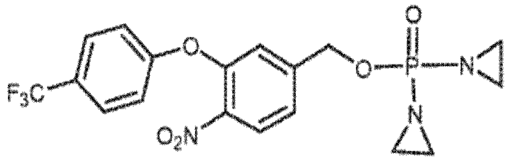 ,

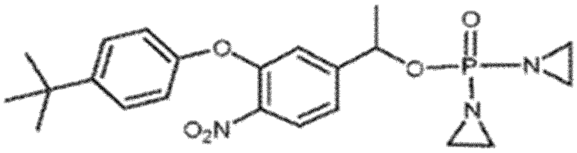,
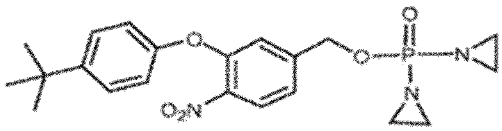,

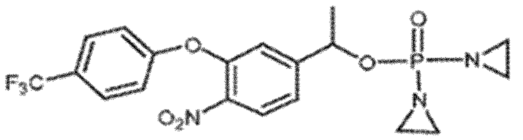,
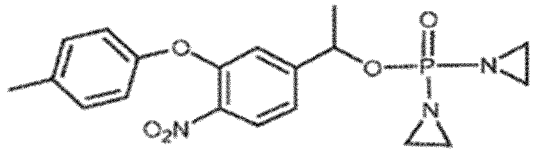,

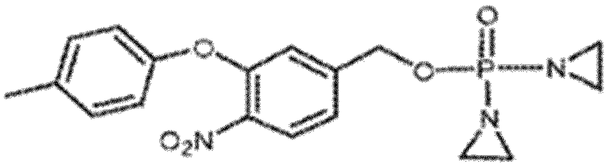,

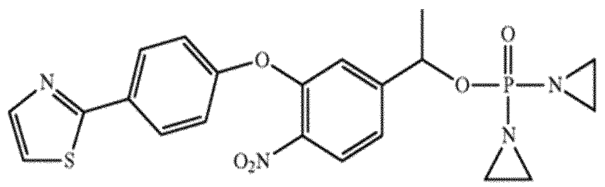,

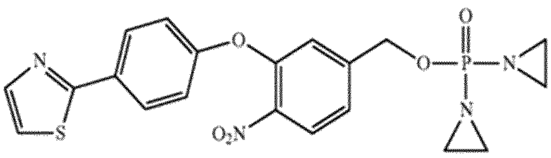,

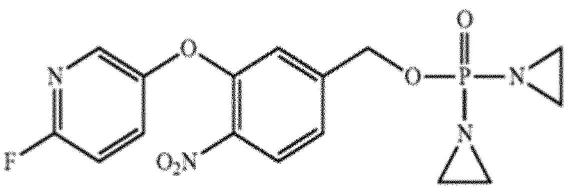,
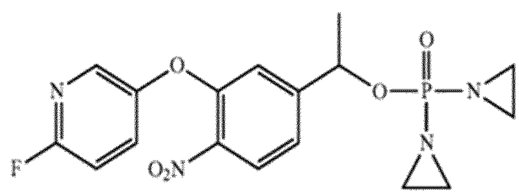,

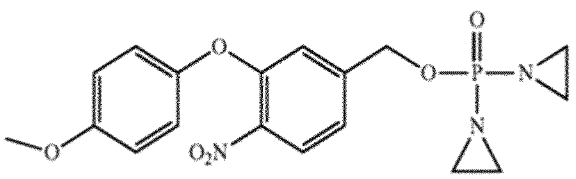,

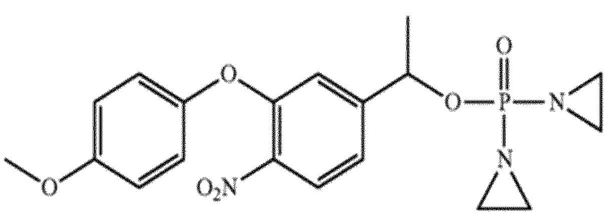,

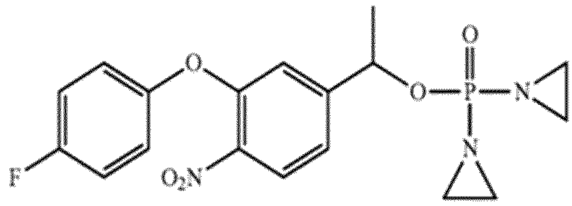

,

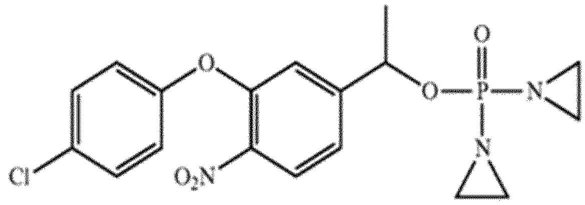

,

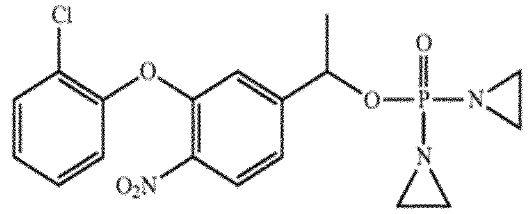

,

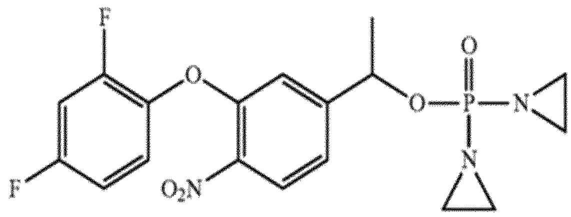

,

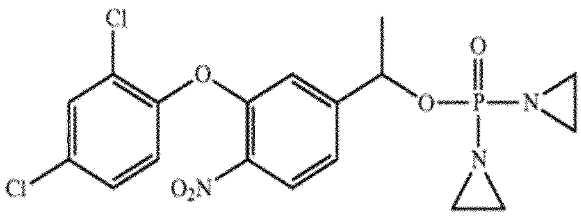

,

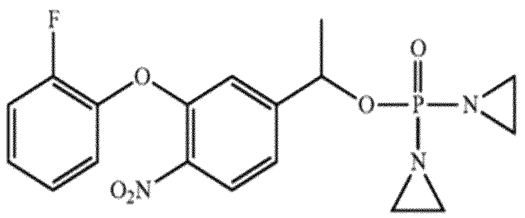

,

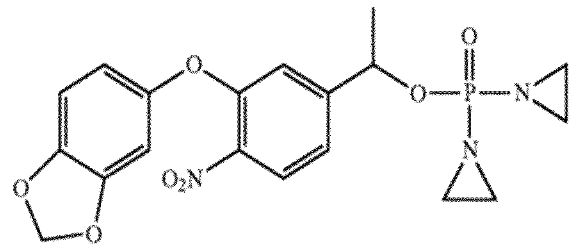

,

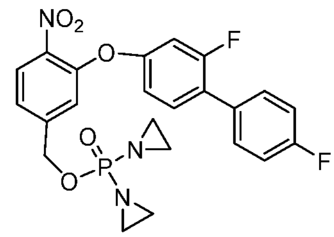

,

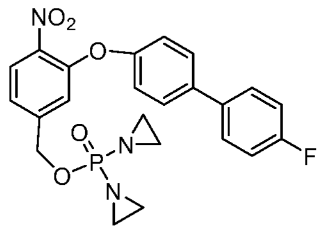 ,

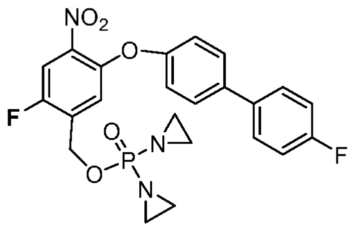 ,

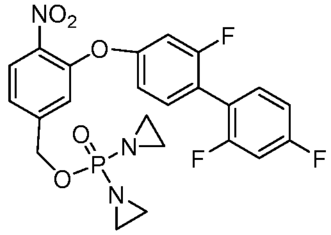 ,

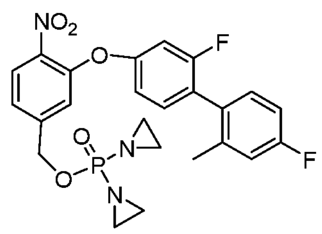 ,

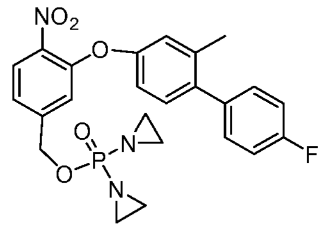 ,

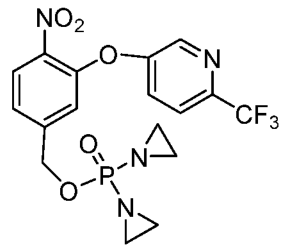 ,

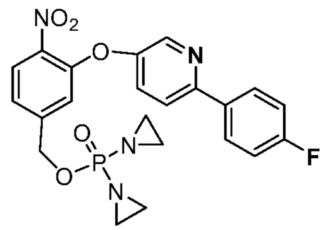 ,

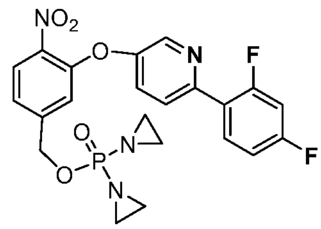 ,

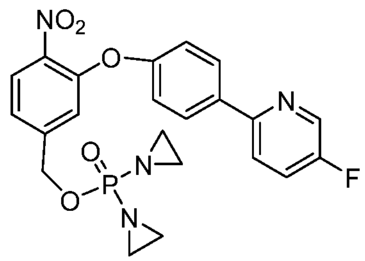 ,

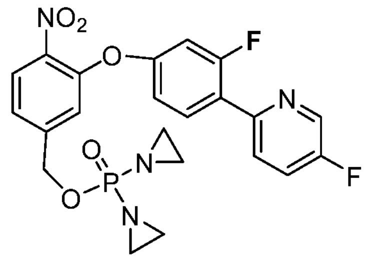 ,

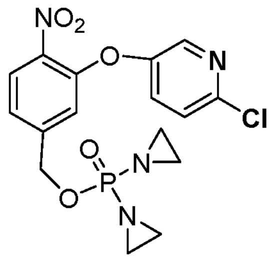 ,

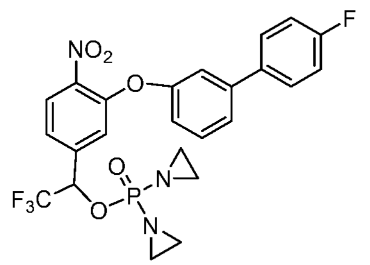 ,

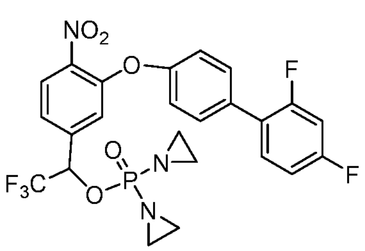 ,

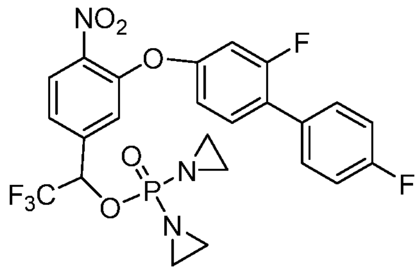 ,

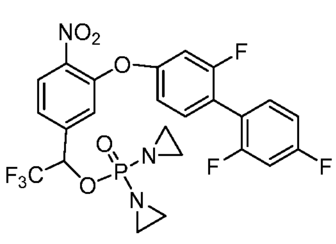 ,

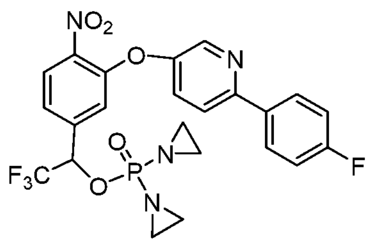 ,

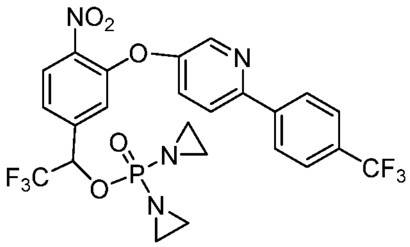 , or 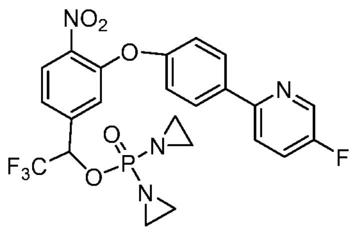 .

In the technical field of the drug design and organic chemistry, a compound (especially a compound contains an active group e.g., phosphoramide, amide, amine, salts or ester) has higher affinity to a solvent (e.g., water or some alcohol solvents, e.g., ethanol), a combination of the solvent and the compound (typically an inorganic salt, like copper sulfate hydrate) often occur. Especially, when the compound is a solid obtained from a solvent by crystallization, precipitation, or concentration etc., it will inevitably be combined with the solvent to obtain a solvate that is combined with the solvent and coated by the solvent. The compound provided in the present disclosure includes an active group of phosphoramide, amide, or hydroxyl, so as to naturally produce the corresponding solvate for the reason described above or the real situation.

The compounds described herein may also be used in the solvate's form, i.e., the pharmaceutically acceptable salt of the compound with the formula I provided in the present disclosure. The solvate is an hydrate or an alcoholate, and the alcoholate includes ethanolate.

With regard to the pharmaceuticals or formulations described herein, the prepared pharmaceuticals contain the indicated compounds or salts or solvates thereof in a specific dosage range, and/or the prepared pharmaceuticals are administered in a specific formulation and a way of administration.

In the use described herein, the prepared pharmaceuticals further include a pharmaceutically acceptable excipient. The pharmaceuticals may be in any formulations administered in clinical, such as a tablet, a suppository, a dispersible tablets, an enteric coated tablets, a chewable tablets, a fast disintegrating an oral tablet, a capsule, a sugar-coated tablet, a granules, a dusty agent, an oral solution, a small-volume injection, a lyophilized powder for injection, or a transfusion for injection. According to the specific formulations and the administration ways, the pharmaceutically acceptable excipients of the pharmacceuticals are one or more of a diluent agent, a hydrotropic agent, a disintegrating agent, a suspending agent, a lubricant, an adhesive, a filler, a corrigens, a sweetener, an antioxidant, a surfactant, an anticorrosive agent, an encapsulant, and a pigment etc. Preferably, the patient is a mammal. More preferably, the patient is a person.

A method of using a compound of any one of claims 1-22, or a pharmaceutically acceptable salt thereof, for treating an cancer, tumor expressing AKR1C3 enzyme or treating a disease or cell proliferative disease caused by the cancer, the tumor expressing AKR1C3 enzyme. The method includes administering an effective dose of the compound or a pharmaceutically acceptable salt thereof.

## DETAILED DESCRIPTION

The present disclosure will be described below with reference to specific examples. Those skilled in the art can understand that these examples are only for illustrating the present invention and do not limit the scope of the present disclosure in any way.

The experimental methods described in the following embodiments are normal methods if there is no special illustration. The raw materials of drugs and reagents are purchased products for commercial if there is no special illustration.

The definitions below are provided to help readers. All terms, symbols and other scientific or medical terms used herein are indicated to the meanings commonly understood by person having ordinary skill in the chemical and medical fields. In some situations, terms with commonly understood meanings are defined herein for clear explanation and/or for immediate reference. Additionally, definitions herein should not be explained to have material difference with the terms commonly understood in the art.

All indicated numerical values (e.g., pH, temperature, time, concentration, and weight, including the ranges thereof) are usually the approximation with the increment (+) or decrement (-) of 0.1, 1.0, or 10.0. All indicated numerical values are understood to have the term "about" in front. The agents herein are exemplary and the equivalents thereof are known in the art.

The term "$C_x$-$C_y$" or "$C_{x-y}$" in front of the group refers to the range of the number of the carbon atoms in the group. For example, $C_1$-$C_6$ alkyl group refers to the alkyl group with at least 1 and at most 6 carbon atoms.

The term "alkoxyl group" refers to an -O- alkyl group.

The term "amino group" refers to $NR^pR^q$, wherein $R^p$ and $R^q$ are independently hydrogen or a $C_1$-$C_6$ alkyl

group, or $R^p$ and $R^q$ and the nitrogen atom bonded thereto form a 4-15 membered heterocycle.

The term "aryl group" refers to the aromatic group with carbon atoms, a single ring (e.g., phenyl), or a multiple fused ring (e.g., naphthyl or anthryl) and without heteroatom. For the multicyclic ring system with fused, bridged, or spiro aromatic ring and non-aromatic ring but without heteroatom on the ring, the term "aryl" or "Ar" is acceptable when the attachment point is located in the carbon atom on the aromatic ring (e.g., 5,6,7,8-tetrahydronaphth-2-yl group is an aryl group due to the attachment point is located in the 2-position of aromatic phenyl group).

According to the detailed description of the present disclosure, $C_6$-$C_{10}$ aryl group may be a phenyl group, a naphthyl group, and any substituted phenyl groups and or naphthyl groups.

The term "heteroaryl group" refers to an aromatic group having 1-14 carbon atoms and 1-6 heteroatoms selected from oxygen, nitrogen, and sulfur and includes a single ring (e.g., imidazol-2-yl, and imidazol-5-yl) and a multicyclic ring system (e.g., imidazopyridyl, benzotriazolyl, benzimidazol-2-yl, and benzimidazol-6-yl). For the multicyclic ring system with aromatic ring and non-aromatic ring including fused ring, bridged ring and spiro ring, the term "heteroaryl" (e.g., 1,2,3,4-tetrahydroquinolin-6-yl and 5,6,7,8-tetrahydroquinolin-3-yl) is applied when at least one heteroatom is existed, and the attachment point is located at an atom on the aromatic ring. In some embodiments, the nitrogen and/or sulfur atom of the heteroaryl group is optionally oxidized to be a moiety of the N-oxide (N→O), sulfinyl or sulfonyl. The term "heteroaryl" or "5-15 membered heteroaryl" includes (but no limited thereto) acridinyl, azocinyl, benzimidazolyl, benzofuranyl, benzothiophenyl, benzoxazolyl, benzothiazolyl, benzotriazole, benzotetrazole, benzisoxazolyl, benzisothiazolyl, benzothienyl, benzimidazolinyl, carbazolyl, NH-carbazolyl, carbolinyl, chromanyl, chromenyl, cinnolinyl, dithiazinyl, furanyl, furazanyl, imidazolidyl, imidazolinyl, imidazopyridyl, imidazolyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolyl, isoxazolyl, naphthyridinyl, octahydroisoquinolinyl, oxadiazolyl, oxazolidinyl, oxazolyl, pyrimidinyl, phenanthridinyl, phenanthrolinyl, phenazinyl, phenothiazinyl, phenoxathiinyl, phenoxazinyl, phthalazinyl, hexahydropyrazinyl, pteridinyl, purinyl, pyranyl, pyrazinyl, pyrazolidinyl, pyrazolinyl, pyrazolyl, azosinyl, pyridoxazolyl, pyrido-oxazolyl, pyrido-imidazolyl, pyrido-thiazolyl, pyridinyl, pyridyl, pyrrolyl, quinazolinyl, quinolyl, quinolinyl, quinuclidinyl, tetrahydroisoquinolyl, tetrahydroquinolyl, tetrazolyl, thiadiazinyl, thiadiazolyl, thianthranyl, thiazolyl, thienyl, thienothiazolyl, thieno-oxazolyl, thieno-imidazolyl, triazinyl, xanthenyl.

The term "alkyl group" refers to an aliphatic group with carbon atoms. In some embodiments, alkyl group refers to univalent aliphatic group with 1-6 carbon atoms. The term "$C_{x-y}$ alkyl" refers to an alkyl group with x-y carbon atoms. The term "alkyl" includes linear hydrocarbyl group and branched hydrocarbyl group. For example, methyl ($CH_3$-), ethyl ($CH_3CH_2$-), n-propyl ($CH_3CH_2CH_2$-), isopropyl ($(CH_3)_2CH$-), n-butyl ($CH_3CH_2CH_2CH_2$-), isobutyl ($(CH_3)_2CHCH_2$-), sec-butyl ($(CH_3)(CH_3CH_2)CH$-), tert-butyl ($(CH_3)_3C$-), n-pentyl, and neopentyl.

The term "cycloalkyl group" refers to saturated or unsaturated cyclic group with at least 3 carbon atoms and without heteroatom, and the cyclic group includes a single ring, a fused ring, a bridged ring and a spiro ring. For the multicyclic ring system with aromatic ring and non-aromatic ring and without heteroatom, the term "cycloalkyl" is applied when the attachment point is located at the carbon on the non-aromatic ring (e.g., 5,6,7,8-tetrahydronaphth-5-yl). The term "cycloalkyl" or "$C_3$-$C_8$ cycloalkyl" includes cycloalkenyl e.g., adamantlyl, cyclopropyl, cyclobutyl, cyclopentyl, cyclooctyl and cyclohexenyl.

The term "heterocyclic", "heterocycle", "heterocycloalkyl group" or "heterocyclyl group" refers to a saturated or unsaturated cyclic group with carbon atoms and 1-6 heteroatoms selected from nitrogen, oxygen, and sulfur, and the cyclic group includes a single ring, a fused ring, a bridged ring and a spiro ring. For a multicyclic ring system with a aromatic ring and a non-aromatic ring and without heteroatom, the term "heterocyclic", "heterocycle", "heterocycloalkyl group" or "heterocyclyl group" is applied when the attachment point is located at the atom on the non-aromatic ring (e.g., 1,2,3,4-tetrahydro-quinolin-3-yl, 5,6,7,8-tetrahydro-quinolin-6-yl, and decahydroquinolin-6-yl). In some embodiments, heterocyclyl group is 3-15 membered, 4-14 membered, 5-13 membered, 7-12 membered or 5-7 membered heterocycle. In some other embodiments, heterocycle contains 4 heteroatoms. In some other embodiments, heterocycle contains 3 heteroatoms. In some other embodiments, heterocycle contains 2 heteroatoms at most. In some embodiments, the nitrogen and/or sulfur atom of heterocycloalkyl group is oxidized to be a moiety of the N-oxide, sulfinyl or sulfonyl. The term "heterocyclyl group" includes (but no be limited thereto) tetrahydrofuranyl, piperidyl, N-methyl-piperid-3-yl, hexahydropyrazinyl, N-methyl-pyrrolidin-3-yl, pyrrolidin-3-yl, 2-pyrrolidon-1-yl, morpholinyl and pyrrolidinyl. The numerical multiplier of the indicated number of carbon atoms (e.g., $C_3$-$C_{10}$) refers to the total number of the carbon atoms excepts for the number of the heteroatoms of the moiety of heterocyclyl group. The divalent heterocyclyl group contains the appropriate content of hydrogen.

The term "ether" refers to $C_1$-$C_6$ alkyl group with 1-3 substituted $C_1$-$C_6$ alkoxyl group. Alkoxyl group refers to -O-alkyl.

The term "halogen" or "halogeno" refers to one or more of fluorine, chlorine, bromine and iodine.

The term "alkenyl group" refers to a linear hydrocarbyl group or a branched hydrocarbyl group with carbon atoms. For example, the alkenyl group refers to a linear hydrocarbyl group or a branched hydrocarbyl group with 2-6 or 2-4 carbon atoms and with at least one unsaturated position of vinyl (>C=<). For example, the term "$C_{x-y}$ alkenyl" refers

to an alkenyl group with x-y carbon atoms. The term "$C_{x-y}$ alkenyl" is vinyl, allyl, 1,3-butadienyl etc.

The term "alkynyl group" refers to a linear univalent hydrocarbyl group or a branched univalent hydrocarbyl group, containing at least a triple bond, with more than two carbon atoms, and in some embodiments with 2-6 or 2-4 carbons. The term "alkynyl group" also refers to these alkyl group with a triple bond and a double bond. For example, the term "$C_{2-6}$ alkynyl" includes ethynyl and propynyl etc.

The term "aminophosphate ester alkylating agent" refers to an alkylating agent having at least one $Z^5$-$X^5$-$Y^5$ bonded to -O-P(Z1). In $Z^5$-$X^5$-$Y^5$, $Z^5$ is a heteroatom, such as nitrogen, sulfur, or oxygen; $X^5$ is an optionally-substituted ethylene group; $Y^5$ is a halogeno or another leaving group; or $Z^5$-$X^5$-$Y^5$ forms a moiety of $NCH_2CH_2$, and $Z^1$ is as defined above. The alkylating agent may react with a DNA, another nucleic acid, or a protein. In some situations, the alkylating agent may cross-link DNA.

The group may be substituted by one or more substituents (e.g., 1, 2, 3, 4, or 5 substituents). Preferably, the substituent is selected from the group consisting of an oxo group, a halogeno, -CN, $NO_2$, $-N_2^+$, $-CO_2R^{100}$, $-OR^{100}$, $-SR^{100}$, $-SOR^{100}$, $-SO2R^{100}$, $-NR^{100}SO_2R^{100}$, $-NR^{101}R^{102}$, $-CONR^{101}R^{102}$, $-SO_2NR^{101}R^{102}$, alkyl, a alkoxyl group, $-CR^{100}=C(R^{100})_2$, $-CCR^{100}$, a $C_3$-$C_{10}$ cycloalkyl group, a $C_3$-$C_{10}$ heterocyclyl group, and the divalent substituent e.g., $-O-(CH_2)-O-$ or $-O-(CH_2)_2-O-$, wherein the divalent substituent is unsubstituted or substituted by 1-4 methyl, wherein $R^{100}$, $R^{101}$ and $R^{102}$ is respectively independently hydrogen, a$C_1$-$C_8$ alkyl group, a $C_3$-$C_{12}$ cycloalkyl group,a $C_3$-$C_{10}$ heterocyclyl group, a $C_6$-$C_{12}$ aryl group, and a$C_2$-$C_{12}$ heteroaryl group, or $R^{100}$ and $R^{102}$ and the nitrogen atom bonded thereto form 5-7 membered heterocycle; wherein the alkyl group, cycloalkyl group, aryl group and heteroaryl group are respectively optionally substituted by 1-3 halogeno, 1-3 $C_1$-$C_6$ alkyl group, 1-3 $C_1$-$C_6$ haloalkyl group or 1-3 $C_1$-$C_6$ alkoxyl group. Preferably, the substituent is selected from the group consisting of chlorine, fluorine, $-OCH_3$, methyl. ethyl, iso-propyl, cyclopropyl, $-CO_2H$ (and a salt thereof), $C_1$-$C_6$ alkyl ester, $CONMe_2$, CONHMe, $CONH_2$, $-SO_2Me$, $-SO_2NH_2$, $-SO_2NMe_2$, $-SO_2NHMe$, $-NHSO_2Me$, $-NHSO_2CF_3$, $-NHSO_2CH_2Cl$, $-NH_2$, $-OCF_3$, $-CF_3$ and $-OCHF_2$.

The term "alkylene" refers to a divalent saturated aliphatic group having carbon atoms. In some embodiments, the alkylene refers to a divalent aliphatic group with1-6 carbon atoms and loses one more hydrogen atom. The term "$C_{u-v}$ alkylene" refers to an alkylene with from u to v carbon atoms. The term "alkylene" includes a linear hydrocarbyl group or a branched hydrocarbyl group. For example, the alkylene includes methylene, ethylene, propylene, 2-methyl propene, and amylene etc.

The term "heteroalkylene" refers to the carbon atom on alkylene chain is substituted by a heteroatom (e.g., O, S, N, or P) or substituted by a substituent containing a heteroatom.

The term "drug" used herein with respect to D includes (but not limited thereto) gemcitabine, erlotinib, meturedepa, uredepa, altretamine, imatinib, triethylenemelamine, 2,4,6-trimethylmelamine, chlorambucil, chlornaphazine, estramustine, gefitinib, afatinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, mannomustine, mitobronitol, mitolactol, pipobroman, aclacinomycins, actinomycin, anthramycin, azaserine, bleomycin, cactinomycin, carubicin, doxorubicin, carzinophilin, chromomycin, dactinomycin, daunorubicin, daunomycin, 6-diazo-5-oxo-L-norleucine, mycophenolic acid, nogalamycin, olivomycin, peplomycin, plicamycin, porfiromycin, puromycin, streptonigrin, streptozocin, tubercidin, ubenimex, zinostatin, zorubicin, denopterin, pteropterin, trimetrexate, fludarabine, mercaptopurine, thiamiprine, tioguanine, ancitabine, azacitidine, 6-azauridine, carmofur, cytarabine, dideoxyuridine, doxifluridine, enocitabine, floxuridine, fluorouracil, tegafur, L-asparaginase, pulmozyme, aceglatone, aldoisophosphamide glycoside, aminolevulinic acid, amsacrine, bestrabucil, bisantrene, defofamide, demecolcine, diaziquone, elfornithine, elliptinium acetate, etoglucid, flutamide, hydroxyurea, interferon-a, interferon-β, interferon-y, interleukin-2, lentinan, mitoguazone, mitoxantrone, mopidamol, nitracrine, pentostatin, phenamet, pirarubicin, podophyllinic acid, 2-ethylhydrazide, procarbazine, razoxane, sizofiran, spirogermanium, paclitaxel, tamoxifen, erlotonib, teniposide, tenuazonic acid, trisethyleneiminoquinone, 2,2',2"-triclorotriethylamine, urethane, vinblastine, and vincristine.

The term "administering" or "applying" a drug refers to directly administering or applying a drug (which may be administered or applied to a patient by a medical professional or maybe self-administered or applied) and/or indirectly administering or applying a drug, which may be a drug-prescribing behavior. For example, a physician instructing a patient to self-administer or administer a drug and/or providing a prescription for the drug to the patient is administering or applying the drug to the patient.

The term "cancer" refers to the potentially unlimited growth leukemia, lymphoma, cancers and other malignant tumors (including solid tumors) which are partially expanded via the invasion and fully expanded via the metastasis. Examples for the cancer includes (but not be limited thereto) the cancer of the adrenal gland, bone, brain, breast, bronchus, colon, rectum, gallbladder, head, neck, kidney, throat, liver, lung, nervous tissue, pancreas, prostate, parathyroid, thyroid, skin, and stomach. Some other embodiments of the cancer include acute and chronic lymphocytic and granulocytic tumors, adenocarcinoma, adenoma, basal cell carcinoma, cervical intraepithelial neoplasia, cancer in situ, Ewing's sarcoma, epidermoid carcinoma, giant cell tumor, multiform neuroglioblastoma, hair cell tumor, intestinal ganglioma and proliferative corneal nerve tumor, islet cell carcinoma, Kaposi's sarcoma, leiomyoma, leukemia, lymphoma, malignant carcinoid tumor, malignant melanoma, malignant hypercalcemia, equine tumor, myeloid epithelial cancer,

metastatic skin cancer and mucosal neuroma, myeloma, mycosis fungoides, neuroblastoma, osteosarcoma, osteogenic and other sarcoma, ovarian tumor, pheochromocytoma, polycythemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesions, reticular cell sarcoma and Wilm's tumor.

The terms "patient" and "individual" may be exchanged and refer to a mammal in need of the cancer therapy. Generally, the patient refers to a human. Generally, the patient refers to a human diagnosed with cancer. In some embodiments, the terms "patient" and "individual" refer to a non-human mammal that may be used to select, characterize, and evaluate the drug and therapy. For example, the non-human mammal is the non-human primates, dog, cat, rabbit, pig, mouse, or rat.

The term "prodrug" refers to a compound transformed via metabolism or other ways to a biologically active or more active compound (or a drug) after administering or applying. Relative to the drug, the prodrug is chemically modified in such a way that it is less active or inactive relative to the drug, but the chemical modification results in the production of the corresponding drug through metabolism or other biological processes after administration of the prodrug. Relative to the active drug, prodrugs may have altered metabolic stability, transport characteristics, fewer side effects or lower toxicity, or improved flavor. Please refer to the reference documents, such as Nogrady (1985) Medicinal Chemistry A Biochemical Approach, Oxford University Press, New York, pp. 388-392, which is entirely incorporated herein by reference. On the other hand, the prodrug may be synthesized by a reactant other than the corresponding drug.

The term "solid tumor" refers to (but is not limited thereto) a solid tumor of a metastatic tumor in the bone, brain, liver, lung, lymph glands, prostate, skin, and sarcoma soft tissue.

The term " cancer or tumor of the central nervous system" refers to the benign tumor or the malignant tumor (cancer) in the skull or other central nervous systems, including meningioma, hypophysoma, craniopharyngioma, neurinoma, glioma, ependymoma, primitive neurectodermal tumor, lymphoma of the central nervous system, germ cell tumor, and metastatic tumor.

The term "therapeutically effective dose" of the drug refers to the amount of the drug having the expected therapeutic effect (e.g., the relief, improvement, ease, or elimination of one or more cancers in the patient) when administered or applied to a patient having a cancer. The therapeutic effect does not necessarily have to occur by administering or applying a single dose and may only occur by administering or applying a series of doses. Therefore, the therapeutically effective dose may be administered or applied once or repeatedly.

The term "therapy" of a condition of an illness or a patient refers to obtain beneficial or expected results (including the clinical outcome) by adopting steps. For the purpose of the present disclosure, the beneficial or expected results include (but is not limited thereto) the relief or improvement of one or more cancer symptoms, the weakening of the disease degree, the delay or ease of the disease course, the improvement, relief or stability of the disease state, or other beneficial results. In some cases, the therapy of the cancer may be used to partially react to the cancer or to stabilize the disease.

The term "tumor cell" refers to the tumor of any suitable species, such as mammals, e.g., the mouse, rat, dog, cat, horse, or human.

The above description of the specific embodiments of the present disclosure does not limit the present disclosure, and those skilled in the art can make various changes or deformations according to the present disclosure.

The present disclosure is based on the three following applications:

(1) The patent application no. PCT/US2016/021581 and the patent publication no. WO2016/145092 correspond to the China patent application no. 2016800150788 and the China patent publication no. CN107530556A.
(2) The patent application no. PCT/US2016/025665 and the patent publication no. WO2016/061342 correspond to the China patent application no. 2016800200132 and the China patent publication no. CN108136214A.
(3) The patent application no. PCT/US2016/062114 and the patent publication no. WO2017/087428 correspond to the China patent application no. 2016800446081 and the China patent publication no. CN108290911A.

Hence, the above three applications are hereby incorporated by reference into the text of the present application.

The experiments and embodiments of the present disclosure are shown as follows.

The experiments of the inhibitory activity of the proliferation of the cancer cells in vitro, and the experimental data of the interaction of P-GP glycoprotein of the compounds designed and synthesized by the applicant are disclosed below. Hereby declared, the right of the following experimental data is belonged to the applicant.

P-glycoprotein (P-GP, also known as multidrug resistance protein), which is a high-molecular-weight protein discovered on the plasma membrane of the multidrug-resistant tumor cell, has a transport-pump-like structure. P-GP pumps out a variety of chemotherapy drugs out of cells and reduces intracellular drug concentration. Therefore, the P-GP is closely related to the resistance in clinical chemotherapy.

The role of the chemotherapy drug for treating the tumor or cancer of the central nervous system (such as the brain) is limited. The limitation is mainly due to the blood-brain barrier (BBB), the low permeability of the chemotherapy

drug to the tumor system, or the tumor tissue caused by BBB, i.e., the chemotherapy drug may not eliminate the cancer cells by passing through the blood-brain barrier and entering the brain.

The process that the small molecules (e.g., the chemotherapy drug) pass through the blood-brain barrier is more complicated. The blood-brain barrier, located between the systemic blood circulation and the cerebrospinal fluid, is formed by specialized brain microvascular endothelial cells, along with surrounding cells and perivascular astrocytes through tight junctions between adjacent cells when most molecules are forced to pass through, rather than forming a physical barrier around vascular endothelial cells.

The small hydrophilic molecules are allowed to pass through the membrane transportation system of the BBB, whereas the bulky hydrophilic molecules, such as many chemotherapy drugs and macromolecular drugs, are excluded from the central nervous system unless they may be actively transported by specific proteins. More particularly, the "efflux pumps" of BBB for protecting the brain tissue (e.g., P-gp) may actively exclude some chemotherapy drugs and bulky molecular drugs.

Therefore, even that the small hydrophilic molecular chemotherapy drugs, including small molecule targeted antitumor drug molecule, may pass through the blood-brain barrier and enter the brain to play a role. However, the small hydrophilic molecular chemotherapy drugs may not work because they are excluded from the central nervous system under the action of the P-GP. In other words, the transmembrane structure of P-GP has the function of the energy dependent "drug pump," which may pump out the hydrophobic lipophilic drugs (e.g., VCR, Dox, and VP-16) and decrease the intracellular drug concentration, so that the cytotoxicity is decreased or complete loss of cytotoxicity.

For the reasons described above, the experimental data of the interaction between P-GP and the compound is used to evaluate the effective degree of the compound on the inhibitory activity of the proliferation of the tumor cells in the central nervous system.

The applicant declares that some of the specific compounds disclosed in the following experiments can be synthesized based on the specific synthetic methods and synthetic routes of the compounds disclosed in the present invention, with reference to similar methods and operations disclosed in patent publications (the patent application no. PCT/US2016/021581 and the patent publication no. WO2016/145092 correspond to the China patent application no. 2016800150788 and the China patent publication no. CN107530556A; the patent application no. PCT/US2016/025665 and the patent publication no. WO2016/061342 correspond to the China patent application no. 2016800200132 and the China patent publication no. CN108136214A; the patent application no. PCT/US2016/062114 and the patent publication no. WO2017/087428 correspond to the China patent application no. 2016800446081 and the China patent publication no. CN108290911A) or other publications. And the applicant has confirmed the structures by NMR and mass spectrometry.

**1. The evaluation of the potential of the sample as the P-gp substrate, which took MDCKII-MDR1 cells as the model and Verapamil as the inhibitor.**

**The preparation of the bacteria for the penetration test:**

(1) MDCKII-MDR1 cells were cultured in the cell culture bottle. The conditions of the incubator were 37°C, 5% of $CO_2$, and 95% of the relative humidity. When the cell growth confluence was up to 70-90%, the cells were inoculated to the transwell.

(2) Before the cell inoculation, 50 μL of the culture medium was respectively added to every well of the upper compartment of the transwell, and 25 mL of the culture medium was added to the lower compartment. The transwell was incubated in the incubator under 37°C and 5% $CO_2$. After 1 hour, the transwell may be used to inoculate the cells.

(3) After the cells were incubated, the cell suspension was transferred to a round bottom centrifuge tube, and the cell suspension was centrifuged at 120 g for 5 min.

(4) Cells were resuspended in the culture medium, and the final concentration was $1.56\times10^6$ cells/mL. The cell suspension was transferred to the upper compartment of the 96-well transwell, wherein each upper compartment contains 50 μL of the cell suspension. The final inoculum density was $5.45\times10^5$ cells/$cm^2$.

(5) The culture medium was replaced after inoculating for 48 h. After culturing for 4-8 days, the medium was changed every other day.

(6) The process of replacing the culture medium was as the follow. The upper compartment and the lower compartment were separated. The medium in the lower compartment was discarded, and then the medium in the upper compartment was discarded. Finally, 75 μL of fresh medium was added to each upper compartment and 25 mL of fresh medium was added to the lower compartment.

**Evaluation of the cell monolayer integrity**

(1) The MDCKII-MER1 cells should be totally confluent and complete the differentiation after culturing for 4-8 days. The MDCKII-MER1 cells can be used for the penetration test at this time.
(2) Millipore was used to measure the resistance of the cell monolayer, and the resistance of every well was recorded.
(3) After the measuring, the transwell was put back to the incubator.
(4) The calculation of the resistance: measured resistance (ohms) × area of the membrane = TEER value (ohm·cm$^2$). If TERR value was < 42 ohm·cm$^2$, the well would not be used to the penetration test.

**Penetration test of the drug**

(1) The transwell of the MDCKII-MER1 cells was taken out from the incubator. The cell monolayer was rinsed two times by the buffer solution and incubated at 37°C for 30 min.
(2) The transport rate of the compound from the top to the bottom was tested. 75 μL of the buffer solution contained the sample was added to each upper compartment, and 235 μL of the buffer solution was added to each lower compartment.
(3) The transport rate that the compound was transported from the bottom to the top was tested. 75 μL of the buffer solution was added to each upper compartment, and 235μL of the buffer solution contained the sample was added to each lower compartment.
(4) 50 μL of the sample was transferred to the acetonitrile containing the internal standard (100 nM Alprazolam, 200 nM Labetalol, 200 nM caffeine and 2 μM Ketoprofen), and the mixture was as the dosing sample at 0 min.
(5) For testing the transportation that the sample was under the condition that the P-GP inhibitor Verapamil was added. Verapamil was both added to the buffer salt of the dosing end and the receiving end of the transwell contained MDCKII-MDR1 cells, and the final concentration was 100 μM.
(6) The transportation of the up and down were merged up, the sample was incubated at 37°C for 2 h.
(7) After the incubation was finished, 50μL of the samples from each well of the upper compartment and the lower compartment were respectively added to the sample tubes. 200 μL of the acetonitrile containing the internal standard (100 nM Alprazolam, 200 nM Labetalol, 200 nM caffeine and 2 μM Ketoprofen) was added to each sample tube. After the sample tubes were vortexed for 10 min, the sample tubes were centrifuged at 3220 g for 30 min. 100 μL of the supernatant was taken to be diluted with the same volume of the water, and the LC-MS/MS analysis was executed. Each sample was adopted to the three parallel incubation.
(8) After the cell monolayer was incubated for 2 h, the integrity of the cell monolayer was evaluated by the migration assay using the fluorescence yellow, the fluorescence yellow storage solution was diluted by the buffer solution, and the final concentration was 100 μM/L. 100 μL of the fluorescence yellow solution was added to each well of the upper compartment, and 300 μL of the buffer was added to each well of the lower compartment. After the transwell was incubated at 37°C for 30 min, 80 μL of the solution from each well of the upper compartment and the lower compartment was respectively transferred to a new 96-well plate. ELISA (Enzyme-linked Immunosorbent Assay) reader was used to perform the fluorimetry with the excitation wavelength of 480 nm and the emission wavelength of 530 nm.

**Data analysis**

The peak area was calculated by the result of the ion chromatography. The permeability coefficient (Papp, unit: cm/s×10$^{-6}$) of the compound was calculated by the formula:

$$P_{app} = \frac{V_A}{Area \times time} \times \frac{[drug]_{acceptor}}{[drug]_{initial,donor}}$$

In the formula, "$V_A$" refers to the volume of the solution on the acceptor (Ap→Bl was 0.235 mL, Bl→Ap was 0.075 mL), *"Area"* refers to the area of the permeable membrane of the 96-well transwell (0.143 cm$^2$), *"time"* refers to the incubation time (s), "$[drug]_{acceptor}$" refers to the drug concentration of the acceptor after the incubation, and "$[drug]_{initial,donor}$" refers to the initial concentration of the drug administration before the incubation.
The efflux ratio was calculated by the formula:

$$Efflux\ Ratio = \frac{P_{app\ (B-A)}}{P_{app\ (A-B)}}$$

In the formula, "$P_{app\ (B-A)}$" refers to the permeability coefficient from the lower compartment to the upper compartment, and the term "$P_{app\ (A-B)}$" refers to the permeability coefficient from the upper compartment to the lower compartment.

The recovery rate was calculated by the formula:

$$Recovery\ \% = \frac{[drug]_{acceptor} \times V_A + [drug]_{donor} \times V_D}{[drug]_{initial,donor} \times V_D} \times 100$$

In the formula, "$V_A$" refers to the volume of the solution on the acceptor (mL), "$V_D$" refers to the volume of the solution on the donor (mL), "$[drug]_{acceptor}$" refers to the drug concentration of the acceptor after the incubation, "$[drug]_{donor}$" refers to the drug concentration of the donor after the incubation, and the term "$[drug]_{initial,donor}$" refers to the initial concentration of the drug administration before the incubation.

The LY Leakage of the cell monolayer was calculated by the formula:

$$\text{LY Leakage} = \left(\frac{I_{acceptor} \times 0.3}{I_{acceptor} \times 0.3 + I_{donor} \times 0.1}\right) \times 100$$

In the formula, "$I_{acceptor}$" refers to the fluorescence intensity of the acceptor (0.3 mL), "$I_{donor}$" refers to the fluorescence intensity of the donor (0.1 mL), expressed by %LY. LY<1.5% refers to the cell monolayer was intact.

The efflux ratios of different compounds that were in the presence of or in the absence of P-glycoprotein inhibitor, Verapamil, were obtained after respectively testing a part of the compounds.

The efflux ratios of Metoptolol, Prazosin and Imatinib were also tested as the control groups.

There were two efflux ratios of one compound (in the absence of Verapamil and in the presence of Verapamil). The closer these two values were, the less effective the P-GP on the drug, i.e., the drug was not the substrate of the P-GP and could enter the blood-brain barrier.

**2. The evaluation of the inhibitory effect of the prepared compounds to the proliferation of the cancer cells.**

The cytotoxicity analysis of the human tumor cell lines in vitro was used.

The report of the proliferation of the non-small cell lung cancer (NSCLC) human tumor line H460 in vitro was shown in the below table of the compounds.

IC50 (half maximal inhibitory concentration) values were in nanomoles and were obtained by the following. Cells were exposed in the compounds with different concentration for 2 hours. The compounds were washed off, and fresh medium was added into plates. A growth rate and a cell-survival rate were obtained by cell staining and compared with a control group treated with the medium only.

Particularly, exponentially growing cells were inoculated in a 96-well plate for 24 h and under the conditions of 37°C, 5% $CO_2$, 95% air and 100% relative humidity, and then the tested compound was added. The compound was dissolved in 100% DMSO at 200 times of the desired final test concentration. When the drug was added, the compound was further diluted at 4 times of the desired final test concentration by the complete medium. 50 μL aliquots of the compound with specific concentration were added to the microwells contained 150 μL of medium, and then the final drug concentration was obtained. After the drug was added, the plate was further incubated for 2 h under the conditions of 37°C, 5% $CO_2$, 95% air and 100% relative humidity, then the drug was washed, and the fresh medium was added., and then the plate was further incubated for 70 h under the conditions of 37°C, 5% $CO_2$, 95% air and 100% relative humidity. After the incubation was finished, the Alamar Blue was used to analyze and quantify the alive cells. The 50% inhibitory concentration ($IC_{50}$) caused by the drug was calculated by the computer software and the result was shown in the table below.

Similarly, to further prove that the compound was activated by the AKR1C3 (the member C3 of the aldo-keto reductase family 1), the proliferation test of some compounds on the cancer cells H460 was carried out in the presence of the specific AKR1C3 inhibitor (3 μmol concentration). Before 2 h of the compound was prepared, the compound with the inhibitor was added to the cell culture. The inhibitor was

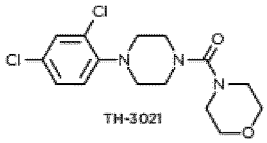

TH-3021

by reference to Flanagan et al., compound 36 in Bioorganic and Medicinal Chemistry (2014) pp. 962-977.

| Number | compound | Efflux ratio (MDCK-MDR1 ) | | Inhibitory effect of the proliferation of the cancer cells | |
|---|---|---|---|---|---|
| | | Without Verapamil | With Verapamil | $IC_{50}$ (nM) | $IC_{50}$ of +TH3021 (nM) |
| 1 | 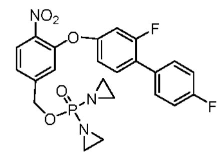 | 1 | 0.57 | 0.87 | 40 |
| 2 | 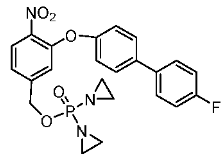 | 1.53 | 0.46 | 1.89 | 105.4 |
| 3 | 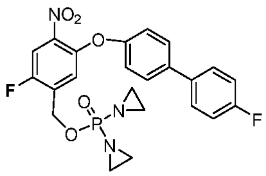 | 1.2 | 0.59 | 1.84 | 47.78 |
| 4 | 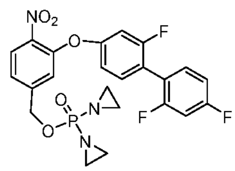 | 0.87 | 0.59 | 0.67 | 16.34 |
| 5 | 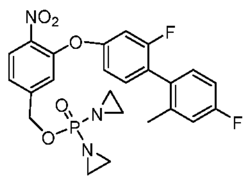 | 1.69 | 0.61 | 0.59 | 5.037 |
| 6 | 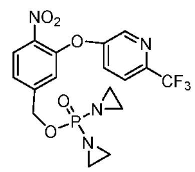 | untested | untested | 0.46 | 38.23 |
| 7 | 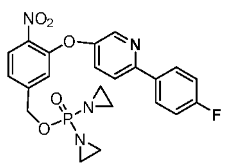 | 1.54 | 0.54 | 0.71 | 19.72 |

(continued)

| Number | compound | Efflux ratio (MDCK-MDR1 ) | | Inhibitory effect of the proliferation of the cancer cells | |
|---|---|---|---|---|---|
| | | Without Verapamil | With Verapamil | $IC_{50}$ (nM) | $IC_{50}$ of +TH3021 (nM) |
| 8 | 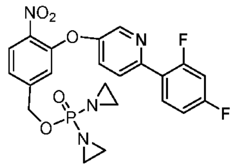 | untested | untested | <1.5 | 26.83 |
| 9 | 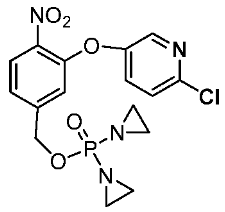 | untested | untested | <1.5 | 56.15 |
| 10 | 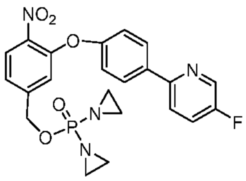 | 1.43 | 0.61 | 1.15 | 29.63 |
| 11 | 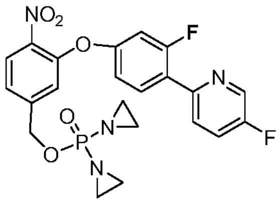 | untested | untested | 0.2108 | 92.23 |
| 12 | 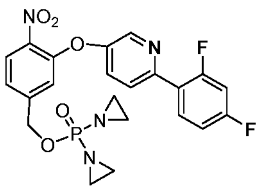 | untested | untested | <1.5 | 11.4 |
| 13 | 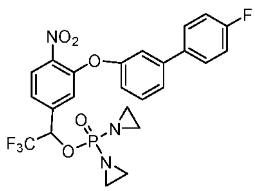 | untested | untested | 29.99 | 1887 |
| 14 | 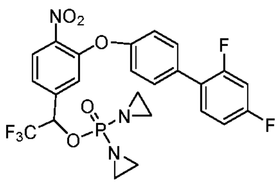 | 1.26 | 0.75 | 11.39 | >1000 |

(continued)

| Number | compound | Efflux ratio (MDCK-MDR1 ) | | Inhibitory effect of the proliferation of the cancer cells | |
|---|---|---|---|---|---|
| | | Without Verapamil | With Verapamil | $IC_{50}$ (nM) | $IC_{50}$ of +TH3021 (nM) |
| 15 | 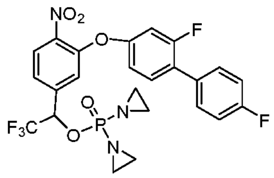 | 1.36 | 1.12 | 6.782 | 1913 |
| 16 | 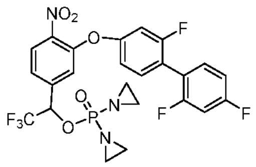 | 1.96 | 0.71 | 7.258 | >500 |
| 17 | 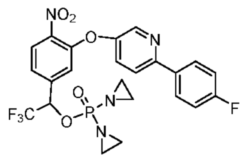 | untested | untested | 35.45 | >10000 |
| 18 | 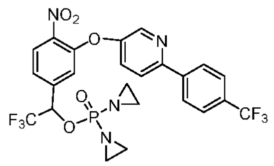 | untested | untested | 312.9 | >10000 |
| 19 | 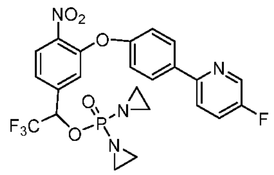 | untested | untested | 46.81 | >10000 |
| Control group | Metoptolol | 0.96 | untested | untested | untested |
| | Prazosin | 2.87 | 0.57 | untested | untested |
| | Imatinib | 18.06 | 0.90 | untested | untested |

**3. The compound preparation and the spectrum**

"THF" refers to tetrahydrofuran; "DCM" refers to dichloromethane; "EA" or "EtOAc" refers to ethyl acetate; "TEA" refers to triethylamine; "HPLC" refers to high performance liquid chromatography; "MTBE" refers to methyl tert-butyl ether; "DMAP" refers to 4-dimethylaminopyridine; "DBAD" refers to di-tert-butyl azodicarboxylate; "TFA" refers to trifluoroacetate; "LCMS" refers to liquid chromatography-mass spectrometry; "EtOH" refers to ethanol; "t-BuOH" refers to tert-butanol; "DMF" refers to 2,5-dimethylfuran; "PE" refers to petroleum ether; "eq" refers to the chemical equivalence; "TBAF" refers to tetra-n-butylammonium fluoride; and "DIPEA" refers to N,N-diisopropylamine.

All chemical agents, drugs which were not indicated the source were analytical reagent (AR) or chemical pure (CP) and were purchased from commercial reagent companies.

Other English abbreviations were by reference to the explanation in organic chemistry.

Synthesis of **compound 1**

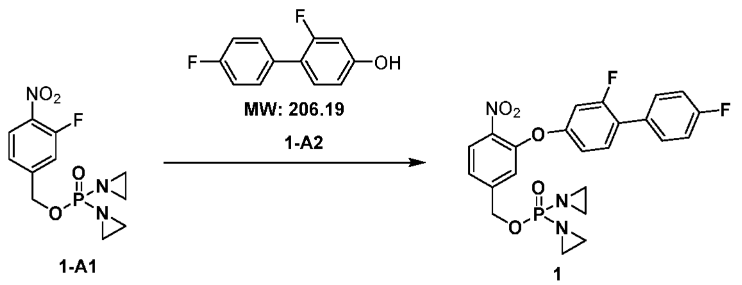


Compound 1-A1 (80.0 mg, 0.266 mmol, synthesized by the synthesis method of compound 3-A4) and 1-A2 (78.3 mg, 0.380 mmol, purchased) were dissolved in acetone (4 mL), then $Cs_2CO_3$ (199.3 mg, 0.612 mmol) was added and the solution was stirred at room temperature. HPLC was used to detect the extent of reaction. After 2 h, the reaction was finished. The solid was removed by the suction filtration, the original solution was concentrated and separated by column chromatography. The solution after preparation was extracted by DCM (10 mL×3), concentrated, freeze-dried, and then the pure product of compound 1 was obtained (17 mg, percent yield 13.1%, light yellow solid).

**$^1$H-NMR (400 MHz, $CDCl_3$):** δ 8.02 (d, J = 8.4 Hz, 1H), 7.48 (dd, J = 7.2, 5.5 Hz, 2H), 7.39 (t, J = 8.6 Hz, 1H), 7.30 (d, J = 8.5 Hz, 1H), 7.19 (s, 1H), 7.14 (t, J = 8.7 Hz, 2H), 6.89 (dd, J = 8.5, 2.0 Hz, 1H), 6.83 (dd, J = 11.1, 2.3 Hz, 1H), 5.19 (d, J = 8.0 Hz, 2H), 2.21-2.12 (m, 8H).

**MS:** Calculated 487.1, found 488.1 ($[M+H]^+$).

Synthesis of **compound 2**

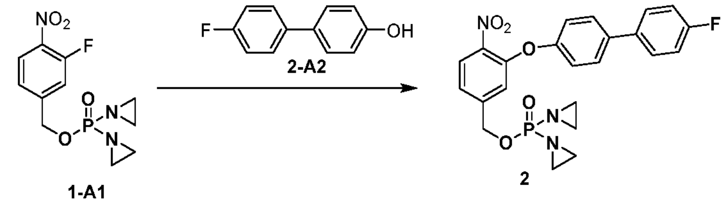


Compound 1-A1 (150.0 mg, 0.50 mmol, synthesized by the synthesis method of compound 3-A4) and compound 2-A2 (134 mg, 0.71 mmol, purchased) were dissolved in acetone (10 mL), then $Cs_2CO_3$ (199.3 mg, 0.612 mmol) was added and the solution was stirred at room temperature. HPLC was used to detect the extent of reaction. After 2 h, the reaction was finished. The solution was separated by the suction filtration with the diatomaceous earth. The filter cake was washed by EtOAc (10 mL×3) and combined the organic phase. The original solution was concentrated and separated by column chromatography. The solution after preparation was extracted by DCM (10 mL×3), concentrated at 35°C, freeze-dried, and then the pure product of compound 2 was obtained (26 mg, percent yield 11.1%, light yellow oily substance).

**$^1$H-NMR (400 MHz, MeOD):** δ8.01 (d, J = 8.4 Hz 1H),7.68-7.62 (m, 4 H), 7.31 (d, J = 8.4 Hz 1 H), 7.20-7.15 (m, 5 H), 5.18 (d, J = 8.4 Hz 2 H), 2.17-2.10 (m, 8 H).

**MS:** Calculated 469.1, found 470.2 ($[M+H]^+$).

Synthesis of **compound 3**

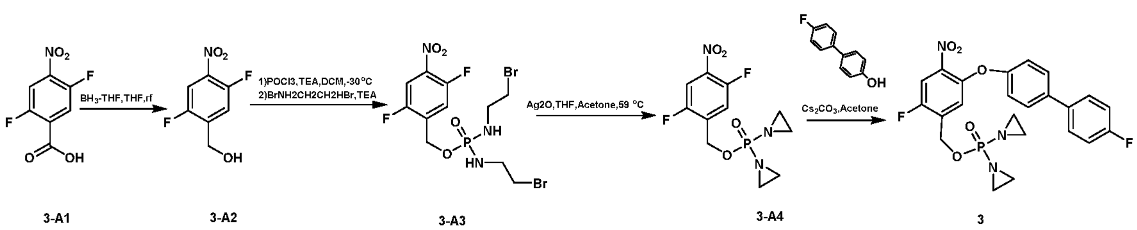

The synthesis of **compound 3-A2**

Under nitrogen protection, compound 3-A1 (5 g, 24.62 mmol, purchased) was dissolved in anhydrous THF (50 mL) and the solution was lowered to 0°C. The gas was exchanged 3 times, borane-tetrahydrofuran complex (61.5 mL · 61.5 mmol, 1 mol/L in THF) was slowly dripped to the reaction solution. The solution was kept at 0°C for 30 min, and then warmed up to 65°C. After 3 h, the reaction was finished. The reaction solution was naturally cooled to room temperature and slowly added to cold water (300 g). The reaction solution was extracted by DCM (50 mL×3). The organic phase was washed by 1 M HCl (50 mL×3), washed by water, and washed by brine, concentrated, and dried to obtain the product (4.6 g, percent yield 98.9%, off-white solid).

**$^1$H-NMR (400 MHz, DMSO-*d6*):** δ 8.04 (dd, *J* = 9.2, 6.1 Hz, 1H), 7.58 (dd, *J* = 11.7, 5.8 Hz, 1H), 5.72 (s, 1H), 4.63 (s, 2H).

**MS:** Calculated MS: 189.1, found: 188.0 ($[M-H]^-$).

Synthesis of **compound 3-A3**

Under nitrogen protection, $POCl_3$ (610 mg, 3.97 mmol) was added to anhydrous DCM (5 mL), and compound 3-A2 (500 mg, 2.64 mmol) was added. The reaction solution was lowered to -20°C. TEA (454 mg, 4.49 mmol) in DCM (3 mL) was slowly added to the reaction solution. The reaction solution was kept at -20°C for 30 min. After 3 h, the reaction of 3-A2 was finished. The reaction solution was cooled to -40°C, and 2-bromoethylamine hydrobromide (1.6 g, 7.92 mmol) was slowly added, TEA (801 mg, 7.92 mmol) in DCM was dropwise added to the solution. The reaction solution was kept at -40°C for 30 min, and the reaction was finished. 5 mL of $H_2O$ was slowly added, and the reaction solution was naturally warmed up to room temperature. The reaction solution was extracted by DCM (8 mL×3), the organic phase was washed by $H_2O$ (5 mL×2), concentrated, dried, and separated by column chromatography (100-200 mesh silica gel, EA: n-heptane = 1:1-EA) to obtain the product (260 mg, yield 20.5%, off-white solid).

**$^1$H NMR (400 MHz, $CDCl_3$):** δ 7.81 (dd, *J*= 8.6, 5.9 Hz, 1H), 7.48 (dd, J = 10.5, 5.7 Hz, 1H), 5.14 (d, *J*= 7.6 Hz, 2H), 3.52-3.34 (m, 8H).

**MS:** Calculated 480.9, found 481.9 ($[M+H]^+$).

Synthesis of **compound 3-A4**

Under nitrogen protection, compound 3-A3 (5 g, 24.62 mmol, purchased) was dissolved in THF (4 mL) and acetone (6 mL), the reaction solution was warmed up to 59°C. After 4 h, the reaction was finished. The reaction solution was cooled to room temperature, removed by the suction filtration with the diatomaceous earth. The solid was washed by acetone (5 mL), and the original solution was concentrated to obtain the crude product (190 mg, light-yellow solid). The next step was executed directly.

**$^1$H-NMR (400 MHz, MeOD):** δ 7.99 (dd, *J* = 9.1, 6.0 Hz, 1H), 7.61 (dd, *J* **=** 10.9, 5.8 Hz, 1H), 5.30 (d, *J*= 7.9 Hz, 2H), 2.32-1.95 (m, 8H).

**MS:** Calculated 319.1, found 320.0 ($[M+H]^+$).

Synthesis of **compound 3**

Under nitrogen protection, compound 3-A4 (80 mg, 0.25 mmol) and 4-hydroxy-4'-fluorobiphenyl (71 mg, 0.38 mmol) were dissolved in acetone (5 mL), $CsCO_3$ was added (205 mg, 0.63 mmol). The reaction solution was stirred for 3 h. After 3 h, the reaction was finished. The solution was separated by the suction filtration with the diatomaceous earth. The solid was washed by acetone (3 mL), the original solution was concentrated. After the column chromatography, the pure product was obtained (17 mg, percent yield 14.0%, white solid).

**$^1$H-NMR (400 MHz, MeOD):** δ 7.91 (d, J = 9.1 Hz, 1H), 7.67-7.61 (m, 4H), 7.30 (d, *J* **=** 6.0 Hz, 1H), 7.20-7.14 (m, 4H), 5.25 (d, *J* = 8.0 Hz, 2H), 2.15-2.10 (m, 8H).

**MS:** Calculated 487.1, found 488.0 ($[M+H]^+$).

Synthesis of **compound 4**

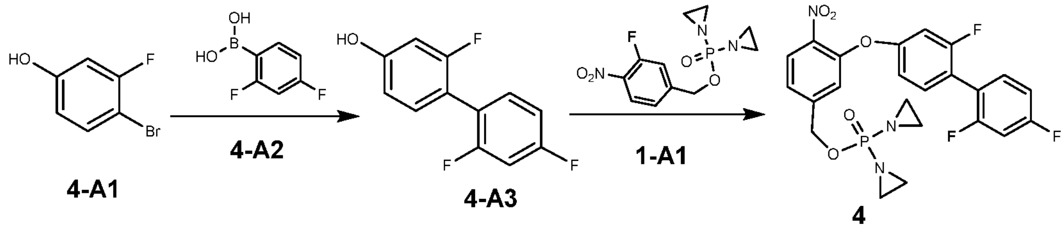

4-A1 4-A2 4-A3 1-A1 4

Synthesis of **compound 4-A3**

Under nitrogen protection, compound 4-A1 (1.0 g, 5.24 mmol, purchased) and compound 4-A2 (909 mg, 5.76 mmol, purchased) were added in the mixed solution of 1,4-dioxane (18 mL) and $H_2O$ (2 mL), $K_2CO_3$ (2.2 g, 15.71 mmol) was added to the reaction solution. $N_2$ was exchanged 3 times, $Pd(OAc)_2$ (59 mg, 0.26 mmol) and $PPh_3$ (69 mg, 0.26 mmol) were added to the reaction solution. $N_2$ was exchanged 3 times again, the reaction solution was warmed up to 100°C. After 1.5 h, the reaction was finished.

Post treatment: the reaction solution was cooled to room temperature, separated by the suction filtration with the diatomaceous earth and washed by DCM. The original solution was extracted by DCM (5 mL×3), concentrated, dried and separated by reversed-phase chromatography (water:MeCN = 50%:50%), then the product was obtained (238 mg, percent yield 20.3%, yellow solid).

**$^1$H-NMR (400 MHz, MeOD):** $\delta$7.39-7.33 (m, 1H), 7.16 (t, $J$ = 8.4 Hz, 1H), 7.03-6.97 (m, 2H), 6.68 (dd, ,$J$ = 8.4 Hz, 2.4 Hz, 1H), 6.60 (dd, $J$= 12.0, 2.4 Hz, 1H).

**MS:** Calculated224.1, found 223.0 ([M-H]$^-$).

Synthesis of **compound 4**

Under nitrogen protection, compound 4-A3 (80 mg, 0.27 mmol) and compound 1-A1 (101 mg, 0.45 mmol, synthesized by the synthesis method of compound 3-A4) were dissolved in acetone (5 mL), $Cs_2CO_3$ (216 mg, 0.66 mmol) was added to the reaction solution, and then the reaction solution was stirred. After 1.5 h, the reaction was finished.

Post treatment: the reaction solution was separated by the suction filtration with the diatomaceous earth and washed by DCM. The original solution was concentrated, dried, separated by column chromatography and freeze-dried to obtain the product (35.5 mg, percent yield 26.4%, light yellow oily substance).

**$^1$H-NMR (400 MHz, MeOD):** $\delta$8.06 (d, $J$ = 8.4 Hz, 1H), 7.48-7.40 (m, 3H), 7.32 (s, 1H), 7.10-7.05 (m, 2H), 6.99-6.95 (m, 2H), 5.23 (d, $J$ = 8.4 Hz, 2H), 2.22-2.13 (m, 8H).

**MS:** Calculated505.1, found 506.1 ([M+H]$^+$).

Synthesis of **compound 5**

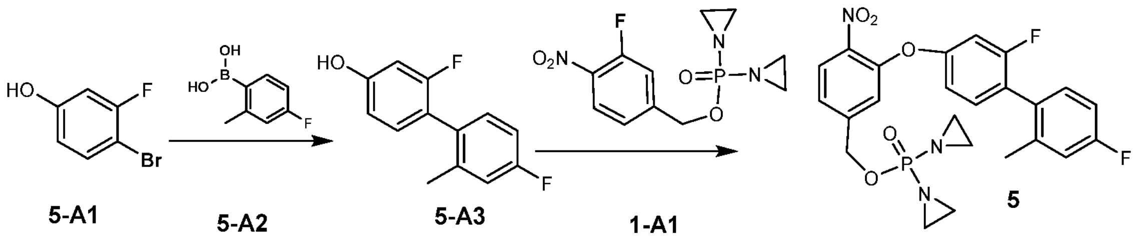

5-A1 5-A2 5-A3 1-A1 5

Synthesis of **compound 5-A3**

Under nitrogen protection, compound 5-A1 (1.0 g, 5.24 mmol, purchased) and compound 5-A2 (886 mg, 5.76 mmol, purchased) were added in the mixed solution of 1,4-dioxane (18 mL) and $H_2O$ (2 mL), and then $K_2CO_3$ (2.2 g, 15.71 mmol) was added to the reaction solution. $N_2$ was exchanged 3 times, $Pd(OAc)_2$ (59 mg, 0.26 mmol) and $PPh_3$ (69 mg, 0.26 mmol) were added to the solution. $N_2$ was exchanged 3 times again, the temperature was warmed up to 100°C. After 1.5 h, the reaction was finished.

Post treatment: The reaction solution was cooled to room temperature, separated by the suction filtration with the diatomaceous earth and washed by DCM. The original solution was extracted by DCM (5 mL×3), concentrated,

dried and separated by column chromatography (200-300 mesh silica gel, n-heptane:EA=5:1) to obtain the product (895 mg, percent yield 77.6%, off-white solid).

**$^1$H-NMR (400 MHz, $CDCl_3$):** δ7.16-7.12 (m, 1H), 7.06 (t, *J* = 8.4 Hz, 1H), 6.99-6.89 (m, 2H), 6.72-6.65 (m, 2H), 6.32 (s, 1H), 2.19 (s, 3H).

**MS:** Calculated 220.1, found 219.1 ($[M-H]^-$).

Synthesis of **compound 5**

Under nitrogen protection, compound 5-A3 (80 mg, 0.27 mmol) and compound 1-A1 (95 mg, 0.45 mmol, synthesized by the synthesis method of compound 3-A4) were dissolved in acetone (5 mL), $Cs_2CO_3$ (216 mg, 0.66 mmol) was added to the reaction solution, then the solution was stirred for 2 h at room temperature, and the reaction was finished.

Post treatment: The reaction solution was separated by the suction filtration with the diatomaceous earth and washed by DCM. The original solution was concentrated, separated by column chromatography and freeze-dried to obtain the product (13 mg, percent yield 7.1%, yellow oily substance).

**$^1$H-NMR (400 MHz, MeOD):** δ 8.06 (d, *J* = 8.4 Hz, 1H), 7.40 (d, *J* = 8.4 Hz, 1H), 7.33-7.27 (m, 2H), 7.24-7.16 (m, 1H), 7.06-7.04 (m, 1H), 7.01-6.93 (m, 3H), 5.24 (d, *J* = 8.4 Hz, 2H), 2.22-2.16 (m, 11H).

**MS:** Calculated 501.1, found 502.1 ($[M+H]^+$).

Synthesis of **compound 6**

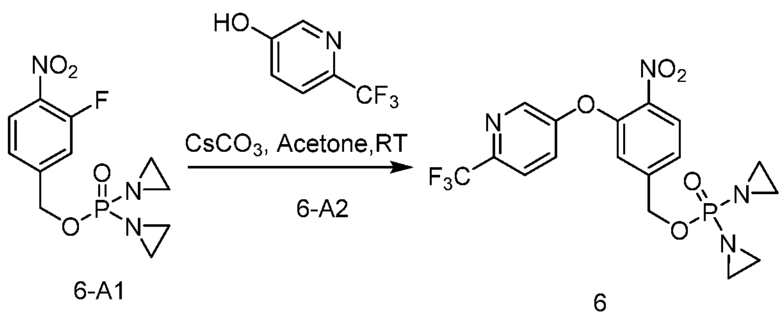


6-A1

6

Under nitrogen protection, compound 6-A1 (80 mg, 0.266 mmol, synthesized by the synthesis method of compound 3-A4) and compound 6-A2 (73.2 mg, 0.452 mmol, purchased) were dissolved in acetone (5 mL), $Cs_2CO_3$ (216.3 mg, 0.664 mmol) was added to the reaction solution, and then the reaction solution was stirred at room temperature. After 1.5 h, the reaction was finished.

Post treatment: The reaction solution was separated by the suction filtration with the diatomaceous earth and the solid was washed by DCM. The original solution was concentrated, separated by column chromatography to obtain the pure product (17.4 mg, percent yield 14.7%, light-yellow oily substance).

**$^1$H-NMR (400 MHz, MeOD):** δ8.45 (d, J = 2.8 Hz, 1H), 8.08 (d, J = 8.4 Hz, 1H), 7.69 (d, J = 8.8 Hz, 1H), 7.41-7.39 (m, 2H), 7.23 (s, 1H), 5.21 (d, J = 8.0 Hz, 2H), 2.22-2.13 (m, 8H).

**MS:** Calculated 444.1, found 445.1 ($[M+H]^+$).

Synthesis of **compound 7**

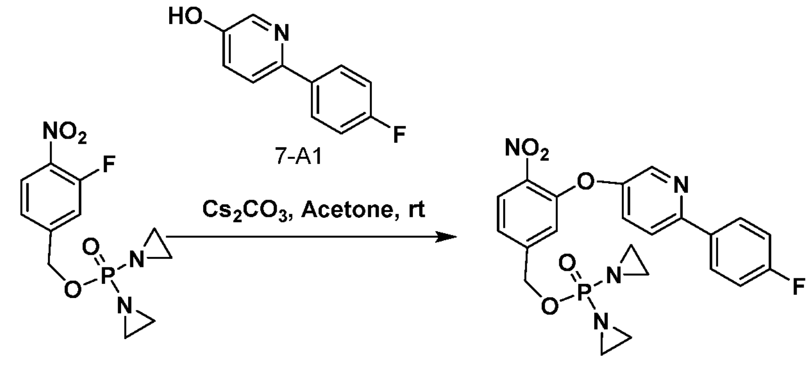


7-A2 **7**

Compound 7-A1 (94 mg, 0.50 mmol) and compound 7-A2 (100 mg, 0.33 mmol, synthesized by the synthesis method of compound 3-A4) were dissolved in acetone (5 mL), $Cs_2CO_3$ (372 mg, 0.83 mmol) was added to acetone, and then the reaction solution was stirred at room temperature. HPLC was used to detect the extent of reaction. After 1.0 h, the reaction was finished.

The reaction solution was separated by the suction filtration with the diatomaceous earth and the solid was washed by DCM (10 mL×3). The original solution was concentrated, separated by column chromatography, extracted by DCM (10 mL×3) and freeze-dried to obtain compound 7 (42 mg, percent yield 26.9%, light-yellow oily substance).

**$^1$H-NMR (400 MHz, MeOD):** δ8.43 (d, J = 2.7 Hz, 1H), 8.09 (d, J = 8.4 Hz, 1H), 8.02 (dd, J = 8.8, 5.4 Hz, 2H), 7.91 (d, J = 8.7 Hz, 1H), 7.57 (dd, J = 8.7, 2.9 Hz, 1H), 7.42 (d, J = 7.8 Hz, 1H), 7.29 (s, 1H), 7.21 (t, J = 8.8 Hz, 2H), 5.22 (d, J = 8.4 Hz, 2H), 2.26-2.08 (m, 8H).

**MS:** Calculated 470.1, found 471.1 ([M+H]$^+$).

Synthesis of **compound 8**

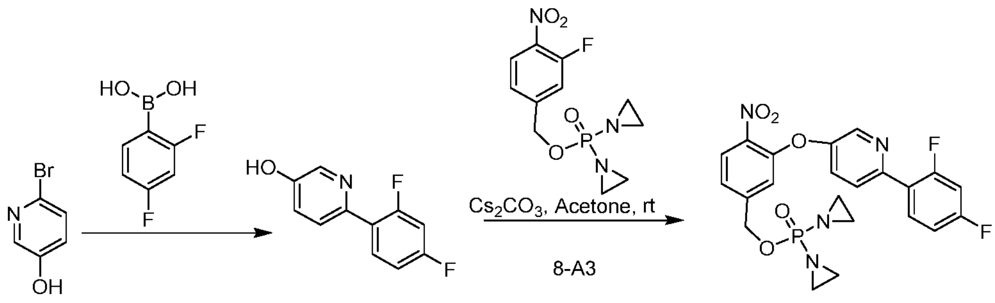


8-A1 8-A2 8

Compound 8-A1 (3.0 g, 17.2 mmol, purchased) was added in the mixed solution of 1,4-dioxane and $H_2O$ (9:1, 60 mL), $K_2CO_3$ (4.7 mg, 34.4 mmol) was added to the solution. $N_2$ was exchanged 3 times, $PPh_3$ (225 mg, 0.86 mmol) and $Pd(OAc)_2$ (193 mg, 0.86 mmol) were added in proper order, and then $N_2$ was exchanged 3 times again. The reaction was reacted at 100°C. HPLC was used to detect the extent of reaction, and the reaction was finished after 10 h.

The reaction solution was separated by the suction filtration with the diatomaceous earth and the solid was washed by DCM (15 mL×3). The solid was washed. The original solution was concentrated, dry packed, separated by column chromatography (Heptane:EtOAc = 5:1), and mixed with MTBE (3 mL) to obtain compound 8-A2 (1.5 g, percent yield 42.0%, white solid).

**$^1$H-NMR (300 MHz, MeOD):** δ8.19 (s, 1H), 7.78-7.75 (m, 1 H), 7.58 (d, J = 8.4 Hz, 1 H), 7.29 (d, J = 8.4 Hz, 1 H), 7.08-7.05 (m, 2 H).

**MS:** Calculated 207.0, found 208.2 ([M+H]$^+$).

Compound 8-A2 (103 mg, 0.50 mmol) and compound 8-A3 (100 mg, 0.33 mmol, synthesized by the synthesis method of compound 3-A4) were dissolved in acetone (5 mL), $Cs_2CO_3$ (270 mg, 0.83 mmol) was added to the reaction solution, then the reaction solution was stirred at room temperature, the reaction solution was light yellow. HPLC was used to detect the extent of reaction and the reaction was finished after 2.0 h

The reaction solution was separated by the suction filtration with the diatomaceous earth and the solid was washed by acetone (10 mL×3). The original solution was concentrated, separated by column chromatography, extracted by DCM (10 mL×3) and freeze-dried to obtain compound 8 (26 mg, percent yield 16.0%, yellow liquid).
**$^1$H-NMR (400 MHz, MeOD):** δ8.47 (d, J = 2.8 Hz, 1H), 8.10 (d, J = 8.4 Hz, 1H), 7.95-7.92 (m, 1H), 7.85-7.82 (m, 1H), 7.59-7.57 (m, 1H), 7.46-7.44 (m, 1H), 7.33 (s, 1H), 7.11-7.08 (m, 2H), 5.23 (d, J = 8.4 Hz, 2H), 2.19-2.15 (m, 8H).
MS: Calculated488.1, found 489.1 ([M+H]$^+$).

Synthesis of **compound 9**

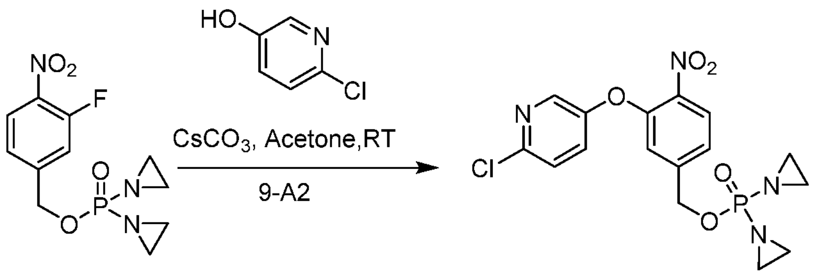


9-A1 9

Under nitrogen protection, compound 9-A1 (80 mg, 0.266 mmol, synthesized by the synthesis method of compound 3-A4) and compound 9-A2 (58 mg, 0.452 mmol, purchased) were dissolved in acetone (5 mL), $Cs_2CO_3$ (216.3 mg, 0.664 mmol) was added to the reaction solution, and then the reaction solution was stirred at room temperature. After 1.5 h, the reaction was finished.
The reaction solution was separated by the suction filtration with the diatomaceous earth and the solid was washed by DCM. The original solution was concentrated, separated by HPLC to obtain the pure product (19.8 mg, percent yield 18.1%, yellow liquid).
**$^1$H-NMR (400 MHz, MeOD):** δ8.18 (d, J = 2.8 Hz, 1H), 8.09 (d, J = 8.4 Hz, 1H), 7.55-7.44 (m, 3H), 7.30 (s, 1H), 5.22 (d, J = 8.0 Hz, 2H), 2.22-2.15 (m, 8H).
**MS (retention time 3.103 min):** Calculated 410.1, found 411.0 ([M+H]$^+$).

Synthesis of **compound 10**

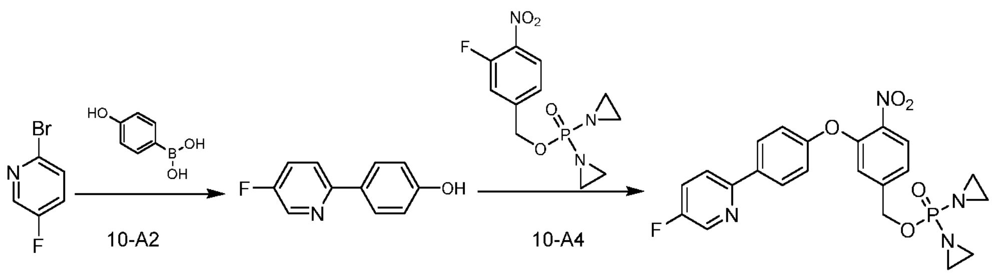


10-A1 10-A3 10

Under nitrogen protection, 2-bromo-5-fluoropyridine (compound 10-A1, 2.0 g, 11.36 mmol) and 4-hydroxyphenylboronic acid (compound 10-A2, 1.9 g, 13.64 mmol) were added to the mixed solution of DME (40 mL) and $H_2O$ (9 mL). $N_2$ was exchanged 3 times, $Pd(PPh_3)_4$ (396 mg, 0.35 mmol) and $Na_2CO_3$ (2.4 g, 22.73 mmol) were added, then $N_2$ was exchanged 3 times again. The reaction solution was warmed up to 80°C. HPLC was used to detect the extent of reaction. After 2.5 h, the reaction was finished.
The reaction solution was cooled to room temperature and extracted by EtOAc (60 mL×3). The organic phase was washed by water, washed by brine, and separated by column chromatography (200-300 mesh silica gel, n-heptane:EA = 15:1-12:1) to obtain the compound 10-A3 (1.4 g, percent yield 54.1%, light yellow solid).

**$^1$H-NMR (300 MHz, MeOD):** δ8.43 (d, J = 2.7 Hz, 1H), 7.81-7.77 (m, 3H), 7.64-7.58 (m, 1H), 6.87 (d, J = 8.7 Hz, 1H).

**MS:** Calculated 189.1, found 190.1 ([M+H]$^+$).

Under nitrogen protection, compound 10-A4 (80 mg, 0.27 mmol, synthesized by the synthesis method of compound 3-A4) and compound 10-A3 (88 mg, 0.45 mmol) were dissolved in acetone (5 mL), $Cs_2CO_3$ (216 mg, 0.66 mmol) was added to the solution, and then the solution was stirred at room temperature. After 2 h, the reaction was finished.

Post treatment: The reaction solution was separated by the suction filtration with the diatomaceous earth and washed by DCM. The original solution was concentrated, separated by column chromatography and freeze-dried to obtain compound 10 (2 mg, percent yield 1.5%, yellow oily substance).

**$^1$H-NMR (400 MHz, MeOD):** δ 8.52 (d, J = 2.8 Hz, 1H), 8.04 (dd, J = 8.6, 3.1 Hz, 3H), 7.92 (dd, J = 8.8, 4.3 Hz, 1H), 7.68 (dd, J = 8.4, 2.9 Hz, 1H), 7.35 (d, J = 8.3 Hz, 1H), 7.22 (s, 1H), 7.17 (d, J = 8.8 Hz, 2H), 5.20 (d, J = 8.3 Hz, 2H), 2.28 - 2.03 (m, 8H).

**MS:** Calculated 470.1, found 471.1 ([M+H]$^+$).

Synthesis of **compound 11**

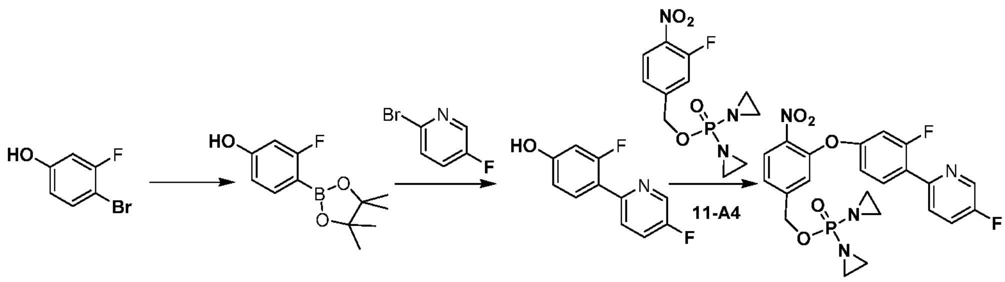


11-A1 11-A2 11-A3 11

Compound 11-A1 (4.0 g, 20.9 mmol) and $B_2pin_2$ (8.0 g,31.4 mmol) were added in the mixed solution of 1,4-dioxane and $H_2O$ (9:1, 40 mL), and then KOAc (4.7 mg, 34.4 mmol) and $Pd(PPh_3)_4$ (1.21 g, 1.1 mmol) were added to the reaction solution. $N_2$ was exchanged 3 times, and the reaction was reacted at 95°C. HPLC was used to detect the extent of reaction.

After the reaction was finished, the reaction solution was separated by the suction filtration with the diatomaceous earth and the solid was washed by EtOAc (10 mL×3), the original solution was concentrated, extracted by EtOAc (30 mL×3), washed by water (40 mL), washed by brine (40 mL×2), dried by $MgSO_4$ and concentrated, and separated by column chromatography (Heptane:EtOAc = 10:1) to obtain compound 11-A2 (1.8 g, percent yield 36.7%, white solid).

**$^1$H-NMR (400 MHz, CDCl3):** δ7.61 (t, J = 7.6 Hz, 1H), 6.61 (dd, J = 8.0, 2.0 Hz, 1H), 6.52 (dd, J = 10.8, 2.0 Hz, 1 H), 5.39 (s, 1 H), 1.34 (s,12 H).

**MS:** Calculated 238.1, found 239.2 ([M+H]$^+$).

Compound 11-A2 (1.15 g, 4.83 mmol) was dissolved in the mixed solution of 1,4-dioxane and $H_2O$ (9:1) and the solution was under nitrogen protection, $K_3PO_4$ (1.5 g, 7.1 mmol) and 2-bromo-5-fluoropyridine (500mg, 2.8 mmol) were added to the reaction solution. $N_2$ was exchanged 3 times, and the reaction was reacted at 90°C. HPLC was used to detect the extent of reaction.

After the reaction was finished, the reaction solution was separated by the suction filtration with the diatomaceous earth and the solid was washed by EtOAc (20 mL×3), the original solution was concentrated, washed by water (10 mL), dried by $Na_2SO_4$ and concentrated, and separated by column chromatography (200-300 mesh silica gel, Heptane:EA = 3:1) to obtain compound 11-A3 (500 mg, percent yield 76.2%, white solid).

**$^1$H-NMR (400MHz, MeOD):** δ10.24 (s, 1H), 8.64 (d, J = 2.4 Hz 1H), 7.78-7.73 (m, 3H), 6.75-6.73 (m, 1H), 6.69-6.65 (m, 1H).

**MS:** Calculated 207.1, found 208.2 ([M+H]$^+$).

Compound 11-A4 (80 mg, 0.27 mmol, synthesized by the synthesis method of compound 3-A4) was dissolved in acetone (5 mL), $Cs_2CO_3$ (199 mg, 0.612 mmol) was added to the reaction solution, and then the solution was stirred at room temperature. HPLC was used to detect the extent of reaction, and the reaction was finished after 1.5 h.

The reaction solution was separated by the suction filtration with the diatomaceous earth and the solid was washed by EtOAc (10 mL×3), the original solution was concentrated, separated by column chromatography, extracted

by DCM (10 mL×3) and freeze-dried to obtain compound 11 (17 mg, percent yield 13.1%, light yellow oily substance).

**$^1$H-NMR (400 MHz, MeOD):** δ8.57 (d, J = 2.8 Hz, 1H), 8.08 (d, J = 8.4 Hz, 1H), 7.94 (t, J = 9.6 Hz, 1H), 7.86-7.83 (m, 1 H), 7.72-7.67 (m, 1 H), 7.44 (d, J = 8.4 Hz 1 H), 7.34 (s, 1 H), 7.00-6.95 (m, 2 H), 5.23 (d, J = 8.4 Hz, 2H), 2.22-2.14 (m, 8 H).

**MS:** Calculated 488.1, found 489.2 ([M+H]$^+$).

Synthesis of **compound 12**

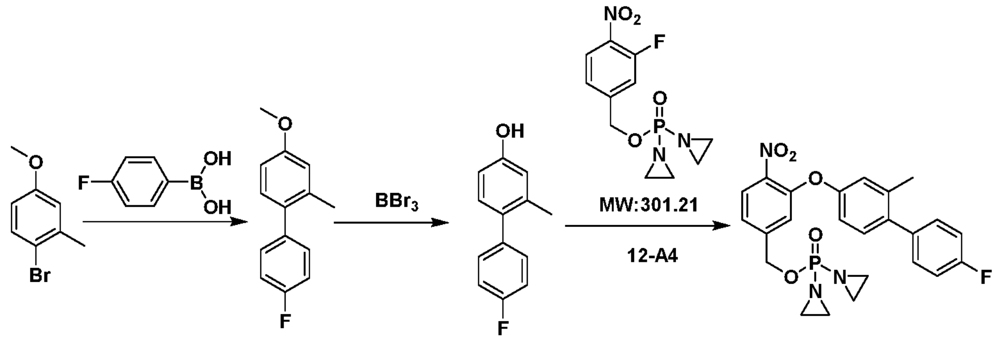


12-A1 12-A2 12-A3 12

Compound 12-A1 (4.0 g, 19.9 mmol) and 4-fluorophenylboronic acid (3.3g, 23.9 mmol) were added to the mixed solution of 1,4-dioxane and $H_2O$ (9:1, 40 mL), $K_2CO_3$ (8.2 g, 59.7 mmol) was added to the reaction solution, and $N_2$ was exchanged 3 times. $PPh_3$ (262 mg, 1.0 mmol) and $Pd(OAc)_2$ (224 mg, 0.995 mmol) were added to the reaction solution, and $N_2$ was exchanged 3 times again. The reaction was reacted at 100°C. HPLC was used to detect the extent of reaction.

After the reaction was finished, the reaction solution was separated by the suction filtration with the diatomaceous earth and the solid was washed by EtOAc, the original solution was concentrated, extracted by EtOAc (20 mL×3), dried, concentrated, and separated by column chromatography to obtain compound 12-A2 (700 mg, percent yield 16.2%, white solid).

**$^1$H-NMR (400 MHz, DMSO):** δ7.34-7.31 (m, 2H),7.21 (t, J = 8 Hz, 2H), 7.10 (d, J = 8 Hz, 1H), 6.85 (s,1H), 6.81 (d, J = 8 Hz, 1H), 3.75 (s, 3 H), 2.18 (s, 3 H).

Compound 12-A2 (510 mg, 2.36 mmol) was dissolved in DCM (18 mL), and the reaction solution was cooled to 0°C. After the $BBr_3$ (1.2 g, 4.72 mmol) was added to the reaction solution, the temperature was natural warmed up to room temperature, and the reaction solution was stirred at room temperature for 1 h. HPLC was used to detect the extent of reaction until the reaction was finished.

Post treatment: the reaction solution was quenched by water (15 mL), extracted by DCM (10 mL×5) and dried to obtain the crude product, and the crude product was separated by column chromatography to obtain compound 12-A3 (300 mg, percent yield 62.9%, light yellow liquid).

**$^1$H-NMR (400 MHz, DMSO):** δ9.40 (s, 1H),7.32-7.28(m, 2 H), 7.20 (t, J = 8 Hz, 2 H), 6.99 (d, J = 8Hz, 1 H), 6.68 (s, 1 H), 6.66-6.63 (m, 1 H), 2.14 (s, 3 H).

**MS:** Calculated 202.1, found 200.6 ([M-H]$^-$).

Compound 12-A4 (80 mg, 0.27 mmol, synthesized by the synthesis method of compound 3-A4) and compound 12-A3 (76.8 mg, 0.38 mmol) were dissolved in acetone (4 mL), $Cs_2CO_3$ (199 mg, 0.61 mmol) was added to the reaction solution, and then the solution was stirred at room temperature. HPLC was used to detect the extent of reaction. After 1.5 h, the reaction was finished.

The solid of the reaction solution was separated by the suction filtration, the original solution was concentrated, separated by column chromatography, extracted by DCM (10 mL×3), concentrated at 35°C water bath, and freeze-dried to obtain compound 12 (21 mg, percent yield 16.4%, yellow oily substance).

**$^1$H-NMR (400 MHz, $CD_3OD$):** δ8.00 (d, J = 8.4 Hz, 1H), 7.35-7.29 (m, 3 H), 7.25 (d, J = 8.4 Hz, 1 H), 7.19-7.14 (m, 3 H), 7.03 (d, J = 2.0 Hz, 1 H), 6.97-6.94 (m, 1 H), 5.19 (d, J = 8.4 Hz, 2 H). 2.25 (s, 3 H). 2.18-2.12 (m, 8 H).

**MS:** Calculated 483.1, found 484.1 ([M+H]$^+$).

Synthesis of **compound 13**

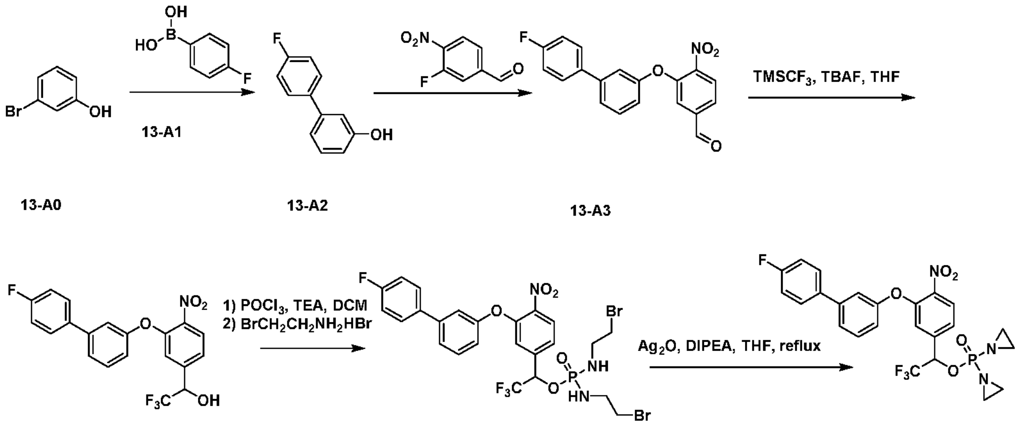


13-A0 13-A2 13-A3

13-A4 13-A5 13

Under nitrogen protection, m-bromophenol (13-A0, 1.5 g, 8.57 mmol) and 4-fluorophenylboronic acid (13-A1, 1.0 g, 7.15 mmol) were added to the mixed solution of 1,4-dioxane (30 mL) and $H_2O$ (5 mL). $N_2$ was exchanged 3 times, $Pd(OAc)_2$ (224 mg, 0.995 mmol), $PPh_3$ (94 mg, 0.36 mmol) and $K_2CO_3$ (3.0 g, 21.44 mmol) were added to the reaction solution and $N_2$ was exchanged 3 times again, the reaction solution was warmed up to 100°C. The reaction was under supervision for 1 h and the reaction was finished.

Post treatment: the reaction solution was cooled to room temperature, extracted by EtOAc (50 mL×3) after the suction filtration. The organic phase was washed by water, washed by brine, separated by column chromatography (200-300 mesh silica gel, n-heptane:EA = 12:1-9:1) to obtain compound 13-A2 (1.1 g, percent yield 76.2%, white solid).

$^1$H-NMR (300 MHz, MeOD): δ7.59-7.54 (m, 2H), 7.23-7.10 (m, 3H), 7.04-6.99 (m, 2H), 6.76 (dd, J = 8.1,1.5 Hz, 1H).

MS: Calculated 188.1, found 189.1 ($[M+H]^+$).

Compound 13-A2 (580 mg, 3.08 mmol) and 3-fluoro-4-nitrobenzoaldehyde (434 mg, 2.57 mmol) were dissolved in MeCN (10 mL), $K_2CO_3$ (887 mg, 6.42 mmol) was added to the reaction solution under nitrogen, and the reaction solution was warmed up to 80°C, the reaction was under supervision for 2 h and the reaction was finished.

The reaction solution was cooled to room temperature, separated by the suction filtration with the diatomaceous earth, washed by DCM. The original solution was concentrated and separated by column chromatography (MeCN:$H_2O$ = 50%:50%) to obtain compound 13-A3 (320 mg, percent yield 33.7%, yellow oily substance).

**$^1$H-NMR (400 MHz, CDCl3):** δ9.98 (s, 1H), 8.05 (d, J = 8.4 Hz, 1H), 7.71 (d, J = 1.6 Hz, 1H), 7.69-7.43 (m, 5H), 7.28-7.27 (m, 1H), 7.15-7.11 (m, 2H), 7.08-7.05 (m, 1H).

Under nitrogen protection, compound 13-A3 (350 mg, 1.04 mmol) and $TMSCF_3$ (296 mg, 2.08 mmol) were dissolved in THF (4 mL). After the reaction solution was cooled to 0°C, TBAF (0.01mL, 0.01 mmol, 1M in THF) was added to the reaction solution and the temperature was kept at 0°C for 30 min. After the reagent 14-A3 was totally vanished, 3N HCl (2 mL) was added to the reaction solution, and the solution became clear. The reaction solution was keeping stirring for 1 h to totally transfer to the product.

The reaction solution was extracted by DCM (5 mL×3). The organic phase was washed by water (5 mL×3), dried, concentrated, and separated by column chromatography (200-300 mesh silica gel, n-heptane:EA = 12:1-10:1) to obtain the product (350 mg, percent yield 82.8%, yellow solid).

**$^1$H-NMR (400 MHz, CDCl3):** δ8.00 (d, J = 8.4 Hz, 1H), 7.53-7.34 (m, 5H), 7.24-7.23 (m, 2H), 7.15-7.11 (m, 2H), 7.03-7.00 (m, 1H), 5.03-5.02 (m, 1H).

**MS:** Calculated 407.1, found 408.0 ($[M+H]^+$).

Under nitrogen protection, $POCl_3$ (188 mg, 1.23 mmol) was dissolved in DCM (5 mL), and the reaction solution was cooled to -40°C. Compound 13-A4 (250 mg, 0.61 mmol) was dissolved in DCM (2 mL) and added to the reaction solution with TEA (155 mg, 1.53 mmol), the reaction solution was kept at -40°C. After 3 h, compound 15-A3 was totally transferred to intermediate, 2-bromoethylamine hydrobromide (1.0g, 4.91 mmol) and TEA (497 mg, 4.91 mmol) were added to the reaction solution. The reaction was supervised and finished after 30 min.

$NH_4Cl$ (5 mL) was added to the reaction solution at 0°C, the reaction solution was extracted by DCM (20 mL×3). The organic phase was washed by water, washed by brine, dried, concentrated, and separated by column chromatography (200-300 mesh silica gel, n-heptane:EA = 2:1-1:1) to obtain compound 13-A5 (200 mg, percent yield 46.6%, yellow solid).

**$^1$H-NMR (400 MHz, $CDCl_3$):** δ8.01 (d, J = 8.4 Hz, 1H), 7.54-7.33 (m, 5H), 7.25 (d, J = 2.0 Hz, 1H), 7.18 (s, 1H), 7.15-7.10 (m, 2H), 7.03 (d, J = 1.2 Hz, 1H), 5.70-5.60 (m, 1H), 3.38-3.08 (m, 8H).

**MS:** Calculated 699.0, found 699.9 ($[M+H]^+$).

Under nitrogen protection, compound 13-A5 (150 mg, 0.22 mmol) was dissolved in THF (15 mL), $Ag_2O$ (497 mg, 2.1 mmol) and DIPEA (277 mg, 2.1 mmol) were added to the reaction solution, the reaction solution was warmed up to 65°C and stirred for 1.5 h.

After the reaction was finished, the reaction solution was cooled to room temperature, separated by the suction filtration with the diatomaceous earth, the solid was washed by DCM, the original solution was concentrated and separated by column chromatography to obtain compound 13 (56 mg, percent yield 48.6%, white solid).

**$^1$H-NMR(400 MHz, MeOD):** δ 8.08 (d, J = 8.4 Hz, 1H), 7.67-7.59 (m, 2H), 7.56-7.46 (m, 3H), 7.31 (d, J = 2.2 Hz, 2H), 7.22-7.13 (m, 2H), 7.10-7.05 (m, 1H), 6.05-5.95 (m, 1H), 2.29-1.89 (m, 8H).

**MS:** Calculated 537.1 , found 538.1 ($[M+H]^+$).

Synthesis of **compound 14**

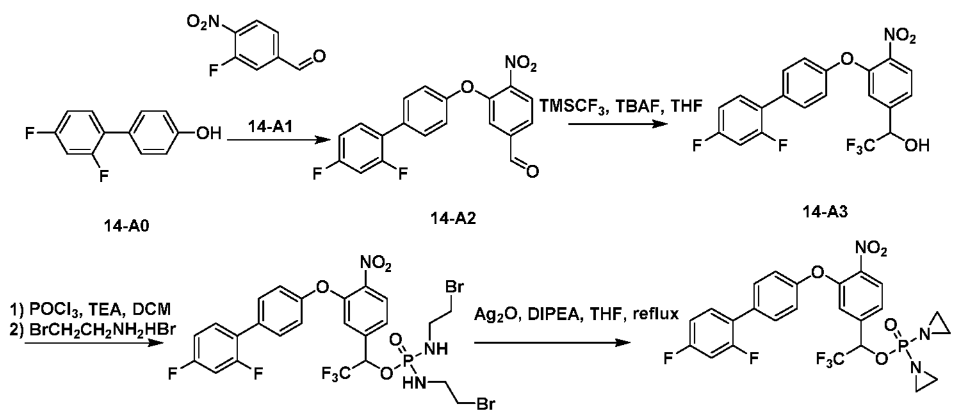


14-A0 14-A2 14-A3 14-A4 14

Compound 14-A1 (500 mg, 2.96 mmol) and compound 14-A0 (554 mg, 2.69 mmol) were dissolved in MeCN (10 mL), $K_2CO_3$ (743 mg, 5.38 mmol) was added to the solution under nitrogen, the reaction solution was warmed up to 80°C, and the reaction was supervised and finished after 1.5 h.

The reaction solution was cooled down to room temperature, washed by DCM. The original solution was concentrated and separated by column chromatography (200-300 mesh silica gel, n-heptane:EA = 15:1-10:1) to obtain the product (535 mg, percent yield 56.0%, brown oily substance).

**$^1$H-NMR (400 MHz, $CDCl_3$):** δ9.99 (s, 1H), 8.05 (d, J = 8.4 Hz, 1H), 7.71 (d, J = 8.4 Hz, 1H), 7.56-7.55 (m, 3H), 7.41 (dd, J1= 8.4 4.8 Hz, 1H), 7.16 (d, J = 8.4 Hz, 2H),7.00-6.91 (m, 2H).

Under nitrogen protection, compound 14-A2 (400 mg, 1.13 mmol) and $TMSCF_3$ (320 mg, 2.25 mmol) were dissolved in THF (4 mL), the reaction solution was cooled down to 0°C, TBAF (0.02ml, 0.02 mmol, 1M in THF) was added, and the temperature was kept at 0°C for 30 min. After compound 14-A2 was totally vanished, 3N HCl (2 mL) was added to the reaction solution, and the reaction solution was became clear. The reaction solution was keeping stirred for 1 h, extracted by DCM (5 mL×3). The organic phase was washed by water (5 mL×3), dried and concentrated to obtain the crude product (405 mg, percent yield 56.4%, yellow solid). The crude product was used to the next reaction directly.

**MS:** Calculated 425.3, found 426.0 ($[M+1]^+$).

Under nitrogen protection, $POCl_3$ (209 mg, 1.36 mmol, J&K Scientific) was dissolved in DCM (5 mL), and the reaction solution was cooled to -40°C. Compound 14-A3 (290 mg, 0.68 mmol) was dissolved in DCM (2 mL) and added to the reaction solution with TEA (173 mg, 1.70 mmol), the reaction solution was kept at -40°C. After 2 h, compound 15-A3 was totally transferred to intermediate, 2-bromoethylamine hydrobromide (1.0g, 4.91 mmol) and TEA (497 mg, 4.91 mmol) were added to the reaction solution. The reaction was supervised and finished after 30 min.

Saturated $NH_4Cl$ (5 mL) solution was added to the reaction solution at 0°C, the reaction solution was extracted by DCM (20 mL×3). The organic phase was washed by water, washed by brine, dried, concentrated, and separated by

column chromatography (200-300 mesh silica gel, n-heptane:EA = 5:1-3:1) to obtain compound 14-A4 (250 mg, percent yield 51.1%, yellow solid). **$^1$H-NMR(400 MHz, $CDCl_3$):** δ8.00 (d, J = 8.4 Hz, 1H), 7.54 (d, J = 7.6 Hz, 2H), 7.44-7.38 (m, 1H), 7.35 (d, J= 8.4 Hz, 1H), 7.21 (s, 1H), 7.13 (d, J = 8.8 Hz, 1H), 7.00-6.95 (m, 2H), 5.68 - 5.62 (m, 1H), 3.42-3.11 (m, 8H).

**MS:** Calculated 717.2, found 717.9 ($[M+H]^+$).

Under nitrogen protection, compound 14-A4 (230 mg, 0.32 mmol) was dissolved in THF (15 mL), $Ag_2O$ (372 mg, 1.60 mmol) and DIPEA (207 mg, 1.60 mmol) were added to the reaction solution, the reaction solution was warmed up to 65°C and stirred for 1.5 h.

After the reaction was finished, the reaction solution was cooled to room temperature, separated by the suction filtration with the diatomaceous earth. The solid was washed by DCM, the original solution was concentrated and separated by column chromatography to obtain the pure product of compound 14 (102 mg, percent yield 57.5%, white solid).

**$^1$H-NMR (400 MHz, $CDCl_3$):** δ8.01 (d, J = 8.4 Hz, 1H), 7.54 (d, J = 1.6 Hz, 2H), 7.51-7.36 (m, 2H), 7.24 (s, 1H), 7.12-7.11 (m, 2H), 6.97-6.93 (m, 2H), 5.72-5.69 (m, 1H), 2.26-2.01 (m, 8H).

**MS:** Calculated 555.1, found 556.1 ($[M+H]^+$).

Synthesis of **compound 15**

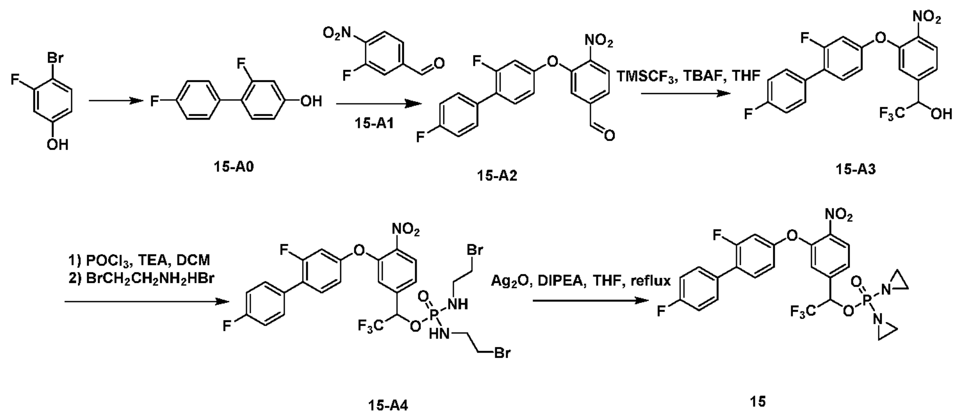


Under nitrogen protection, 4-bromo-3-fluorophenol (5.0 g, 26.2 mmol, purchased, 97%) and 4-fluorophenylboronic acid (4.0 g, 28.8 mmol) were dissolved in the mixed solution of dioxane and water (100 mL, dioxane:water = 9:1), $K_2CO_3$ (10.8 g, 78.6 mmol) was added to the reaction solution, the gas was exchanged 3 times, $Pd(OAc)_2$ (295 mg, 1.31 mmol) and $PPh_3$ (345mg, 1.31 mmol, J&K Scientific, 97%) were added to the reaction solution, the gas was exchanged 3 times again, the reaction solution was warmed up to 100°C and stirred overnight.

After the reaction was finished, the reaction solution was cooled to room temperature, separated by the suction filtration with the diatomaceous earth, washed by EA, the original solution was concentrated and pH was adjusted by 1N HCl to 3. The original solution was extracted by EA (50 mL×3), washed by water (10 mL×3), washed by brine, dried, concentrated and separated by column chromatography (100-200 mesh silica gel, n-heptane:EA = 20:1) to obtain compound 15-A0 (3.5 g, percent yield 64.8%, white solid).

**$^1$H-NMR(300 M, DMSO-d6):** δ10.02 (s, 1H), 7.52-7.47 (m, 2H), 7.34-7.23 (m, 3H), 6.71-6.64 (m, 2H).

**MS:** Calculated 206.1, found 204.8 ($[M-H]^-$).

Under nitrogen protection, compound 15-A1 (930 mg, 5.50 mmol, 97%) and compound 16-A0 (1.36 g, 6.60 mmol) were dissolved in acetonitrile (20 mL), $K_2CO_3$ (1.52 g, 11.0 mmol, J&K Scientific, 99%) was added, the reaction solution was warmed up to 85°C and stirred for 2 h, and the reaction was finished.

The reaction solution was cooled down to room temperature, separated by the suction filtration, the original solution was concentrated and separated by column chromatography (200-300 mesh silica gel, n-heptane:EA = 15:1) to obtain compound 15-A2 (1.10 g, percent yield 56.4%, light yellow solid).

**$^1$H-NMR (400 MHz, DMSO-d6):** δ10.05 (s, 1H), 8.30 (d, J = 8.3 Hz, 1H), 7.92 (dd, J = 8.3, 1.5 Hz, 1H), 7.78 (d, J = 1.5 Hz, 1H), 7.69-7.55 (m, 3H), 7.36-7.28 (m, 2H), 7.27-7.21 (m, 1H), 7.09 (dd, J = 8.5, 2.3 Hz, 1H).

Under nitrogen protection, compoundd 15-A2 (700 mg, 1.970 mmol) was dissolved in anhydrous THF (10 mL), TTMS (476mg, 3.35mmol, 98%) was added to the reaction solution, the reaction solution was kept at 0°C for 1.5 h and the reaction was finished. 3N HCl (2 mL) was added to the reaction solution, and the reaction solution was natural warmed up to room temperature and stirred for 1 h.

5 mL $H_2O$ was added to the reaction solution, the reaction solution was extracted by DCM (10 mL×3), washed

by water (5 mL×3), dried, concentrated, and separated by column chromatography (200-300 mesh silica gel, n-heptane:EA = 15:1-10:1) to obtain compound 15-A3 (810 mg, percent yield 96.7%, yellow oily substance).

**$^1$H-NMR(400 MHz, DMSO-d6):** δ8.19 (d, J = 8.5 Hz, 1H), 7.63-7.52 (m, 4H), 7.44 (s, 1H), 7.32 (t, J = 8.9 Hz, 2H), 7.21 (d, J = 5.8 Hz, 1H), 7.14 (dd, J = 11.7, 2.4 Hz, 1H), 6.97 (dd, J = 8.5, 2.1 Hz, 1H), 5.44-5.37(m, 1H).

Under nitrogen protection, $POCl_3$ (580 mg, 3.76 mmol, 97%) was dissolved in anhydrous DCM (10 mL), and the reaction solution was cooled to -40°C. Compound 15-A3 (800 mg, 1.88 mmol) in DCM (4 mL) was added, and TEA (476 mg, 4.70 mmol) was added to the reaction solution, the reaction solution was kept at -40°C to -35°C for 2 h. LC-MS was used to detect the extent of reaction. When compound 15-A3 was vanished and transferred to intermediate, and the temperature was -40°C, 2-bromoethylamine hydrobromide (3.08 g, 15.04 mmol) was added, TEA (1.52 g, 15.04 mmol) in DCM (2 mL) was added to the reaction solution, the temperature was kept at -40°C for 1 h, and the intermediate was totally transferred.

The reaction solution was natural warmed up to room temperature, saturated $NH_4Cl$ aqueous solution (5 mL) was added to the reaction solution. The reaction solution was extracted by DCM (10 mL×3), washed by water (3 mL×3), dried, concentrated, and separated by column chromatography (200-300 mesh silica gel, n-heptane:EA = 1:1-100% EA) to obtain compound 15-A4 (600 mg, percent yield 44.4%, white solid).

**$^1$H-NMR(400 M, $CDCl_3$):** δ8.04 (d, J = 8.4 Hz, 1H), 7.55-7.47 (m, 2H), 7.46-7.38 (m, 2H), 7.27 (s, 1H), 7.19-7.10 (m, 2H), 6.95-6.83 (m, 2H), 5.69 (dd, J = 11.4, 6.1 Hz, 1H), 3.50-3.18 (m, 8H).

**MS:** Calculated 716.9, found 717.8 ($[M+H]^+$).

Under nitrogen protection, compound 15-A4 (600 mg, 0.837 mmol) was dissolved in THF (15 mL), $Ag_2O$ (1.16 mg, 5.02 mmol) and DIPEA (649 mg, 5.02 mmol) were added to the reaction solution, the reaction solution was warmed up to 65°C and stirred for 3 h.

After the reaction was finished, the reaction solution was cooled to room temperature, separated by the suction filtration with the diatomaceous earth, the solid was washed by DCM, the original solution was concentrated, 1.5 mL of anhydrous diethyl ether was added to the reaction solution, and the reaction solution was separated by column chromatography to obtain the pure product of compound 15 (56 mg, percent yield 48.6%, white solid).
**$^1$H-NMR(400 MHz, MeOD):** δ8.12 (t, J = 8.1 Hz, 1H), 7.55 (m, 4H), 7.45 (s, 1H), 7.19 (t, J = 8.8 Hz, 2H), 6.98 (dd, J = 7.8, 5.3 Hz, 2H), 6.15-5.98 (m, 1H), 2.39-1.93 (m, 8H).

**MS:** Calculated 555.1, found 556.1 ($[M+H]^+$).

Synthesis of **compound 16**

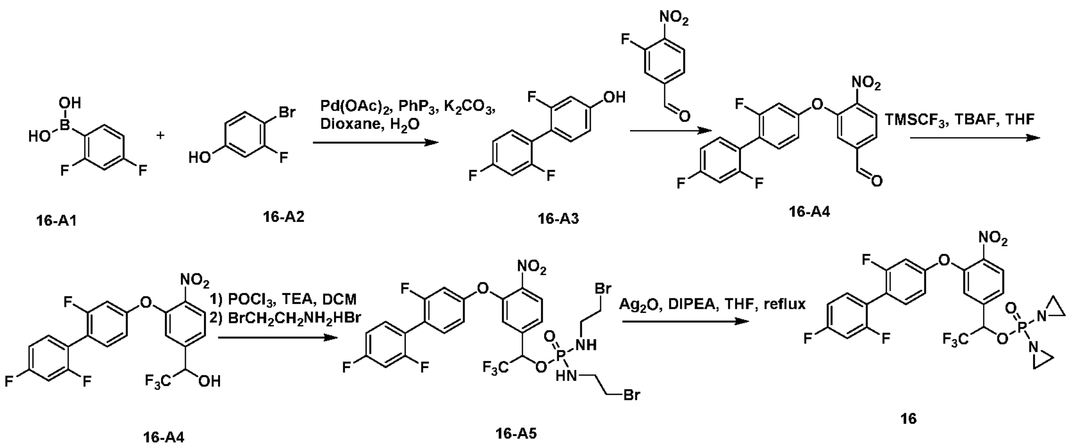


16-A1 16-A2 16-A3 16-A4 16-A4 16-A5 16

Under nitrogen protection, compound 16-A1 (1.7 g, 11 mmol), compound 16-A2 (2 g, 10 mmol), $Pd(OAc)_2$ (21.5 mg, 0.1 mmol) and $PPh_3$ (39mg, 0.15 mmol) and $K_2CO_3$ (2.8 g, 20 mmol) were dissolved in dioxane (20 mL) and water (2 mL), the reaction solution was warmed up to 80°C and stirred overnight.

After the reaction was finished, the reaction solution was separated by the suction filtration with the diatomaceous earth, concentrated, stirred, and separated by column chromatography (100-200 mesh silica gel, n-heptane:EA = 5:1) to obtain compound 16-A3 (900 mg, percent yield 40%, white solid).

**$^1$H-NMR(300 MHz, DMSO):** δ 10.17 (s, 1H), 7.55-7.08 (m, 3H), 6.79-6.50 (m, 3H).MS: Calculated 224.0, found 222.6[$(M-H)^-$].

Under nitrogen protection, compound 16-A3 (550 mg, 2.2 mmol) and 3-fluoro-4-nitrobenzoaldehyde (370 mg,

2.2 mmol) were dissolved in MeCN (5 mL), $K_2CO_3$ (830 mg, 6 mmol) was added under nitrogen, the reaction solution was warmed up to 80°C and stirred for 4 h.

After the reaction was finished, the reaction solution was separated by the suction filtration with the diatomaceous earth, concentrated, stirred, separated by column chromatography (100-200 mesh silica gel, n-heptane:EA = 10:1) to obtain the crude product of compound 16-A4 (700 mg, purity 76.2%, white solid), and the crude product was used to the next step directly.

**$^1$H-NMR (300 MHz, DMSO):** δ 10.03 (s, 1H), 8.31 (d, J = 8.2 Hz, 1H), 7.94 (d, J = 8.4 Hz, 1H), 7.83 (s, 1H), 7.59-7.50 (m, 2H), 7.40 (t, J = 8.8 Hz, 1H), 7.32-7.16 (m, 2H), 7.10 (d, J = 8.6 Hz, 1H).

Under nitrogen protection, compound 16-A4 (680 mg, 1.82 mmol) and TTMS (510 mg, 3.6 mmol) were dissolved in THF (8 mL), TBAF (0.1 mL, 0.1 mmol, 1M, Energy Chemical) in THF was added to the reaction solution at 0°C, and the temperature was kept at for 6 h. 1N HCl (2 mL) was added to the reaction solution and the reaction solution was stirred for 10 min.

The reaction solution was concentrated to separated THF, the crude product was extracted by water (10 mL) and DCM (20 mL), the organic phase was separated by concentration and stir, the crude product was separated by column chromatography (100-200 mesh silica gel, n-heptane:EA = 10:1) to obtain compound 16-A5 (400 mg, percent yield 49.6%, purity 90%).

**$^1$H-NMR(300 MHz, DMSO):** δ 8.19 (d, J = 7.8 Hz, 1H), 7.76 (t, J = 8.5 Hz, 1H), 7.57-7.51 (m, 2H), 7.38 (s, 1H), 7.28-7.19 (m, 3H), 6.89 (dd, J = 8.8, 1.7 Hz, 1H), 5.40-5.36 (m, 1H).

Under nitrogen protection, $POCl_3$ (168 mg, 1.1 mmol, J&K Scientific) was dissolved in ultra-dry DCM (5 mL), and the reaction solution was cooled to -30°C. Compound 16-A5 (220 mg, 0.5 mmol) was dissolved in DCM (5 mL) and TEA (170 mg, 1.65 mmol) was added, the temperature was kept at -30°C. After 6 h, the raw materials were totally vanished, 2-bromoethylamine hydrobromide (897 mg, 4.4 mmol) was added to the reaction solution at -30°C, and then TEA (440 mg, 4.4 mmol) was added.

After the reaction was finished, the temperature was cooled down to 0°C, saturated $NH_4Cl$ aqueous solution (10 mL) was added to the reaction solution. The reaction solution was extracted by DCM (15 mL×2), washed by water (5 mL×4), dried, and concentrated to obtain the crude product of compound 16-A6 (250 mg, yellow solid). The crude product was used to the next step directly.

**MS:** Calculated 732.9, found 733.9 ($[M+H]^+$).

Under nitrogen protection, compound 16-A6 (250 mg, 0.34 mmol) was dissolved in THF (10 mL), $Ag_2O$ (210 mg, 1.7 mmol) and DIPEA (220 mg, 1.7 mmol) were added to the reaction solution, and the temperature was warmed up to 65°C.

After 2h, the reaction was finished, the reaction solution was separated by the suction filtration with the diatomaceous earth, the solid was washed by DCM (20 mL), the original solution was concentrated and separated by column chromatography to obtain the pure product of compound 16 (22 mg, percent yield 11%, white solid).

**$^1$H-NMR (400 MHz, DMSO):** δ 8.26 (d, J = 8.5 Hz, 1H), 7.67 (d, J = 8.5 Hz, 1H), 7.59-7.48 (m, 3H), 7.41 (dt, J = 10.3, 2.5 Hz, 1H), 7.24-7.19 (m, 2H), 7.03 (dd, J = 8.5, 2.3 Hz, 1H), 6.36-6.32 (m, 1H), 2.20-1.91 (m, 8H).

**MS:** Calculated 573.1, found 574.1 ($[M+H]^+$).

Synthesis of **compound 17**

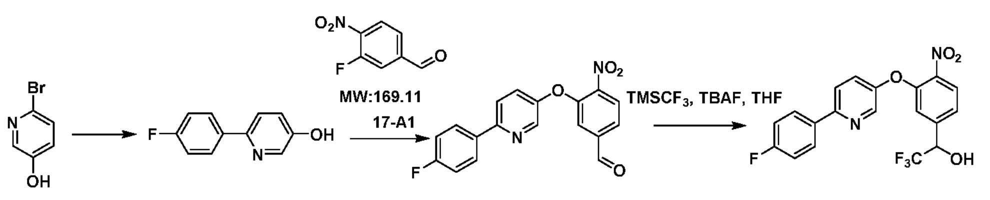


17-A0 17-A2 17-A3

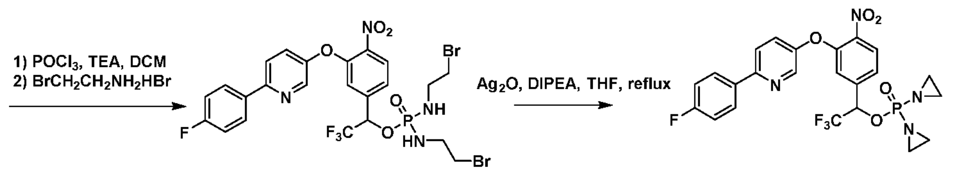


17-A4 17

Under nitrogen protection, 2-bromo-5-hydroxypyridine (3.0 g, 17.2 mmol) and 4-fluorophenylboronic acid (compound 17-A0, 2.7 g, 19.0 mmol) were dissolved in the mixed solution of dioxane and water (60 mL, dioxane:water = 9:1), the gas was exchanged 3 times, $Pd(OAc)_2$ (193 mg, 0.86 mmol) and $PPh_3$ (225 mg, 0.86 mmol) were added to the reaction solution, the gas was exchanged 3 times again, the reaction solution was warmed up to 100°C and stirred overnight.

After the reaction was finished, the reaction solution was cooled to room temperature, separated by the suction filtration with the diatomaceous earth and washed by EA. The original solution was concentrated, 15 mL of water was added to the reaction solution, the reaction solution was extracted by EA (50 mL×3), washed by water (10 mL×3), washed by brine, dried, concentrated, and separated by column chromatography (200-300 mesh silica gel, n-heptane:EA = 15:1) to obtain compound 17-A1 (1.2 g, percent yield 36.9%, white solid).

**$^1$H-NMR(400 M, DMSO-d6):** δ10.03 (s, 1H), 8.20 (d, J = 2.7 Hz, 1H), 8.00 (dd, J = 8.8, 5.6 Hz, 2H), 7.78 (d, J = 8.6 Hz, 1H), 7.32-7.18 (m, 3H).

**MS:** Calculated 189.1, found 190.2 ($[M+H]^+$).

Under nitrogen protection, compound 17-A1 (600 mg, 3.55 mmol) and compound 18-A0 (806 mg, 4.26 mmol) were dissolved in acetonitrile (15 mL), $K_2CO_3$ (980 mg, 7.1 mmol), the reaction solution was warmed up to 85°C and stirred for 2 h.

After the reaction was finished, the reaction solution was cooled to room temperature, separated by the suction filtration with the diatomaceous earth, the original solution was concentrated and separated by column chromatography (200-300 mesh silica gel, n-heptane:EA = 10:1) to obtain compound 17-A2 (760 mg, percent yield 63.3%, light yellow solid).

**$^1$H-NMR(400 MHz, DMSO-d6):** δ ppm10.03 (s, 1H), 8.59 (d, J = 2.8 Hz, 1H), 8.31 (d, J = 8.3 Hz, 1H), 8.19-8.11 (m, 2H), 8.07 (d, J = 8.7 Hz, 1H), 7.90 (dd, J = 8.3, 1.6 Hz, 1H), 7.78-7.70 (m, 2H), 7.37-7.26 (m, 2H).

**MS:** Calculated 338.1, found 339.0 ($[M+H]^+$).

Under nitrogen protection, compound 17-A2 (700 mg, 2.07 mmol) was dissolved in THF (10 mL), TTMS (500 mg, 3.52 mmol) was added to the reaction solution, the reaction solution was cooled down to 0°C, TBAF (0.03mL, 1M in THF) was added, the temperature was kept at 0°C, and the reaction was finished.

3N HCl (2 mL) was added to the reaction solution, the reaction solution was natural warmed up to room temperature and stirred for 1 h. 5 mL water was added to the reaction solution, the reaction solution was extracted by DCM (10 mL×3), washed by water, dried, concentrated, and separated by column chromatography (200-300 mesh silica gel, n-heptane:EA = 15:1-10:1) to obtain compound 17-A3 (550 mg, percent yield 65.1%, yellow oily substance).

**$^1$H-NMR(400 MHz, DMSO-d6):** δ8.52 (d, J = 2.9 Hz, 1H), 8.19 (d, J = 8.5 Hz, 1H), 8.16-8.10 (m, 2H), 8.06 (d, J = 8.8 Hz, 1H), 7.64 (dd, J = 8.8, 2.9 Hz, 1H), 7.55 (dd, J = 8.7, 3.3 Hz, 1H), 7.38 (s, 1H), 7.36-7.28 (m, 2H), 7.18 (dd, J =5.8, 3.4 Hz, 1H), 5.43-5.34 (m, 1H).

**MS:** Calculated 408.1, found 409.2 ($[M+H]^+$).

Under nitrogen protection, $POCl_3$ (414 mg, 2.70 mmol) was dissolved in DCM (10 mL), and the temperature was cooled to -40°C. Compound 17-A3 (550 mg, 1.35 mmol) was dissolved in DCM (4 mL) and added to the reaction solution with TEA (342 mg, 3.36 mmol), the temperature was kept at -40°C to -35°C for 2 h. LC-MS was used to detect the extent of reaction. When compound 17-A3 was vanished, transferred to intermediate, and the temperature was -40°C, 2-bromoethylamine hydrobromide (2.2 g, 10.8 mmol) was added to the reaction solution, TEA (101 g, 10.8 mmol) in DCM (2 mL) was added, the temperature was kept at -40°C for 1 h, and the intermediate was totally transferred.

The reaction solution was natural warmed up to 0°C, saturated $NH_4Cl$ aqueous solution (5 mL) was added. The reaction solution was extracted by DCM (10 mL×3), washed by water (3 mL×3), dried, concentrated and separated by column chromatography (200-300 mesh silica gel, n-heptane:EA = 1:1-100% EA) to obtain compound 17-A4 (520 mg, percent yield 55.0%, white solid).

**$^1$H-NMR(400 M, CDCl3):** δ8.49 (d, J = 2.7 Hz, 1H), 8.06 (dd, J = 8.4, 2.7 Hz, 1H), 8.01-7.94 (m, 2H), 7.77 (d, J = 8.7 Hz, 1H), 7.54-7.48 (m, 1H), 7.43 (d, J = 8.3 Hz, 1H), 7.26 -7.24 (m, 1H), 7.18 (t, J = 8.7 Hz, 2H), 5.69 (dq, J = 12.3, 6.2 Hz, 1H), 3.49-3.16 (m, 8H).

**MS:** Calculated 700.0, found 700.9 ($[M+H]^+$).

Under nitrogen protection, compound 17-A4 (520 mg, 0.743 mmol) was dissolved in THF (15 mL), $Ag_2O$ (1.03 g, 4.46 mmol) and DIPEA (580 mg, 4.46 mmol) were added to the reaction solution, the reaction solution was warmed up to 65°C and stirred for 3 h.

After the reaction was finished, the reaction solution was cooled to room temperature, separated by the suction filtration with the diatomaceous earth, the solid was washed by DCM, the original solution was concentrated and separated by column chromatography to obtain the pure product of compound 18 (106.7 mg, percent yield 26.7%, white solid).

**$^1$H-NMR(400 MHz, MeOD):** δ8.43 (d, J = 2.9 Hz, 1H), 8.16 (d, J = 8.5 Hz, 1H), 8.07-7.97 (m, 2H), 7.92 (d, J = 8.8 Hz, 1H), 7.65-7.54 (m, 2H), 7.46 (s 1H), 7.24-7.19 (m, 2H), 6.13-6.03 (m, 1H), 2.26-2.08 (m, 8H).

**MS:** Calculated 538.1, found 539.1 ($[M+H]^+$).

Synthesis of **compound 18**

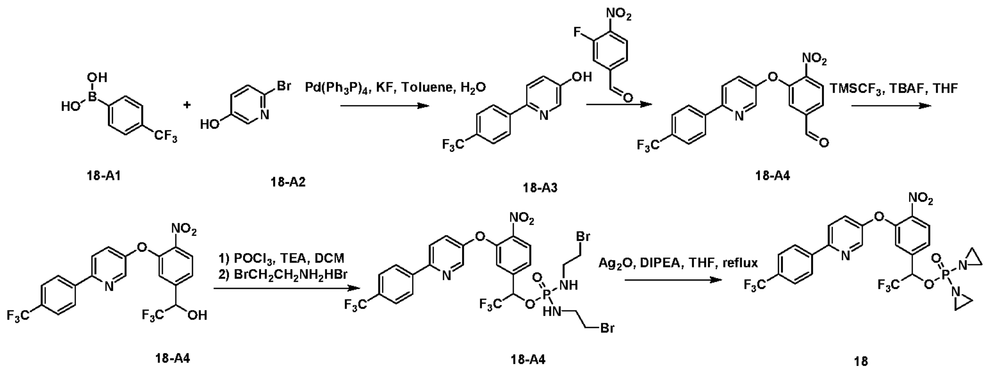


18-A1 18-A2 18-A3 18-A4 18-A4 18-A4 18

Under nitrogen protection, compound 18-A1 (500 mg, 2.65 mmol) and compound 18-A2 (460 mg, 2.65 mmol), $Pd(PPh_3)_4$ (300 mg, 5.2 mmol) were dissolve in toluene (10 mL) and water (1 mL). The reaction solution was warmed up to 80°C and stirred overnight.

After the reaction was finished, the reaction solution was separated by the suction filtration with the diatomaceous earth, concentrated, stirred, and separated by column chromatography (100-200 mesh silica gel, n-heptane:EA = 10:1) to obtain compound 18-A3 (600 mg, percent yield 94.7%, white solid).

**$^1$H-NMR(300 MHz, DMSO):** δ 10.24 (s, 1H), 8.26 (d, J = 2.4 Hz, 1H), 8.19 (d, J = 8.1 Hz, 2H), 7.91 (d, J = 8.7 Hz, 1H), 7.78 (d, J = 8.1 Hz, 2H), 7.29 (dd, J = 8.6, 2.7 Hz, 1H).

**MS:** Calculated 239.0, found 240.0 ($[M+H]^+$).

Under nitrogen protection, compound 18-A3 (870 mg, 2.9 mmol) and 3-fluoro-4-nitrobenzoaldehyde (490 mg, 2.9 mmol) were dissolved in MeCN (8 mL), $K_2CO_3$ (830 mg, 6 mmol) was added to the reaction solution, the reaction solution was warmed up to 80°C and stirred for 4 h.

After the reaction was finished, the reaction solution was separated by the suction filtration with the diatomaceous earth, concentrated, stirred, and separated by column chromatography (100-200 mesh silica gel, n-heptane:EA = 10:1) to obtain compound 18-A4 (640 mg, white solid).

**$^1$H-NMR (300 MHz, $CDCl_3$):** δ 10.03 (s, 1H), 8.57 (d, J = 2.7 Hz, 1H), 8.15-8.12 (m, 3H), 7.87-7.75 (m, 4H), 7.60-7.51 (m, 2H).

**MS:** Calculated 388.0, found 389.1 ($[M+H]^+$).

Under nitrogen protection, compound 18-A4 (640 mg, 1.64 mmol) and TTMS (430 mg, 3 mmol) were dissolved in THF (5 mL), TBAF in THF (0.1 mL, 0.1 mmol, 1M) was added at 0°C, the reaction solution was kept at for 6 h, 1N HCl (2 mL) was added and stirred for 10 min. THF in the reaction solution was separated by concentrated. The crude product was extracted by water (10 mL) and DCM (20 mL), the organic phase was separated by concentrated and stirred, and the crude product was separated by column chromatography (100-200 mesh silica gel, n-heptane:EA = 10:1) to obtain the product (640 mg, percent yield 85.2%, light yellow solid).

$^1$H-NMR(300 MHz, DMSO): δ 8.59 (d, J = 2.7 Hz, 1H), 8.30 (d, J = 7.5 Hz, 2H), 8.19 (t, J = 9.2 Hz, 2H), 7.86 (d, J = 8.5 Hz, 2H), 7.72-7.65 (m,1H), 7.59-7.56 (m, 1H), 7.43 (s, 1H), 7.20 (d, J = 5.8 Hz, 1H), 5.39-5.37 (m, 1H).

MS: Calculated 458.0, found: 459.0 ($[M+H]^+$).

Under nitrogen protection, $POCl_3$ (200 mg, 1.3 mmol, 97%) was dissolved in ultra-dry DCM (10 mL), and the temperature was cooled to -30°C. Compound 18-A5 (300 mg, 0.65 mmol) in DCM (5 mL) was added, TEA (270 mg, 2.6 mmol) was added, the temperature was kept at -30°C to -35°C, the raw materials were totally vanished after 6 h. 2-Bromoethylamine hydrobromide (1.1 g, 5.2 mmol) was added to the reaction solution at -30°C, and TEA (530 mg, 5.2 mmol) was added.

After the reaction was finished, the reaction solution was cooled down to 0°C, saturated $NH_4Cl$ aqueous solution (10 mL) was added. The reaction solution was extracted by DCM (15 mL×2), washed by water (5 mL×4), dried, and concentrated to obtain 200 mg of the crude product, the crude product was yellow solid and used to the next reaction directly.

**1H NMR (400 MHz, CDCl3):** δ 8.46 (d, J=2.6 Hz, 1H), 8.07-7.99 (m, 3H), 7.78-7.75 (m, 1H), 7.68 (d, J = 8.2 Hz, 2H), 7.48-7.33 (m, 3H), 5.65-5.61 (m, 1H), 3.51-3.04 (m, 10H).

**MS:** Calculated 747.9, found 748.9 ($[M+H]^+$).

Under nitrogen protection, compound 18-A6 (150 mg, 0.23 mmol) was dissolved in THF (10 mL), $Ag_2O$ (170 mg, 1.38 mmol, Energy Chemical) and DIPEA (163 mg, 1.38 mmol) were added to the reaction solution, the temperature

was warmed up to 65°C to reaction.

After 2 h, the reaction was finished, the reaction solution was separated by the suction filtration with the diatomaceous earth, the solid was washed by DCM (20 mL), and the original solution was concentrated and separated by column chromatography to obtain the pure product of compound 18(41 mg, percent yield 36%, white solid).

**$^1$H-NMR (400 MHz, DMSO):** δ 8.61 (d, J = 2.8 Hz, 1H), 8.31 (d, J = 8.2 Hz, 2H), 8.27 (d, J = 8.5 Hz, 1H), 8.21 (d, J = 8.8 Hz, 1H), 7.87 (d, J = 8.4 Hz, 2H), 7.72 (dd, J = 8.8, 2.9 Hz, 1H), 7.65 (d, J = 8.5 Hz, 1H), 7.56 (s, 1H), 6.32-6.28 (m, 1H), 2.17-1.90 (m, 8H).

**MS:** Calculated 588.1, found 589.1 ($[M+H]^+$).

Synthesis of **compound 19**

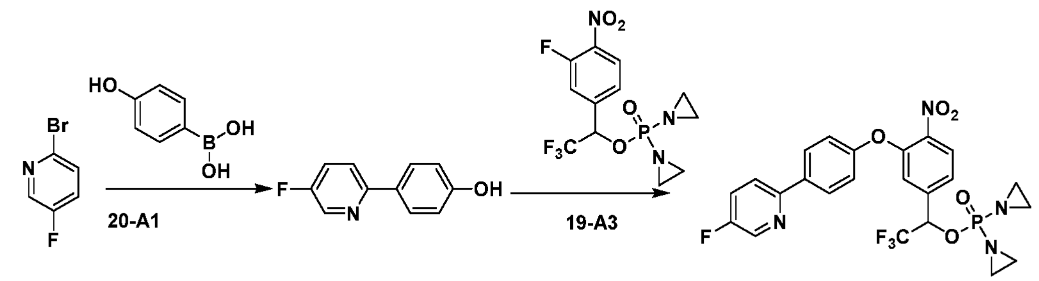


19-A0 19-A2 19

Under nitrogen protection, 2-bromo-5-hydroxypyridine (1.5 g, 8.52 mmol, purchased) and 4-fluorophenylboronic acid (1.4 g, 10.23 mmol) were added to the mixed solution of DME (33 mL) and $H_2O$ (7 mL), the nitrogen was exchanged 3 times, $Pd(PPh_3)_4$ (300 mg, 0.26 mmol) and $Na_2CO_3$ (1.8 g, 17.05 mmol) were added, the nitrogen was exchanged 3 times again, and the reaction solution was warmed up to 80°C to reaction. The reaction was under supervision for 2.5 h.

After the reaction was finished, the reaction solution was cooled down to room temperature, extracted by EtOAc (50 mL×3), the organic phase was washed by water, washed by brine, and separated by column chromatography (200-300 mesh silica gel:n-heptane:EA = 12:1-9:1) to obtain the product (1.4 g, percent yield 86.8%, white solid).

**$^1$H-NMR(300 MHz, MeOD):** δ8.43 (d, J = 2.7 Hz, 1H), 7.81 - 7.77 (m, 3H), 7.64 - 7.58 (m, 1H), 6.87 (d, J = 8.7 Hz, 2H).

**MS:** Calculated 189.1, found 190.1 ($[M+H]^+$).

Under nitrogen protection, compound 19-A3 (i.e. no. 46 compound, 100 mg, 0.27 mmol) was dissolved in acetone (5 mL), compound 19-A2 (10 mg, 0.54 mmol) and $Cs_2CO_3$ (309 mg, 0.95 mmol) was stirred at room temperature and stirred for 2 h.

After the reaction was finished, the reaction solution was separated by the suction filtration with the diatomaceous earth, the solid was washed by acetone, the original solution was concentrated and separated by column chromatography to obtain the pure product of compound 20 (17 mg, percent yield 11.7%, brown solid).

**$^1$H-NMR(400 MHz, MeOD):** δ 8.52 (d, J = 2.9 Hz, 1H), 8.10 (d, J = 8.5 Hz, 1H), 8.06 (d, J = 8.8 Hz, 2H), 7.92 (dd, J = 8.8, 4.3 Hz, 1H), 7.68 (dt, J = 8.6, 2.9 Hz, 1H), 7.54 (d, J = 8.6 Hz, 1H), 7.37 (s, 1H), 7.19 (d, J = 8.8 Hz, 2H), 6.06-5.99 (m, 1H), 2.27-1.97 (m, 8H).

**MS:** Calculated 538.1, found 539.1 ($[M+H]^+$).

Synthesis of **compound 46**

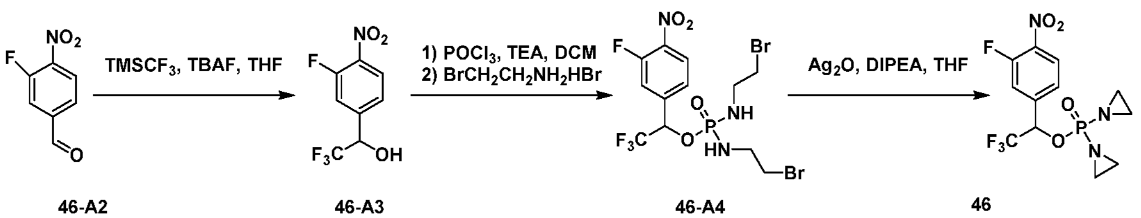


46-A2 46-A3 46-A4 46

Under nitrogen protection, compound 46-A2 (2.0 g, 11.8 mmol, purchased) and $TMSCF_3$ (2.5 g, 17.7 mmol) were dissolved in THF (20 mL), the reaction solution was cooled down to 0°C, TBAF (2.6ml, 0.26 mmol, 1M in THF) was added. After the temperature was kept at 0°C for 30 min, compound 46-A2 was totally vanished, 3N HCl (2 mL) was added, the reaction solution was became clear and keepinf stirring at 0°C for 30 min, the mixture was totally transferred to the product.

The product was extracted by DCM (10 ml×3), the organic phase was washed by water (10 ml×3), dried, concentrated, and separated by column chromatography (200-300 mesh silica gel, n-heptane:EA = 12:1-10:1) to obtain compound 46-A3 (1.5 g, percent yield 53.2%, yellow oily substance).

**$^1$H-NMR(400 MHz, $CDCl_3$):** δ8.13 - 8.09 (m, 1H), 7.60 (d, J =11.6 Hz, 1H), 7.43 (d, J =8.4 Hz, 1H), 5.12 -5.18 (m, 1H), 3.06 (d, J = 4.4 Hz, 1H).

Under nitrogen protection, $POCl_3$ (963 mg, 4.61 mmol) was dissolved in DCM (10 mL), and the temperature was cooled to -40°C. Compound 46-A3 (1.5 g, 6.27 mmol) was dissolved in DCM (20 mL) and added to the reaction solution with TEA (10.1 g, 0.1 mmol), the temperature was kept at -40°C to -35°C. After 2 h. Compound 46-A3 was transferred to intermediate, 2-bromoethylamine hydrobromide (11.99g, 50.16 mmol) and TEA (10.1 g, 0.1 mol) were added to the reaction solution, and the reaction solution was under supervision. After 30 min, the reaction was finished.

Saturated $NH_4Cl$ aqueous solution (20 mL) was added to the reaction solution at 0°C, the reaction solution was extracted by DCM (50 mL×3), the organic phase was washed by water, washed by brine, dried, concentrated and separated by column chromatography (200-300 mesh silica gel, n-heptane:EA = 5:1-1:1) to obtain compound 46-A4 (1.6 g, percent yield 65.3%, yellow oily substance).

**$^1$H-NMR(400 MHz, $CDCl_3$):** δ8.15-8.11 (m, 1H), 7.47 (d, J =11.6 Hz, 1H), 7.44 (d, J =8.8 Hz, 1H), 5.78-5.30 (m, 1H), 3.53-3.05 (m, 10H).

**MS:** Calculated 529.9, found 531.9 ($[M+H]^+$).

Under nitrogen protection, compound 46-A4 (1.6 g, 3.0 mmol) was dissolved in THF (20 mL), $Ag_2O$ (4.2 g, 18.0 mmol) and DIPEA (2.3 g, 18.0 mmol) were added to the reaction solution, the reaction solution was warmed up to 65°C and stirred for 1.5 h.

After the reaction was finished, the reaction solution was cooled to room temperature and separated by the suction filtration with the diatomaceous earth, the solid was washed by DCM, the original solution was concentrated and separated by column chromatography (200-300 mesh silica gel, n-heptane:EA = 5:1-1:1) to obtain the crude product, and the crude product was separated by column chromatography to obtain the pure product of compound 46 (30 mg, white solid).

**$^1$H NMR (400 MHz, MeOD):** δ8.24-8.20 (m, 1H), 7.70 (d, J =11.6 Hz, 1H), 7.63 (d, J =8.8 Hz, 1H), 6.14-6.10 (m, 1H), 2.28-2.14 (m, 8H).

**MS:** Calculated 369.1, found 370.1 ($[M+H]^+$).

## Claims

1. An anti-cancer compound for acting as a non-PGP substrate, wherein the compound is a compound having a formula 1-1, or a pharmaceutically acceptable salt or a solvate thereof:

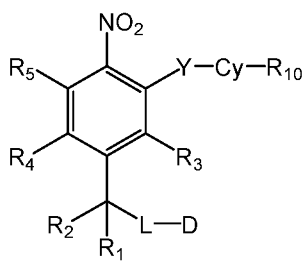

I-1

wherein,

$R_1$ and $R_2$ are respectively independently hydrogen, deuterium, an aryl group or a Z-substituted aryl group, a heteroaryl group, a heterocycle or a Z-substituted heteroaryl group, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group;

$R_3$ is hydrogen, halogen, a cyano group or an isocyano group, a thiocyanato group or an isothiocyanato group, hydroxyl group, a thiol group, an amino group, oxime, hydrazone, OTs, OMs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, or

$R_3$ is $-CONR^6R^7$, $-SO_2NR^6R^7$, $-SO_2R^6$, $-OCO-R^6$,$-OCOO-R^6$, $-COOR^6$, $-NR^6COR^7$, $-OCOR^6$, $-NR^6SO_2R^7$, and $-NR^6CONR^7$;

$R_4$ and $R_5$ are respectively independently hydrogen, halogen, a cyano group or an isocyano group, a thiocyanato group or an isothiocyanato group, a hydroxyl group, a thiol group, an amino group, oxime, hydrazone, OTs, OMs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$, $-NR^6CONR^7$, or

$R_4$ and $R_5$ and the atom of the bonding benzene ring bonded thereto form a 7-15 membered fused ring or a Z-substituted fused ring;

$R^6$ and $R^7$ are each independently hydrogen, a cyano group or an isocyano group, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4-15 membered heterocycle or a Z-substituted heterocycle, a 5-15 membered heteroaryl group or a Z-substituted heteroaryl group, a $C_1$-$C_6$ alkoxyl group or a Z- substituted $C_1$-$C_6$ alkoxyl group, or

$R^6$ and $R^7$ and atoms bonded thereto form a 3-7 membered heterocyclyl group or a Z-substituted 3-7 membered heterocyclyl group;

Cy is a 5-10 membered aromatic ring, heterocyclic ring or heteroaromatic ring, and hydrogens of the ring are each independently substituted by deuterium, halogen, a cyano group or an isocyano group, a thiocyanato group or an isothiocyanato group, a hydroxyl group, a thiol group, an amino group, oxime, hydrazone, OTs, OMs, a $C_1$-$C_6$ aliphatic group, or the

hydrogens of the ring are each independently substituted by $C_1$-$C_6$ aliphatic group substituted by halogen, a cyano group, an isocyano group, a hydroxyl group, a thiol group, an amino group, oxime, hydrazone;

$R_{10}$ is a 3-18 membered cyclohydrocarbyl group, an aryl group or a fused ring, a heterocycle, a fused heterocycle, a heteroaryl group or a Z-substituted $C_3$-$C_{18}$ cycloalkyl group, an aryl or a fused ring, heterocycle, a fused heterocyclyl group, a heteroaryl group or a $C_1$-$C_{18}$ hydrocarbyl group or a Z-substituted hydrocarbyl group; or $R_{10}$ is -Q-Cz,

Q is -O-, -S-, -CO-, $-SO_2-$, -SO-, -OCO-, -OCOO-, $-NR^6CO-$, $-NR^6SO_2-$, $-OCONR^6$; and

Cz is a 3-18 membered cyclohydrocarbyl group, an aryl group or a fused ring, a heterocycle, a fused heterocycle, a heteroaryl group or a Z-substituted 3-18 membered cycloalkyl group, an aryl group or a fused ring, a heterocycle, a fused heterocyclyl group, a heteroaryl group or a $C_1$-$C_{18}$ hydrocarbyl group or a Z-substituted hydrocarbyl group;

Y is -O- or -S- or $-SO_2-$, -SO-;

L and D are selected from the following three situations:

(1) L is selected from -O-, -S-, -OCOO-, $-NR^6CO-$, -OCO-, $-NR^6SO_2-$, $-OCONR^6-$, quaternary ammonium, $-OSO_2$,

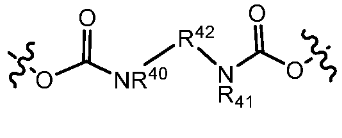 ,

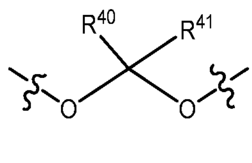 , 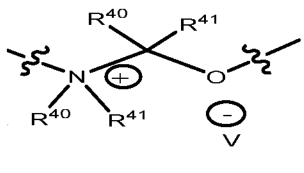 , 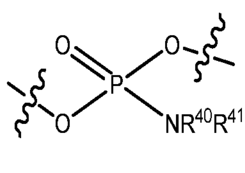 ,

$R^{40}$ and $R^{41}$ are independently hydrogen, a $C_1$-$C_6$ alkyl group, a $C_2$-$C_6$ alkenyl group, a $C_2$-$C_6$ alkynyl group, a $C_3$-$C_8$ cycloalkyl group, a $C_6$-$C_{10}$ aryl group, a 4-15 membered heterocycle or a 5-15 membered heteroaryl group;

$R^{42}$ is $C_2$-$C_3$ alkylenyl, heterylene or one to three $C_1$-$C_6$ alkyl-substituted $C_2$-$C_3$ alkylenyl, $C_1$-$C_6$ alkyl-substituted heterocycloalkylene;

V(-) is any anion;

D is a moiety for making D-OH an anti-cancer drug, wherein -OH is an aliphatic group, a phenolic hydroxyl group, or a -OH moiety attached to the phosphate ester;

(2) L is selected from

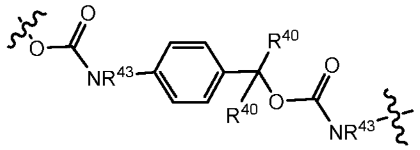

,

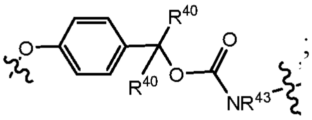

;

$R^{40}$ is defined as above;

$R^{43}$ is hydrogen or form to heterocycle with D,

phenylene is Z-substituted or unsubstituted; and

D is a moiety for making D-$NR^{43}$H an anti-cancer drug; or

(3) L is selected from a bond, -O-C($R^{40}R^{41}$)$_2$, -O-C($R^{40}R^{41}$)-$NR^{40}R^{41}$(+)-C($R^{40}R^{41}$)-, or

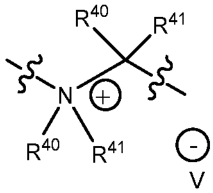

,

wherein

$R^{40}$, $R^{41}$ and V(-) are defined as above; and

D is an anti-cancer drug with a tertiary or secondary nitrogen atom, wherein the tertiary or secondary nitrogen atom is bonded to L; and

an alkyl group, an alkenyl group, an alkynyl group, a cycloalkyl group, an aryl group, a heterocycle, a heteroaryl group, and ether in the definitions of the L and D are Z-substituted or unsubstituted;

the Z-substituted group is halogen, a cyano group or an isocyano group, a thiocyanato group or an isothiocyanato group, a hydroxyl group, a thiol group, an amino group, oxime, hydrazone, OTs, OMs, a $C_1$-$C_3$ alkyl group or a substituted alkyl group, a $C_2$-$C_3$ alkenyl group or a substituted alkenyl group, a $C_2$-$C_3$ alkynyl group or a substituted alkynyl group, a $C_3$-$C_8$ cycloalkyl group or a substituted cycloalkyl group, an aromatic ring, a heterocycle, a heteroaryl group and a fused ring or a substituted aryl group, heterocycle, heteroaryl group and a fused ring, wherein

the substitution manners are mono-substitutied or double-substitutied; and

the substituent is halogen, a cyano group or an isocyano group, a thiocyanato group or an isothiocyanato group, a hydroxyl group, a thiol group, an amino group, oxime, hydrazone, OTs, OMs; and

the compound does not comprise

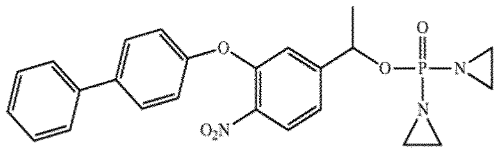,

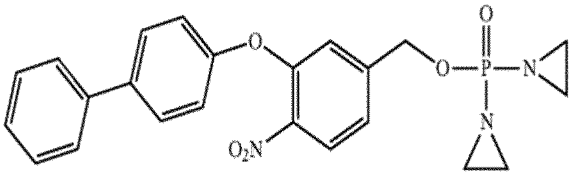,

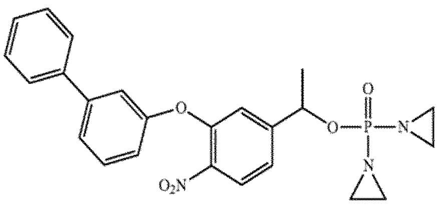, 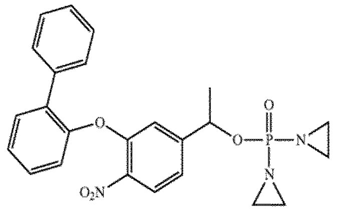,

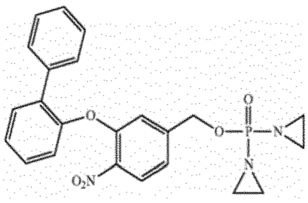, 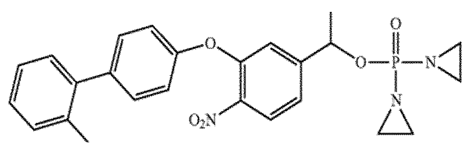,

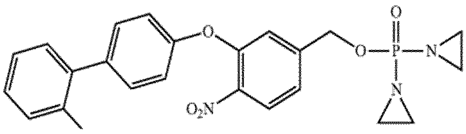, 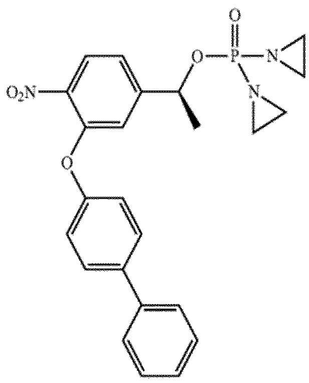,

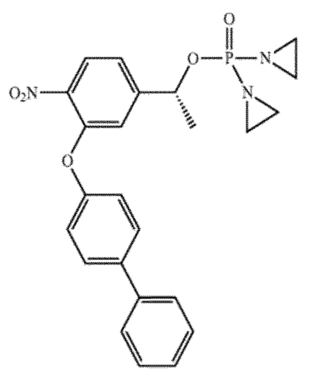, 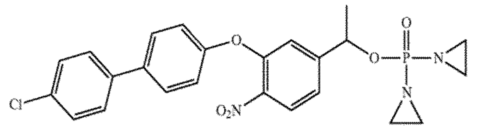,

or

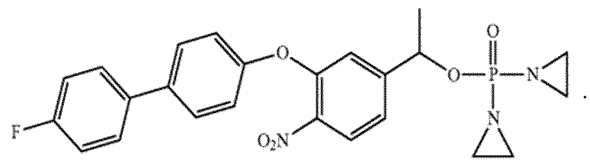

2. The compound of claim 1, wherein

$R_{10}$ is a 3-18 membered cyclic hydrocarbyl group, an aryl group or a fused ring, a heterocycle, fa used heterocycle, a heteroaryl group, or a Z-substituted $C_3$-$C_{18}$ cycloalkyl group, a Z-substituted aryl group or a fused ring, a Z-substituted heterocycle, a Z-substituted fused heterocyclic group, a Z-substituted heteroaryl group, or $-CF_3$ or C1-substituted a hydrocarbyl group; or
$R_{10}$ is -Q-Cz, wherein:

Q is selected from the group consisting of -O-, -S-, -CO-, $-SO_2-$, -SO-, -OCO-, -OCOO-, $-NR^6CO-$, $-NR^6SO_2-$, $-OCONR^6$;
Cz is a 3-18 membered cyclohydrocarbyl group, or a fused ring, a heterocycle, a fused heterocycle, a heteroaryl group or a Z-substituted 3-18-membered cycloalkyl group, a Z-substituted aryl group or a fused ring, a Z-substituted heterocycle, a Z-substituted fused heterocycle group, a Z-substituted heteroaryl group, or $-CF_3$ or C1-substituted a hydrocarbyl group.

3. The compound of claim 1, wherein
-D is $-P(Z^1)(Z^5\text{-}X^5\text{-}Y^5)_n$, wherein

$Z^1$ is O or S, $Z^5$ is N, S, or O; $X^5$ is any substituted ethylidene; $Y^5$ is halogen or $-OSO_2-R^{20}$, $R^{20}$ is any substituted hydrocarbyl group, aryl group, cycloalkyl group, heterocyclyl group and heteroaryl group, and n is 1 or 2; or
$Z^1$ is O or S; $Z^5\text{-}X^5\text{-}Y^5$ is $-NCH_2CH_2$; or
-L- is -O-; -D is $-P(Z^1)(Z^5\text{-}X^5\text{-}Y^5)_n$; $Z^1$ is O or S; $Z^5$ is N, S, or O; $X^5$ is any substituted ethylidene; $Y^5$ is halogen or $-OSO_2-R^{20}$, wherein $R^{20}$ is any substituted hydrocarbyl group, aryl, cycloalkyl group, heterocyclyl group and heteroaryl group, and n is 1 or 2; or
-L- is -O-; $Z^1$ is O or S; and $Z^5\text{-}X^5\text{-}Y^5$ is $-NCH_2CH_2$; or
-L-D is $-OP(Z^1)(NR^{30}CH_2CH_2Cl)_2$, $-OP(Z^1)(NR^{30}CH_2CH_2Br)_2$, $-OP(Z^1)(NR^{30}{}_2)(N(CH_2CH_2X^1)_2)$, $-OP(Z^1)(N(CH_2)_2)_2$, or $-OP(Z^1)(N(CH_2CH_2Cl)_2)_2$, wherein every $R^{30}$ is each independent hydrogen, a $C_1$-$C_6$ hydrocarbyl group, or two $R^{30}$ group and the nitrogen atom bonded thereto form 5-7 membered heterocycle, $Z^1$ is O or S, and $X^1$ is Cl, Br or $-SOS_2Me$; or
-L-D is $-OP(Z^1)(NHCH_2CH_2Cl)_2$, $-OP(Z^1)(NHCH_2CH_2Br)_2$, $-OP(Z^1)(NH_2)(N(CH_2CH_2X^1)_2)$, $-OP(Z^1)(N(CH_2)_2)_2$, or $-OP(Z^1)(N(CH_2CH_2Cl)_2)_2$, and $X^1$ is Cl, Br or $-SOS_2Me$.

4. The compound of claim 1, wherein

L is selected from the group consisting of -O-'-S-'-OCOO-'$-NR^6CO$-'-OCO- ' $-NR^6SO_2$- ' $-OCONR^6$-, quaternary ammonium, $-OSO_2-$;
-D is $-P(Z^1)(Z^5\text{-}X^5\text{-}Y^5)_n$, $Z^1$ is O or S, $Z^5$ is N, S or O, $X^5$ is any substituted ethylidene, $Y^5$ is halogen or $-OSO_2-R^{20}$, $R^{20}$ is any substituted hydrocarbyl group, aryl group, cycloalkyl group, heterocyclyl group, or heteroaryl group, n is 1 or 2.

5. The compound of claim 4, wherein
$Z^1$ is O or S; $Z^5\text{-}X^5\text{-}Y^5$ is $-NCH_2CH_2$.

6. The compound of claim 1, wherein
-L- is -O-; -D is $-P(Z^1)(Z^5\text{-}X^5\text{-}Y^5)_n$; $Z^1$ is O or S; $Z^5$ is N, S or O; $X^5$ is any substituted ethylidene; $Y^5$ is halogen or $-OSO_2-R^{20}$, $R^{20}$ is any substituted hydrocarbyl group, aryl group, cycloalkyl group, heterocyclyl group, or heteroaryl group, and n is 1 or 2.

7. The compound of claim 6, wherein
-L- is -O-; $Z^1$ is O or S; $Z^5\text{-}X^5\text{-}Y^5$ is $-NCH_2CH_2$.

8. The compound of claim 1, wherein -L-D is $OP(Z^1)(NR^{30}CH_2CH_2Cl)_2$, $-OP(Z^1)(NR^{30}CH_2CH_2Br)_2$, $-OP(Z^1)(NR^{30}{}_2)(N(CH_2CH_2X^1)_2)$, $-OP(Z^1)(N(CH_2)_2)_2$, or $-OP(Z^1)(N(CH_2CH_2Cl)_2)_2$, wherein every $R^{30}$ is each independent hydrogen, a $C_1$-$C_6$ hydrocarbyl group, or two $R^{30}$ group and the nitrogen atom bonded thereto form a 5-7 membered heterocycle, $Z^1$ is O or S; $X^1$ is Cl, Br or $-SOS_2Me$.

9. The compound of claim 8, wherein

-L-D is $-OP(Z^1)(NHCH_2CH_2Cl)_2$, $-OP(Z^1)(NHCH_2CH_2Br)_2$, $-OP(Z^1)(NH_2)(N(CH_2CH_2X^1)_2)$, $-OP(Z^1)(N(CH_2)_2)_2$, or $-OP(Z^1)(N(CH_2CH_2Cl)_2)_2$; and
$X^1$ is Cl, Br or $-SOS_2Me$.

10. The compound of claim 1, wherein

$R_{10}$ is a 5-18 membered cycloalkyl group, an aryl group or a fused ring, a heterocycle, a fused heterocycle, a heteroaryl group, or a Z-substituted 5-18 membered cycloalkyl group, an aryl group or a fused ring, a heterocycle, a fused heterocycle, a heteroaryl group, or a $-CF_3$ or C1 substituted hydrocarbyl group; or
$R_{10}$ is -O-Cz, Cz is a 5-18 membered cycloalkyl group, an aryl group, a fused ring, a heterocycle, a fused heterocycle, a heteroaryl group, or a Z-substituted 5-18 membered cycloalkyl, an aryl group, a fused ring, a heterocycle, a fused heterocycle, a heteroaryl group, or a $-CF_3$ or C1 substituted hydrocarbyl group.

11. The compound of claim 1, wherein

$R_{10}$ is 7-18 membered a cycloalkyl group, an aryl group, a fused ring, a heterocycle, a fused heterocycle, a heteroaryl group, or a Z-substituted 7-18 membered cycloalkyl group, an aryl group, a fused ring, a heterocycle, a fused heterocycle, a heteroaryl group, or a $-CF_3$ or C1 substituted hydrocarbyl group; or
$R_{10}$ is -O-Cz, Cz is a 7-18 membered cycloalkyl group, an aryl group, a fused ring, a heterocycle, a fused heterocycle, a heteroaryl group, or a Z-substituted 7-18 membered cycloalkyl group, an aryl group, a fused ring, a heterocycle, a fused heterocycle, a heteroaryl group, or a $-CF_3$ or C1 substituted hydrocarbyl group.

12. The compound of claim 1, wherein the compound comprises the structure of formula 1-2:

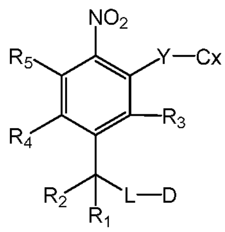

I-2

wherein,
Cx is selected from biphenyl, Z-substituted biphenyl, phenylpyridyl, Z-substituted phenylpyridyl, and biphenyl and phenylpyridyl that with a substituent of $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$, $-NR^6SO_2NR^6R^7$, $-COR^6$, $-NR^6CONR^7$.

13. The compound of claim 12, wherein -Y- is connected to a para-position of a carbon atom connecting two benzenes of -Cx, and a substituent on biphenyl is F or methyl.

14. The compound of claim 12, wherein Cx is selected from the group consisting of -F, $-CF_3$, $-CH_3$ substituted biphenyl and phenylpyridine.

15. The compound of claim 1, wherein $R_3$, $R_4$, and $R_5$ are each independent hydrogen.

**16.** The compound of claim 1, wherein
$R_1$, $R_2$ are each independent hydrogen, deuterium, $-CH_3$, $-CD_3$, $-CF_3$.

**17.** The compound of claim 1, wherein
Y is -O-.

**18.** The compound of claim 1, wherein

Cy is a 5-10 membered heteroaryl group or a benzene ring, or
hydrogens thereon are each independent substituted by deuterium, halogen, a cyano group or an isocyano group, a thiocyanato group or an isothiocyanato group, hydroxyl, a thiol group, an amino group, oxime, hydrazone, OTs, OMs, a $C_1$-$C_6$ aliphatic group; or substituted by a $C_1$-$C_6$ aliphatic group substituted by halogen, a cyano group or an isocyano group, a hydroxyl group, a thiol group, an amino group, oxime, hydrazone.

**19.** The compound of claim 1, wherein
a substituent on Cy is hydrogen, deuterium, halogen, $-CH_3$, or $-CF_3$.

**20.** The compound of claim 1, wherein
Cy is selected from a 5-8 membered aromatic heterocycle, a heteroatom is N, S or O, and the number of the heteroatom is 1, 2, or 3.

**21.** The compound of claim 1, wherein
the compound is selected from the structures below:

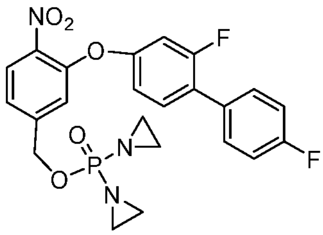,

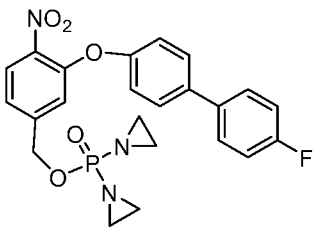, 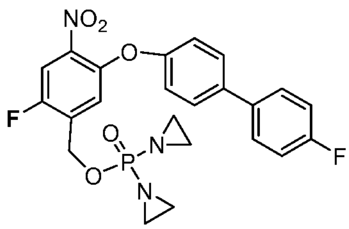, 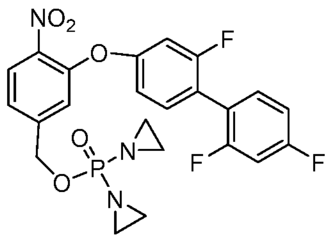,

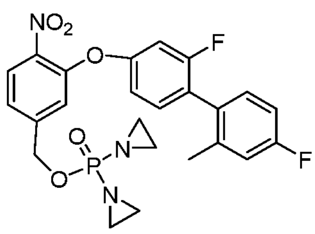, 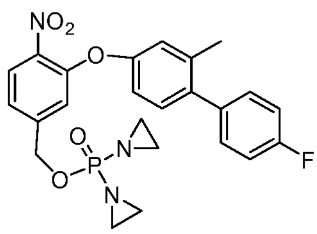, 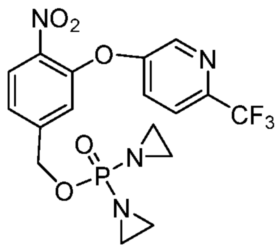,

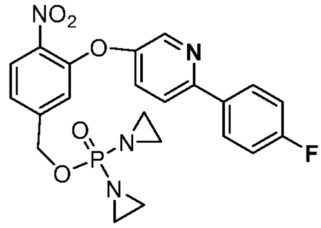, 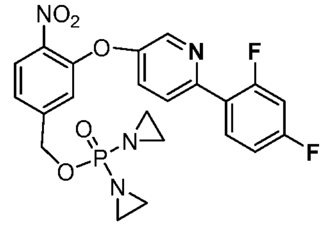, 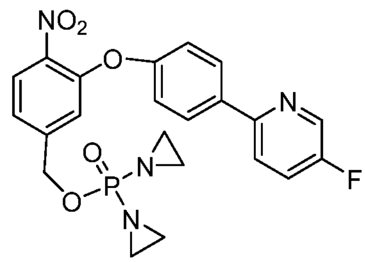,

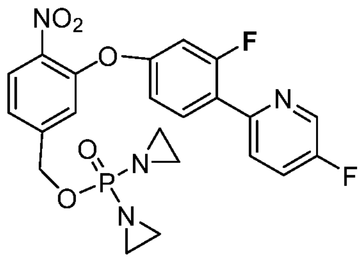, 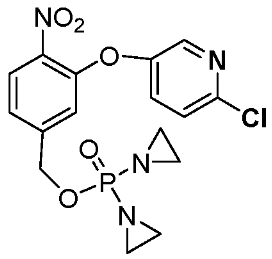, 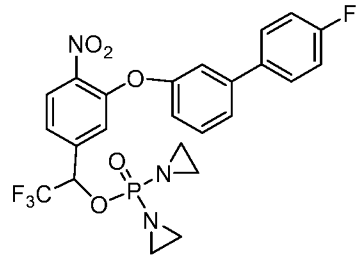,

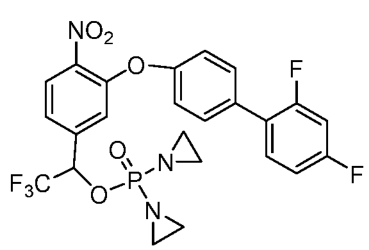, 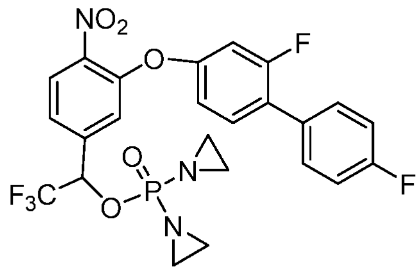,

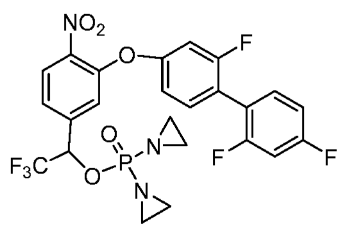, 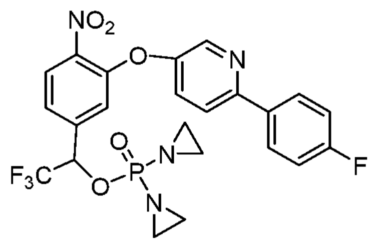,

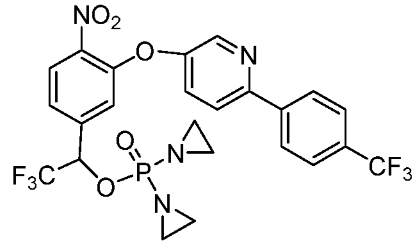, and 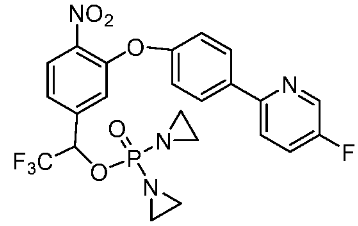.

**22.** The compound of claim 1, wherein:

D-OH is selected from the anti-cancer drug containing -OH below: gemcitabine, estramusting, pudnimnstine, chlorozotocin, ranimustine, mannomustine, mitobronitol, dibromodulcitol, aclacinomycins, anthramycin, bleomycin, carubicin, doxorubicin, carzinophilin, chromomycin, dactinomycin, daunorubicin, mycophenolic acid, nogalamycin, olivomycin, peplomycin, plicamycin, puromycin, streptonigrin, streptozocin, tubercidin, ubenimex, zinostatin, zorubicin, denopterin, fludarabine, ancitabine, azacitidine, 6-azauridine, cytarabine, dideoxyuridine, doxifluridine, enocitabine, floxuridine, L-asparaginase, pulmozyme, aceglatone, elliptinium acetate, etoglucid, interferon-alpha, interferon-beta, interferon-gamma, interleukin-2, lentinan, mitoxantrone, mopidamol, pentostatin, pirarubicin, podophyllinic acid, sizofiran, paclitaxel, teniposide, tenuazonic acidm vinblastine, and vinc-

ristine;

D-$NR^{43}H$ is selected from the anti-cancer drug below: erlotinib, meturedepa, uredepa, imatinib, trimethylolomelamine, gefitinib, uracil mustard, carmustine, chlorozotocin, fotemustine, nimustine, ranimustine, dacarbazine, mannomustine, actinomycin, anthramycin, bleomycin, cactinomycin, carubicin, doxorubicin, carzinophilin, dactinomycin, peplomycin, puromycin, streptozocin, ubenimex, zinostatin, denopterin, pteropterin, trimetrexate, 6-mercaptopurine, thiamiprine, thioguanine, 6-azauridine, carmofur, dideoxyuridine, doxifluridine, enocitabine, floxuridine, 5-fluorouracil, tegafur, L-asparaginase, pulmozyme, amsacrine, bisantrene, demecolcine, diaziquone, elliptinium acetate, flutamide, hydroxyurea, interferon-alpha, interferon-beta, interferon-gamma, interleukin-2, mitoxantrone, nitracrine, pentostatin, phenamet, 2-ethylhydrazide, procarbazine, razoxane, erlotonib, urethane, vinblastine, and vincristine; and

the anti-cancer drug containing secondary or tertiary nitrogen is selected from: altretamine, triethylenemelamine, chlorambuci, chlornaphazine, estramustine, gefitinib, mechlorethamine, mechlorethamine oxide hydrochloride, melphalan, novembichin, phenesterine, prednimustine, trofosfamide, uracil mustard, carmustine, chlorozotocin, fotemustine, nimustine, ranimustine, dacarbazine, pipobroman, actinomycin, anthramycin, carzinophilin, dactinomycin, nogalamycin, porfiromycin, puromycin, streptozocin, tubercidin, fludarabine, ancitabine, azacitidine, cytarabine, dideoxyuridine, enocitabine, floxuridine, L-asparaginase, pulmozyme, aldophosphamide glycoside, bestrabucil, diaziquone, interferon-alpha, interferon-beta, interferon-gamma, interleukin-2, mitoguazone, mopidamo, nitracrine, pentostatin, phenamet, razoxane, spirogermanium, tamoxifen, triaziquone, 2,2',2"-trichlorotriethylamine, vinblastine, and vincristine.

**23.** A drug with a compound described in any one of claims 1-22, wherein
the drug is used to treat an cancer, a tumor expressing AKR1C3 enzyme, or a disease or cell proliferative disease caused by the cancer the tumor expressing AKR1C3 enzyme.

**24.** The drug of claim 23, wherein the cancer or the tumor is a cancer or a tumor of central nervous system, and the disease comprises pain.

**25.** The drug of claim 23, wherein the cancer or the tumor is a primary brain cancer or a primary tumor, or a metastatic cancer or a metastatic tumor transferred to the brain.

**26.** A use of the compound of any one of claims 1-22 for preparing a drug, wherein
the drug is used to treat an cancer, a tumor expressing AKR1C3 enzyme, or a disease or cell proliferative disease caused by the cancer the tumor expressing AKR1C3 enzyme, wherein the cancer or the tumor is a cancer or a tumor of central nervous system, and the disease comprises pain.

**27.** The use of claim 26, wherein the cancer or the tumor is a primary brain cancer or a primary tumor, or a metastatic cancer or a metastatic tumor transferred to the brain.

**28.** The use of claim 26, wherein the compound is selected from:

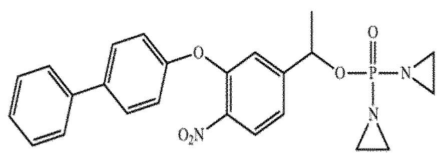,

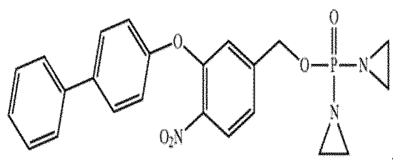,

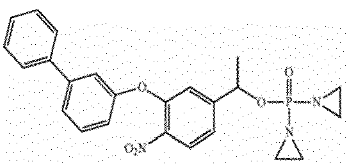,

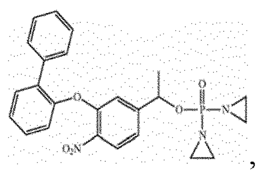,

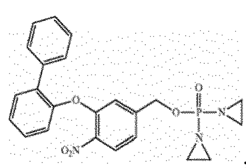,

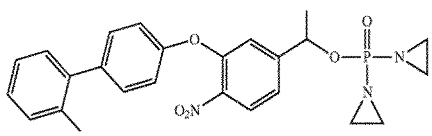, 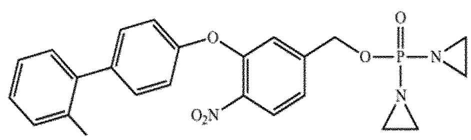,

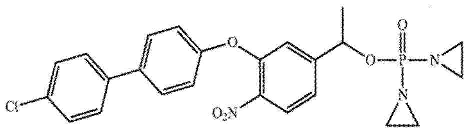,

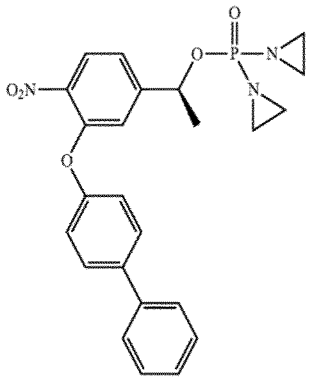, . 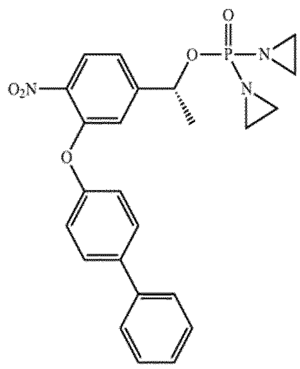,

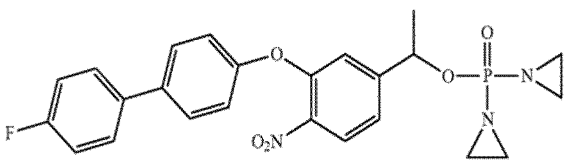,

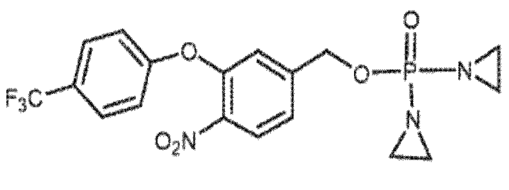, 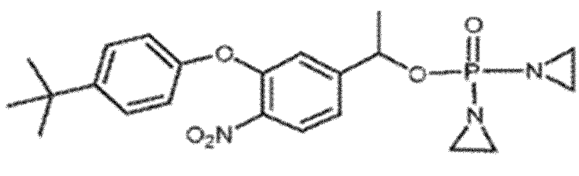,

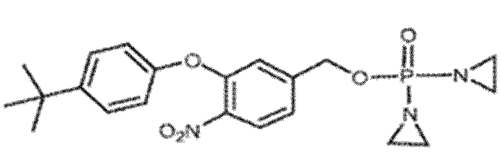, 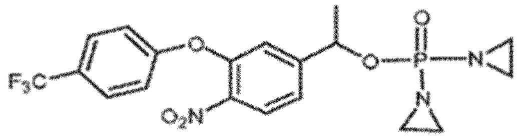,

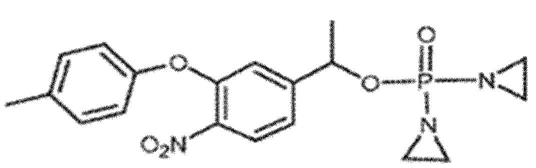,

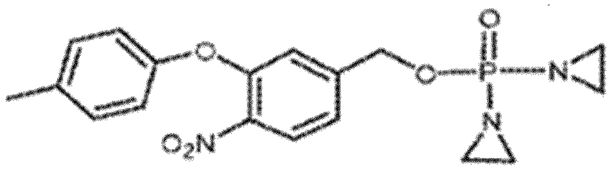

,

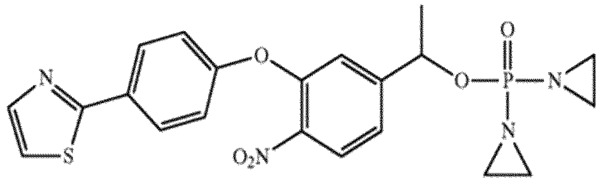

,

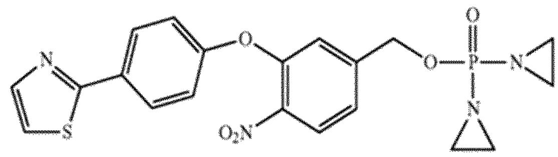

,

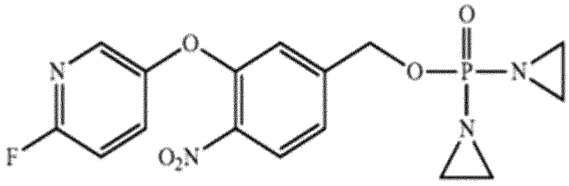

,

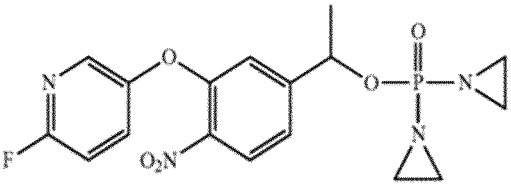

,

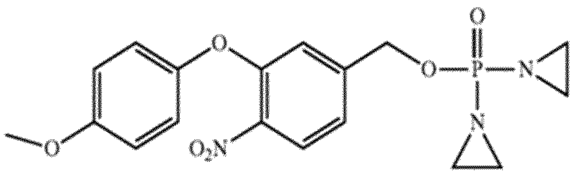

,

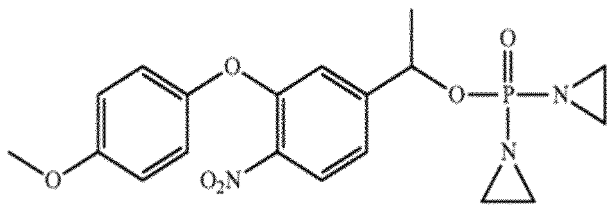

,

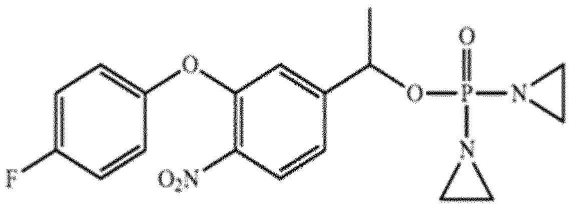

,

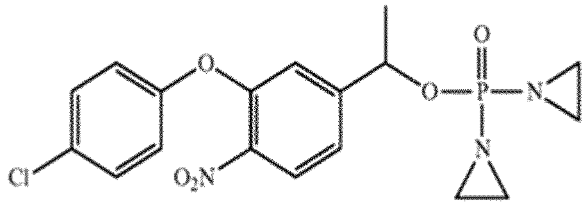

,

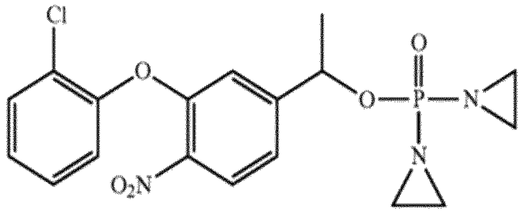

,

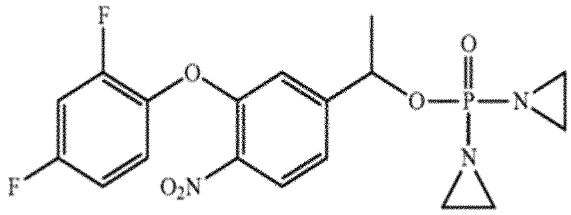

,

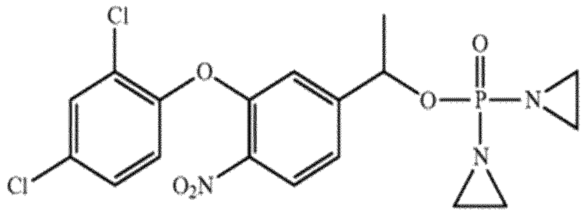

,

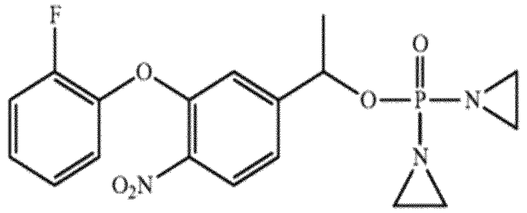

,

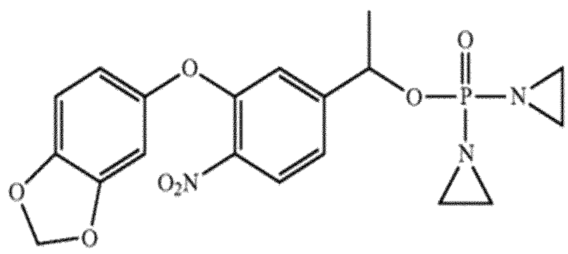

,

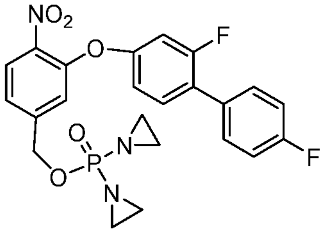,

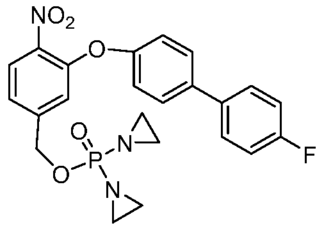,

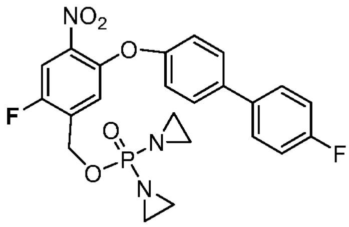,

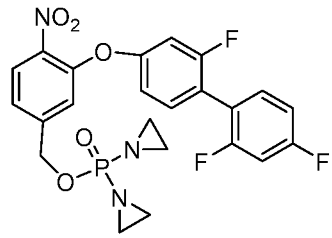,

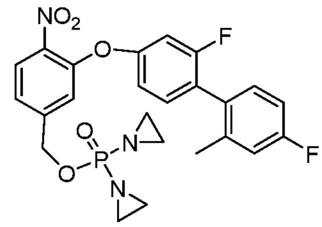,

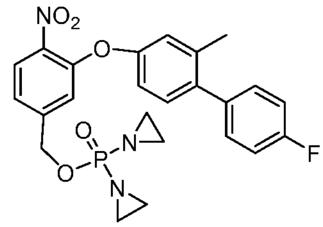,

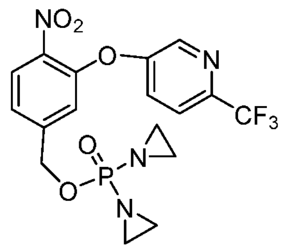,

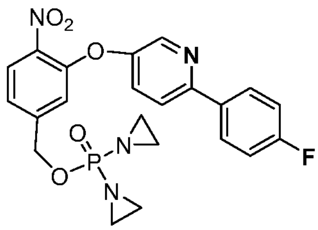,

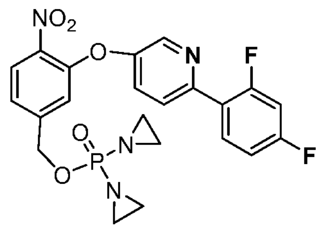,

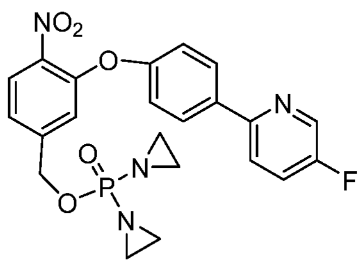,

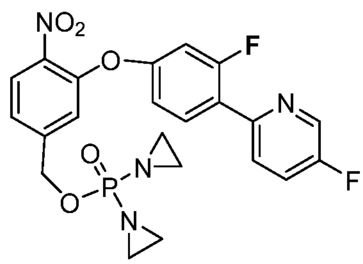,

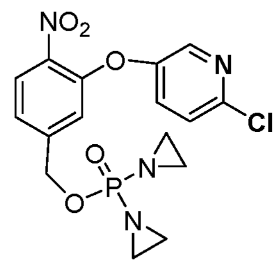,

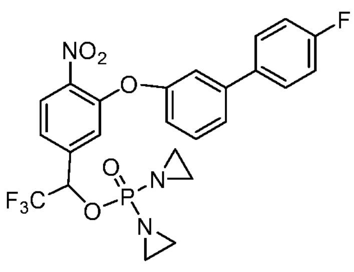,

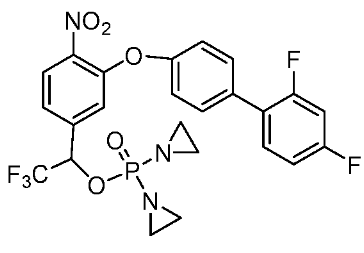,

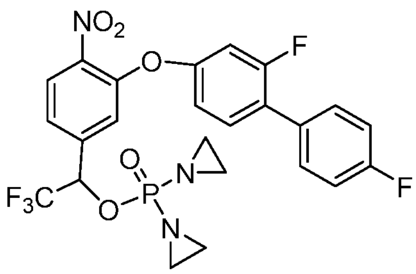,

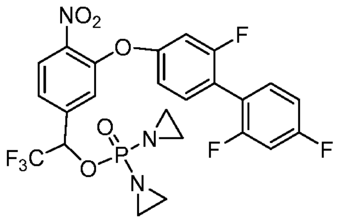

,

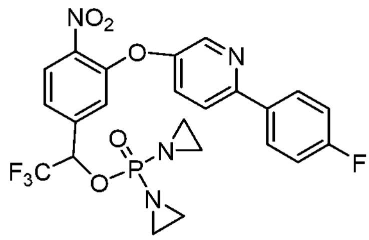

,

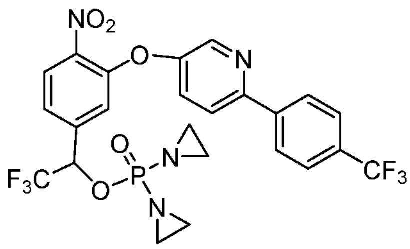

, or

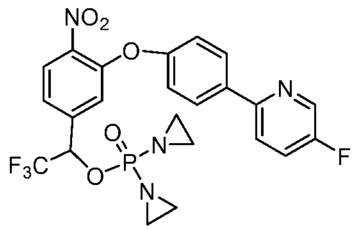

.

**29.** A method of using a compound of any one of claims 1-22, or a pharmaceutically acceptable salt thereof, for treating an cancer, tumor expressing AKR1C3 enzyme or treating a disease or cell proliferative disease caused by the cancer the tumor expressing AKR1C3 enzyme, wherein the method comprises:
administering an effective dose of the compound of any one of claims 1-22 or a pharmaceutically acceptable salt thereof.

**INTERNATIONAL SEARCH REPORT**

International application No.
**PCT/CN2020/125123**

**A. CLASSIFICATION OF SUBJECT MATTER**

C07F 9/564(2006.01)i; A61K 31/04(2006.01)i; A61K 31/06(2006.01)i; A61K 31/664(2006.01)i; A61P 35/00(2006.01)i

According to International Patent Classification (IPC) or to both national classification and IPC

**B. FIELDS SEARCHED**

Minimum documentation searched (classification system followed by classification symbols)

C07F A61K A61P

Documentation searched other than minimum documentation to the extent that such documents are included in the fields searched

Electronic data base consulted during the international search (name of data base and, where practicable, search terms used)

WPI, EPODOC, CNPAT, CNKI, REGISTRY, CAPLUS: 结构式, 苯基, 吡啶, 癌, AKR1C3, 磷

**C. DOCUMENTS CONSIDERED TO BE RELEVANT**

| Category* | Citation of document, with indication, where appropriate, of the relevant passages | Relevant to claim No. |
|---|---|---|
| X | CN 107530556 A (THRESHOLD PHARMACEUTICALS, INC.) 02 January 2018 (2018-01-02)<br>description, pages 10-25, compound, claims 19-20 | 1-29 |
| X | CN 108136214 A (THRESHOLD PHARMACEUTICALS, INC.) 08 June 2018 (2018-06-08)<br>embodiments, in particular description, paragraphs 0156-0157, compound, claims 19-20 | 1-29 |
| X | CN 108290911 A (THRESHOLD PHARMACEUTICALS, INC.) 17 July 2018 (2018-07-17)<br>claims 1-2, 10-11 | 1-29 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 December 2020** | **30 December 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/125123**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29**
because they relate to subject matter not required to be searched by this Authority, namely:

[1] The subject matter of claim 29 relates to a method for treatment of the human or animal body, and thus belongs to subject matter which does not require a search as stipulated in PCT Rule 39.1(iv). The present report is provided on the basis of a use of the compound in preparing a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/125123**

| Patent document cited in search report | | | Publication date (day/month/year) | Patent family member(s) | | | Publication date (day/month/year) |
|---|---|---|---|---|---|---|---|
| CN | 107530556 | A | 02 January 2018 | SG | 11201707293V | A | 30 October 2017 |
| | | | | JP | 6704421 | B2 | 03 June 2020 |
| | | | | EP | 3277380 | A4 | 01 August 2018 |
| | | | | KR | 20190072688 | A | 25 June 2019 |
| | | | | KR | 102034618 | B1 | 21 October 2019 |
| | | | | AU | 2016229136 | B2 | 09 August 2018 |
| | | | | SG | 10201913462 X | A | 30 March 2020 |
| | | | | WO | 2016145092 | A1 | 15 September 2016 |
| | | | | CN | 107530556 | B | 10 April 2020 |
| | | | | KR | 20170128280 | A | 22 November 2017 |
| | | | | US | 10364261 | B2 | 30 July 2019 |
| | | | | IL | 254377 | D0 | 30 November 2017 |
| | | | | AU | 2016229136 | A1 | 05 October 2017 |
| | | | | KR | 20190072689 | A | 25 June 2019 |
| | | | | BR | 112017019287 | A2 | 02 May 2018 |
| | | | | JP | 2018513876 | A | 31 May 2018 |
| | | | | US | 2019225633 | A1 | 25 July 2019 |
| | | | | US | 2018044360 | A1 | 15 February 2018 |
| | | | | TW | 201706262 | A | 16 February 2017 |
| | | | | CA | 2979251 | A1 | 15 September 2016 |
| | | | | EP | 3277380 | A1 | 07 February 2018 |
| | | | | TW | I674258 | B | 11 October 2019 |
| CN | 108136214 | A | 08 June 2018 | WO | 2016161342 | A2 | 06 October 2016 |
| | | | | US | 2018086693 | A1 | 29 March 2018 |
| | | | | EP | 3277381 | A4 | 05 December 2018 |
| | | | | AU | 2016244000 | B2 | 02 July 2020 |
| | | | | WO | 2016161342 | A3 | 10 November 2016 |
| | | | | HK | 1250959 | A1 | 18 January 2019 |
| | | | | IL | 254769 | D0 | 31 December 2017 |
| | | | | AU | 2016244000 | A1 | 12 October 2017 |
| | | | | SG | CN 11201707375 U | A | 30 October 2017 |
| | | | | CN | 108136214 | B | 28 August 2020 |
| | | | | JP | 2019178172 | A | 17 October 2019 |
| | | | | TW | 201706267 | A | 16 February 2017 |
| | | | | CA | 2981494 | A1 | 06 October 2016 |
| | | | | KR | 20170127463 | A | 21 November 2017 |
| | | | | JP | 2018511612 | A | 26 April 2018 |
| | | | | BR | 112017021167 | A2 | 03 July 2018 |
| | | | | JP | 6612892 | B2 | 27 November 2019 |
| | | | | KR | 102001910 | B1 | 19 July 2019 |
| | | | | KR | 20190075145 | A | 28 June 2019 |
| | | | | EP | 3277381 | A2 | 07 February 2018 |
| | | | | SG | 10201913709 Q | A | 30 March 2020 |
| | | | | IL | 254769 | A | 31 May 2020 |
| CN | 108290911 | A | 17 July 2018 | CN | 108290911 | B | 08 May 2020 |
| | | | | BR | 112017025778 | A2 | 14 August 2018 |
| | | | | TW | 201726695 | A | 01 August 2017 |
| | | | | EP | 3390415 | A4 | 22 May 2019 |
| | | | | CA | 2990696 | A1 | 26 May 2017 |

Form PCT/ISA/210 (patent family annex) (January 2015)

**INTERNATIONAL SEARCH REPORT**
**Information on patent family members**

International application No.
**PCT/CN2020/125123**

| Patent document cited in search report | Publication date (day/month/year) | Patent family member(s) | | | Publication date (day/month/year) |
|---|---|---|---|---|---|
| | | IL | 256569 | D0 | 28 February 2018 |
| | | KR | 101985778 | B1 | 04 June 2019 |
| | | EP | 3390415 | A1 | 24 October 2018 |
| | | AU | 2016357728 | B2 | 27 August 2020 |
| | | KR | 20170130615 | A | 28 November 2017 |
| | | JP | 2018517710 | A | 05 July 2018 |
| | | WO | 2017087428 | A1 | 26 May 2017 |
| | | JP | 6695360 | B2 | 20 May 2020 |
| | | HK | 1250988 | A1 | 18 January 2019 |
| | | EP | 3390415 | B1 | 26 February 2020 |
| | | AU | 2016357728 | A1 | 30 November 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016021581 W
- WO 2016145092 A1
- CN 2016800150788
- CN 107530556 A
- WO 2016145092 A
- US 2016062114 W
- WO 2017087428 A
- US 2016025665 W
- WO 2016161342 A
- WO 2016061342 A
- CN 2016800200132
- CN 108136214 A
- CN 2016800446081
- CN 108290911 A


### Non-patent literature cited in the description


- **NOGRADY.** Medicinal Chemistry A Biochemical Approach. Oxford University Press, 1985, 388-392
- **FLANAGAN et al.** *Bioorganic and Medicinal Chemistry,* 2014, 962-977